# EUROPEAN PATENT SPECIFICATION

(11) **EP 0 778 842 B1**
(45) Date of publication and mention of the grant of the patent: **02.11.2005**
(21) Application number: 95925552.2
(22) Date of filing: 06.07.1995
(51) Int. Cl.: C07H 21/00, C07H 21/02, C07H 21/04, C12N 5/10, C12N 9/12, C12N 15/11, C12N 15/52, C12N 15/63

(54) **MAMMALIAN TELOMERASE**
SÄUGETIER-TELOMERASE
TELOMERASE MAMMIFERE

(30) Priority: 07.07.1994 US 272102; 27.10.1994 US 330123; 07.06.1995 US 472802; 07.06.1995 US 482115
(43) Date of publication of application: 18.06.1997
(60) Divisional application: 02080023.1
(73) Proprietor: GERON CORPORATION, Menlo Park, CA 94025 (US)
(72) Inventor: VILLEPONTEAU, Bryant, San Carlos, CA 94070 (US); FENG, Junli, San Carlos, CA 94070 (US); FUNK, Walter, Union City, CA 94587 (US); ANDREWS, William H., Richmond, CA 94805 (US)
(74) Representative: Bizley, Richard Edward
(86) International application number: PCT/US1995/008530
(87) International publication number: WO 1996/001835

(56) References cited:
- EP-A- 0 666 313
- WO-A-93/23572
- WO-A-95/13382
- WO-A-96/01614
- BLACKBURN E H: "TELOMERASES" ANNUAL REVIEW OF BIOCHEMISTRY, vol. 61, 1 January 1992, pages 113-129, XP000676909
- AUTEXIER C ET AL: "FUNCTIONAL RECONSTITUTION OF WILD-TYPE AND MUTANT TETRAHYMENA TELOMERASE" GENES AND DEVELOPMENT, vol. 8, no. 5, 1 March 1994, pages 563-575, XP002004672
- HARLEY C B ET AL: "HUMAN TELOMERASE INHIBITION AND CANCER" PROCEEDINGS OF THE ANNUAL MEETING OF THE AMERICAN ASSOCIATION FOR CANCER RESEARCH, TORONTO, MAR. 18 - 22, 1995, no. MEETING 86, 1 March 1995, page 671/672 XP000645375 AMERICAN ASSOCIATION FOR CANCER RESEARCH
- FENG J ET AL: "THE RNA COMPONENT OF HUMAN TELOMERASE" SCIENCE, vol. 269, no. 5228, 1 September 1995, pages 1236-1241, XP000645335
- BLASCO, M. ET AL.: "Functional characterization and developmental regulation of mouse telomerase RNA" SCIENCE, vol. 269, no. 5228, 1 September 1995, pages 1267-1270, XP000645336
- J. BIOLOGICAL CHEMISTRY, Volume 267, No. 32, issued 15 November 1992, R. YAN et al., "Amino Acid Sequence of the Human Protein Synthesis Initiation Factor Elf-4gamma", pages 23226-23231.
- PROC. NATL. ACAD. SCI. U.S.A., Volume 90, issued February 1993, PROWSE et al., "Identification of a Nonprocessive Telomerase Activity from Mouse Cells", pages 1493-1497.
- NATURE, Volume 344, issued 08 March 1990, YU et al., "In Vivo Alteration of Telomere Sequences and Senescence Caused by Mutated Tetrahymena Telomerase RNAs", pages 126-132.
- COLD SPRING HARBOR SYMPOSIUM ON QUANTITATIVE BIOLOGY, Volume 58, issued 1993, GREIDER, "Telomerase and Telomere-Length Regulation: Lessons from Small Eukaryotes to Mammals", pages 719-723.
- TRENDS IN BIOLOGICAL SCIENCES, Volume 16, issued October 1991, BLACKBURN, "Telomeres", pages 378-381.

## Description

### Background of the Invention

### Field of Invention

The present invention relates to human telomerase, a ribonucleoprotein enzyme involved in human telomere DNA synthesis. The invention provides methods and compositions relating to the fields of molecular biology, chemistry, pharmacology, and medical and diagnostic technology.

### Description of Related Disclosures

The DNA at the ends or telomeres of the chromosomes of eukaryotes usually consists of tandemly repeated simple sequences. Telomerase is a ribonucleoprotein enzyme that synthesizes one strand of the telomeric DNA using as a template a sequence contained within the RNA component of the enzyme. See Blackburn, 1992, Annu. Rev. Biochem. 61:113-129.

The RNA component of human telomerase has not been reported in the scientific literature to date, although human telomerase is known to synthesize telomeric repeat units with the sequence 5'-TTAGGG-3'. See Morin, 1989, Cell 59:521-529, and Morin, 1991, Nature 353:454-456. This knowledge has not been sufficient to enable the isolation and identification of the remainder of the nucleotide sequence of the RNA component of human telomerase. The RNA component of the telomerase enzymes of *Saccharomyces cerevisiae*, certain species of *Tetrahymena*, as well as that of other ciliates, such as *Euplotes* and *Glaucoma*, has been sequenced and reported in the scientific literature. See Singer and Gottschling, 21 Oct. 1994, Science 266:404-409; Lingner et al., 1994, Genes & Development 8:1984-1988; Greider and Blackburn, 1989, Nature 337:331-337; Romero and Blackburn, 1991, Cell 67:343-353; and Shippen-Lentz and Blackburn, 1990, Science 247:546-552. The telomerase enzymes of these ciliates synthesize telqmeric repeat units distinct from that in humans.

There is a great need for more information about human telomerase. Despite the seemingly simple nature of the repeat units of telomeric DNA, scientists have long known that telomeres have an important biological role in maintaining chromosome structure and function. More recently, scientists have speculated that loss of telomeric DNA may act as a trigger of cellular senescence and aging and that regulation of telomerase may have important biological implications. See Harley, 1991, Mutation Research 256:271-282.

Methods for detecting telomerase activity, as well as for identifying compounds that regulate or affect telomerase activity, together with methods for therapy and diagnosis of cellular senescence and immortalization by controlling telomere length and telomerase activity, have also been described. See PCT patent publication Nos. 95/13381, published 18 May 1995; 95/13382, published 18 May 1995; and 93/23572, published November 25, 1993.

Significant improvements to and new opportunities for telomerase-mediated therapies and telomerase assays and screening methods could be realized if nucleic acid comprising the RNA component and/or encoding the protein components of telomerase were available in pure or isolatable form and the nucleotide sequences of such nucleic acids were known. The present invention meets these and other needs and provides such improvements and opportunities.

### Summar of the Invention

The invention provides a substantially pure ribonucleic acid as defined in claim 1. More specific embodiments of this aspect of the invention are defined in claims 2 to 4.

The invention also provides an isolated nucleic acid molecule encoding a ribonucleic acid of the invention, as well as a recombinant plasmid comprising a nucleic acid sequence encoding a ribonucleic acid of the invention. Another aspect of the invention is a host cell transformed with the plasmid.

In another aspect the invention provides an oligonucleotide as defined in claim 8 with more specific embodiments of the oligonucleotides as defined in claims 9 to 14. The invention includes a pharmaceutical formulation comprising the oligonucleotides, as well as the use of the oligonucleotides as a pharmaceutical. In one aspect the oligonucleotides are used to treat neoplasia.

The invention additionally includes a method of inhibiting telomerase activity in a cell *in vitro* as defined in claim 18.

In another aspect the invention provides an isolated, recombinant or synthesized nucleic acid as defined in claim 19, or more specifically as defined in claim 20.

The invention also provides for the use of an oligonucleotide of the invention as a probe or a primer as defined in claim 21, or more specifically as claimed in claim 22.

In another aspect the invention provides a method of determining the level, amount or presence of telomerase RNA component in a mammalian sample as defined in claim 23.

More specific features of the method are defined in claim 24.

The invention also provides a method of detecting telomerase RNA component in a patient sample as defined in claim 25.

The invention further provides a method of detecting the presence of cancer cells in a sample as defined in claim 26.

In another aspect the invention provides a kit for determining the level, amount or presence of human telomerase RNA component in a sample as defined in claim 27.

In a further aspect the invention provides a method for isolating telomerase holoenzyme as defined in claim 28, or as further defined in claim 29.

The invention also provides a method of identifying a telomerase RNA component as defined in claim 30.

In another aspect the invention provides an isolated, recombinant or synthesized nucleic acid as defined in claim 31 or as further defined in claim 32. Also included is a recombinant plasmid as defined in claim 33 or a host cell as defined in claim 34.

Also provided is a non-human transgenic mammal as defined in claim 35.

The present invention therefore provides the RNA component of, as well as the gene for the RNA component of, human telomerase in substantially pure form, as well as nucleic acids comprising all or at least a useful portion of the nucleotide sequence of the RNA component of human telomerase. The present invention also provider RNA component nucleic acids from other species, which nucleic acids share substantial homology with the RNA component of human telomerase, including but not limited to, the RNA components of mammals, such as primates. Other useful nucleic acids of the invention include nucleic acids with sequences complementary to the RNA component; nucleic acids with sequences related to but distinct from nucleotide sequences of the RNA component and which interact with the RNA component or the gene for the RNA component or the protein components of human telomerase in a useful way; and nucleic acids that do not share significant sequence homology or complementarity to the RNA component or the gene for the RNA component but act on the RNA component in a desired and useful way. As described more fully below, the nucleic acids of the invention include both DNA and RNA molecules and modified analogues of either and serve a variety of useful purposes.

One type of useful nucleic acid of the invention is an antisense oligonucleotide, a triple helix-forming oligonucleotide, or other oligonucleotide or oligonucleotide mimetic (e.g., antisense PNA - peptide nucleic acid/polyamide nucleic acid) that can be used in vivo or in vitro to inhibit the activity of human telomerase. Such oligonucleotides can block telomerase activity in a number of ways, including by preventing transcription of the telomerase gene (for instance, by triple helix formation) or by binding to the RNA component of telomerase in a manner that prevents a functional ribonucleoprotein telomerase from assembling or prevents the RNA component, once assembled into the telomerase enzyme complex, from serving as a template for telomeric DNA synthesis. Typically, and depending on mode of action, these oligonucleotides of the invention comprise a specific sequence of from about 10 to about 25 to 200 or more nucleotides that is either identical or complementary to a specific sequence of nucleotides in the RNA component of telomerase or the gene for the RNA component of telomerase.

In an embodiment, the invention provides antisense polynucleotides complementary to telomerase RNA component polynucleotide sequences, typically complementary to polynucleotide sequences which are substantially identical to a naturally-occurring mammalian telomerase RNA component gene sequence. Such antisense polynucleotides are employed to inhibit transcription and/or stability and/or functionality of the telomerase RNA component species and thereby effect a reduction in the amount of the respective telomerase activity in a cell (e.g., a neoplastic cell of a patient). Such antisense polynucleotides can function as telomerase-modulating agents by inhibiting the formation of functional (catalytically active and high fidelity) telomerase holoenzyme required for correct telomere replication and repair in a cell. Antisense polynucleotides can be combined with other antineoplastic therapeutic modalities, such as ionizing radiation or chemotherapy (e.g., with a DNA-damaging agent such as bleomycin, cisplatin, nitrogen mustard, doxyrubicin, nucleotide analogs, and the like). The antisense polynucleotides can promote cell death in susceptible cells (e.g., replicating cells requiring telomerase activity for DNA repair or replication). The antisense polynucleotides may be substantially identical to at least 25 contiguous nucleotides of the complementary sequence of a mammalian telomerase RNA sequence disclosed herein. The antisense polynucleotides are typically ssDNA, ssRNA, methylphosphonate backbone polynucleotides, phosphorothiolate backbone polynucleotides, mixed backbone polynucleotides, polyamide nucleic acids, and the like antisense structures known in the art. In one aspect of the invention, an antisense polynucleotide is administered to inhibit transcription and/or activity of telomerase RNA component and telomerase activity in a cell, such as in a replicable human cell.

In an embodiment, the invention provides a template mismatch polynucleotide, said polynucleotide having a sequence substantially identical to a mammalian RNA component of telomerase and comprising a telomeric repeat template sequence having at least one base mismatch with respect to, but otherwise complementary to, the human telomerase repeat sequence 5'-TTAGGG-3'. A template mismatch polynucleotide typically comprises a single nucleotide mismatch in the naturally-occurring template sequence, and may comprise two nucleotide mismatches in the template sequence, either as adjacent mismatched nucleotides or wherein the mismatched nucleotides are separated by one or more matched (complementary) nucleotides. Template mismatch polynucleotides of the invention are generally capable of exhibiting telomerase activity in conjunction with human telomerase polypeptide component, and thereby producing misincorporation at selected (mismatched) nucleotide positions in the human telomerase repeat sequence consequent to telomere repeat replication, repair, and/or addition, thus generating telomeres which rely on the continued presence of the mutated sense telomerase RNA component for substantial replication and maintenance of telomere length.

Another type of useful nucleic acid of the invention is a ribozyme able to cleave specifically the RNA component of human telomerase, rendering the enzyme inactive. Yet another type of useful nucleic acid of the invention is a probe or primer that binds specifically to the RNA component of human telomerase and so can be used, e.g., to detect the presence of telomerase in a sample. Finally, useful nucleic acids of the invention include recombinant expression plasmids for producing the nucleic acids of the invention. One especially useful type of such a plasmid is a plasmid used for human gene therapy. Useful plasmids of the invention for human gene therapy come in a variety of types, including not only those that encode antisense oligonucleotides or ribozymes but also those that drive expression of the RNA component of human telomerase or a deleted or otherwise altered (mutated) version of the RNA component of human (or other species with RNA component sequences substantially homologous to the human RNA component) telomerase or the gene for the same.

In an embodiment, a polynucleotide having a portion complementary to a mammalian telomerase RNA component sufficient to specifically hybridize under physiological conditions is derivatized by covalent linkage of an additional chemical substituent, either during or after polynucleotide synthesis, thereby forming a derivatized polynucleotide capable of specifically hybridizing to said telomerase RNA component. The derivatized polynucleotide can localize to endogenous telomerase enzyme having said RNA component wherein said derivatized polynucleotide produces an alteration or chemical modification the RNA component and/or protein component of telomerase, and thereby modifies, typically by reducing, telomerase enzymatic activity.

The invention therefore provides polynucleotides which are suitable to diagnose diseases associated with aberrant telomerase RNA component abundance and/or structure. Polynucleotide probes comprising sequences that are substantially identical to or substantially complementary to a mammalian telomerase RNA component nucleotide sequence can be used for diagnosis of disease states (e.g., neoplasia or preneoplasia) by detection of a telomerase RNA component abundance and/or telomerase RNA component structural alterations (e.g., truncation, sequence variations, deletions or insertions, and the like), and/or rearrangements or amplification of the telomerase RNA component gene in cells explanted from a patient, or detection of a pathognomonic mammalian telomerase RNA component allele (e.g., by RFLP or allele-specific PCR analysis). The detection often will be by in situ hybridization using a labeled (e.g., ³²P, ³⁵S, ¹⁴C, ³H, fluorescent, biotinylated, digoxigeninylated) antisense polynucleotide complementary to a mammalian telomerase RNA component, although Northern blotting, dot blotting, or solution hybridization on bulk RNA or poly A⁺ RNA isolated from a cell sample may be used, as may PCR or LCR amplification using telomerase RNA component-specific primers. Cells which contain an altered amount (typically a significant increase) of telomerase RNA component as compared to non-neoplastic cells of the same cell type(s) are identified as candidate diseased cells, such as preneoplastic or frankly neoplastic cells, and may be identified as cells having metastatic potential. Similarly, the detection of pathognomonic rearrangements or amplification of the telomerase RNA component gene locus or closely linked loci in a cell sample will identify the presence of a pathological condition or a predisposition to developing a pathological condition (e.g., cancer, genetic disease). The telomerase RNA component polynucleotide probes are also used for forensic identification of individuals, such as for paternity testing or identification of criminal suspects or unknown decedents.

The invention permits treatment of a condition associated with the telomerase activity within a cell or group of cells by contacting the cell(s) with a therapeutically effective amount of an agent that alters telomerase activity in that cell. Such agents include the telomerase RNA component-encoding nucleic acids, triple helix-forming oligonucleotides, antisense oligonucleotides, ribozymes, and plasmids and other gene therapy vectors (e.g., adenoviral vectors, adeno-associated viral vectors, etc.) for human gene therapy by expression of telomerase RNA component, antisense RNA to telomerase RNA component, or mismatch telomerase RNA component, as described above. In a related aspect, the invention provides pharmaceutical compositions comprising these therapeutic agents together with a pharmaceutically acceptable carrier or salt, which may include formulation in a lipofection complex, liposome, or immunoliposome for targeted delivery of the therapeutic agent. The invention also provides combinations of such telomerase-mediated therapeutic agents with other pharmaceuticals, such as antineoplastic agents and other cytotoxic or cytostatic agents; antifungal agents (e.g., for treatment of AIDS patients); nucleotides, nucleosides, and analogs thereof; and other pharmaceutical agents suitable for treating disease conditions such as neoplasia, hyperplasia, HIV-infection/AIDS and associated pathologies, and other diseases characterized by abnormal telomere metabolism. The methods can comprise the use of a derivatized polynucleotide capable of specifically hybridizing to a telomerase RNA component in a mammalian telomerase, wherein the derivatized polynucleotide is delivered into mammalian cells having telomerase activity and inhibits telomerase activity by localizing to telomerase RNA component and inactivating or inhibiting telomerase activity.

The invention also provides therapeutic agents which inhibit neoplasia or apoptosis by modulating telomerase function by inhibiting or augmenting formation of telomerase RNA component; such agents can be used as pharmaceuticals. Such pharmaceuticals will be used to treat a variety of human and veterinary diseases, such as: neoplasia, hyperplasia, neurodegenerative diseases, aging, AIDS, fungal infection, and the like. In an embodiment, the agent consists of a gene therapy vector capable of transcribing a telomerase RNA component sequence or its complement, or alternatively an enzymatically inactive telomerase RNA component which can competitively inhibit formation of functional telomerase holoenzyme.

The invention provides diagnostic methods for determining the level, amount, or presence of the RNA component of human telomerase, telomerase, or telomerase activity in a cell, cell population, or tissue sample, or an extract of any of the foregoing. In a related aspect, the present invention provides useful reagents for such methods (including the primers and probes noted above), optionally packaged into kit form together with instructions for using the kit to practice the diagnostic method.

The present invention provides a method for diagnosing a disease (e.g., neoplasia) in a human patient, wherein a diagnostic assay (e.g., in situ polynucleotide hybridization of fixed cells by a labelled telomerase RNA component probe that specifically binds human telomerase RNA component or gene sequences) is used to determine if a predetermined pathognomonic concentration of telomerase RNA component is present in a biological sample from a human patient; if the assay indicates the presence of telomerase RNA component outside of the normal range (e.g., beyond the predetermined pathognomonic concentration), the patient is diagnosed as having a disease condition or predisposition.

In an embodiment, polynucleotides of the invention are employed for diagnosis of pathological conditions or genetic diseases that involve neoplasia, hyperplasia; premature or abnormal cell senescence, or other medical conditions related to telomerase function, and more specifically conditions and diseases that involve alterations in the structure or abundance of a telomerase RNA component or gene sequence, or which are linked to a pathognomonic telomerase RNA component allele which can be detected by RFLP and/or allele-specific PCR, or other suitable detection method. Typically, the method is for diagnosing a disease (e.g., neoplasia) in a human patient; wherein a diagnostic assay (e.g., determination of amount and/or structure of telomerase RNA component) is used to determine if a predetermined pathognomonic concentration or structure of telomerase RNA component is present in cells in a biological sample from a human patient; if the assay indicates the presence of a pathognomonic amount of telomerase RNA component outside of the normal range (e.g., beyond the predetermined pathognomonic concentration), the patient is diagnosed as having a disease condition or disease predisposition.

The present invention provides recombinant telomerase preparations and methods for producing such preparations. Thus, the present invention provides a recombinant human telomerase that comprises the protein components of human telomerase as well as the protein components of telomerase from a mammalian species with an RNA component substantially homologous to the RNA component of human telomerase in association with a recombinant RNA component of the invention. Such recombinant RNA component molecules of the invention include those that differ from naturally occurring RNA component molecules by one or more base substitutions, deletions, terminal additions and/or insertions, as well as RNA component molecules identical to a naturally occurring RNA component molecule that are produced in recombinant host cells. The method for producing such recombinant telomerase molecules comprises transforming a eukaryotic host cell that expresses the protein components of telomerase with a recombinant expression vector that encodes an RNA component molecule of the invention, and culturing said host cells transformed with said vector under conditions such that the telomerase protein component and telomerase RNA component are expressed and assemble to form an active telomerase molecule capable of adding sequences (not necessarily the same sequence added by native telomerase) to telomeres of chromosomal DNA.

The invention provides methods for purifying the protein components of human telomerase as well as the protein components of telomerase from a mammalian species with an RNA component substantially homologous to the RNA component of human telomerase. The present invention also provides methods for isolating and identifying nucleic acids encoding such protein components. In related aspects, the present invention provides purified human telomerase and purified telomerase of mammalian species with an RNA component substantially homologous to the RNA component of human telomerase, as well as purified nucleic acids that encode one or more components of such telomerase preparations. The present invention also provides pharmaceutical compositions comprising as an active ingredient the protein components of telomerase or a nucleic acid that encodes or interacts with a nucleic acid that encodes a protein component of telomerase.

The invention provides methods for identifying agents which modulate (i.e., inhibit, augment, or alter specificity) of mammalian telomerase activity. Such telomerase-modulating agents are often small molecules (e.g., less than about 3000 Daltons) and can be used to modify telomerase activity in vitro and in vivo, frequently for therapeutic effect, and are used as laboratory reagents and/or pharmaceuticals.

The invention provides methods for identifying, from a bank or library of agents, candidate agents which modulate telomerase activity by modulating the non-covalent binding of a mammalian telomerase RNA component with a telomerase protein component. The telomerase RNA component can be produced from a recombinant template in a host cell or chemically synthesized, if desired. Typically, a mammalian telomerase protein component, such as may be purified from telomerase-expressing cells, and which may have a naturally occurring telomerase RNA component removed (e.g., by RNAse treatment), is contacted with a mammalian telomerase RNA component under suitable aqueous binding conditions to permit non-covalent binding between the RNA and protein components in the absence of an added agent (i.e., in a control binding reaction). The magnitude of noncovalent interaction between the telomerase RNA component and protein component in the presence of one or more agents selected from a library or bank of agents is compared to the magnitude of noncovalent interaction in a control binding reaction comprising telomerase RNA and protein components and lacking the agent(s); agents which produce a statistically significant increase in the magnitude of noncovalent binding between the telomerase RNA and protein components is thereby determined as a candidate telomerase modulating agent. The relative magnitude of noncovalent binding can be determined by any suitable method, including determination of specific binding affinity, such as by competitive binding assay, determination of telomerase catalytic activity on a suitable telomere repeat template, or other suitable assay of functional noncovalent interaction between the RNA and protein components of mammalian telomerase, such as a gel shift or EMSA (electrophoretic mobility shift assay).

In a noncovalent binding assay for detecting candidate telomerase modulating agents, one or both of the telomerase RNA component or protein component are labeled with a suitable detectable label and noncovalent binding is separately determined in the presence and absence of an agent selected from a library or bank of agents. The determination of noncovalent binding comprises measuring the extent to which a labeled telomerase component (RNA component or protein component) is bound to its cognate telomerase component (protein component or RNA component, respectively), whether said cognate telomerase component is separately labeled or is unlabeled, such as by capturing (e.g., immobilizing) bound complexes comprising said labeled telomerase component and said cognate telomerase component, and separating (e.g., by washing) unbound labeled telomerase component from a said bound complexes thereby generating a separated bound fraction and detecting the bound complexes in the separated bound fraction by determining the presence of detectable label. Agents which produce a statistically significant reduction or enhancement of noncovalent binding of telomerase RNA and protein components are thereby identified as candidate telomerase modulating agents. Agents which reduce noncovalent binding are candidate telomerase inhibitors (or antagonists), whereas agents which enhance noncovalent binding of telomerase components are candidate telomerase agonists.

Candidate telomerase modulating agents may be identified by their ability to produce a statistically significant increase or decrease in enzymatic activity of a mammalian telomerase comprising a purified telomerase protein component and a telomerase RNA component.

Candidate telomerase modulating agents may be identified by their ability to produce a statistically significant reduction or increase in transcription of a reporter polynucleotide sequence (e.g., β-galactosidase gene, luciferase gene, HPRT gene) operably linked to a transcriptional regulatory sequence of a mammalian telomerase RNA component gene, preferably a human telomerase RNA component gene, in a metabolically active mammalian cell. In a variation, an endogenous telomerase RNA component gene in a mammalian cell is targeted with a homologous targeting construct to place a reporter polynucleotide sequence in operable linkage to the upstream transcription regulatory sequence (e.g., promoter) of the endogenous telomerase RNA component gene in the chromosomal locus of the endogenous gene. In an alternative variation, an exogenous polynucleotide comprising a reporter polynucleotide is operably linked to a mammalian telomerase RNA component gene transcription regulatory region (e.g., promoter and upstream transcription factor binding sites); the exogenous polynucleotide is transferred into a mammalian cell wherein it may integrate non-homologously into a chromosomal location and/or is maintained or replicated as an episomal polynucleotide. Agents which produce a statistically significant transcriptional modulation of the reporter polynucleotide in cells treated with the agent are thereby identified as candidate mammalian telomerase modulating agents.

Compositions for identifying candidate telomerase-modulating agents typically may comprise: (1) a mammalian telomerase protein component such as may be purified from telomerase-expressing mammalian cells, preferably from primate (e.g., human) cells, and which is typically stripped of associated RNA component, if any, by treatment with RNAse or other treatment compatible with removal of RNA component and retention the capacity of the protein to reconstitute telomerase activity in the presence of cognate telomerase RNA component in suitable binding conditions, (2) a mammalian telomerase RNA component, preferably a human RNA component produced by transcription of a recombinant polynucleotide in a cell, and (3) aqueous binding conditions (e.g., physiological conditions, telomerase assay conditions), and optionally (4) a reporter polynucleotide comprising at least one replicable or extendable mammalian telomerase repeat sequence, typically complementary to the sequence of the telomere repeat complementary template portion of said RNA component, and optionally (5) nucleotides suitable for replication and/or extension of said telomere repeat sequence(s) of the reporter polynucleotide under telomere assay conditions; an agent is typically added to such a composition for evaluation by noncovalent binding and/or telomerase activity in comparison to a control composition lacking said agent.

Null alleles of mammalian telomerase RNA component genes, such as is produced by homologous gene targeting of a heterologous polynucelotide into a mammalian telomerase RNA component gene to functionally inactivate the telomerase RNA component gene, may be produced. The invention therefore also provides nonhuman knockout animals comprising a telomerase RNA component null allele, and in a variation provides nonhuman knockout animals homozygous for telomerase RNA component null alleles and substantially lacking endogenous telomerase activity resulting from a lack of endogenous telomerase RNA component. Such knockout animals are used as commercial reagents for toxicology screening, for sale to pharmaceutical research laboratories to identify or investigate telomerase-modulating agents, as pets, and as agricultural livestock among other uses.

The invention permits a method for immortalizing mammalian cells, such as desirable fermentation cells in a bioreactor or a desirable cell strain which has advantageous features as a commercial research reagent. The method comprises introducing into a mammlian cell a polynucleotide which expresses a functional telomerase RNA component which is able to form functional telomerase enzyme in the presence of telomerase protein component.

Other features and advantages of the invention will be apparent from the following description of the drawings, preferred embodiments of the invention, the examples, and the claims.

### Definitions

The term "telomerase RNA component polynucleotide" as used herein refers to a polynucleotide of at least 20 nucleotides wherein the polynucleotide comprises a segment of at least 20 nucleotides which: are at least 85 percent identical to a naturally-occurring mammalian telomerase RNA component sequence, typically a primate telomerase RNA component such as a human or monkey telomerase RNA component. Some telomerase RNA component polynucleotides having sequence variations as compared to a naturally-occurring telomerase RNA component sequence or its complement can be suitable as hybridization probes, PCR primers, LCR amplimers, mismatch RNA components, and the like.

The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotides: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length telomerase RNA component gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity.

A "comparison window", as used herein, refers to a conceptual segment of at least 25 contiguous nucleotide positions wherein a polynucleotide sequence may be compared to a reference sequence of at least 25 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman (1981) Adv. Appl. Math. 2: 482, by the homology alignment algorithm of Needleman and Wunsch (1970) J. Mol. Biol. 48: 443, by the search for similarity method of Pearson and Lipman (1988) Proc. Natl. Acad. Sci. (U.S.A.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, WI), or by inspection, and the best alignment (i.e., resulting in the highest percentage of homology over the comparison window) generated by the various methods is selected.

The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 80 percent sequence identity, preferably at least 85 percent identity and often 89 to 95 percent sequence identity, more usually at least 99 percent sequence identity as compared to a reference sequence over a comparison window of at least 20 nucleotide positions, frequently over a window of at least 30-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence, for example, as a segment of the full-length telomerase RNA component gene sequence as disclosed herein.

Specific hybridization is defined herein as the formation of hybrids between a probe polynucleotide (e.g., a polynucleotide of the invention which may include substitutions, deletion, and/or additions) and a specific target polynucleotide (e.g., a telomerase RNA component or genomic gene sequence, wherein the probe preferentially hybridizes to the specific target such that, for example, a single band corresponding to one or more of the RNA species of the telomerase RNA component gene (or specifically cleaved or processed telomerase RNA component species) can be identified on a Northern blot of RNA prepared from a suitable cell source (e.g., a somatic cell expressing telomerase RNA component). Polynucleotides of the invention which specifically hybridize to mammalian telomerase RNA component or human telomeric sequences may be prepared on the basis of the sequence data provided herein according to methods and thermodynamic principles known in the art and described in Maniatis et al., Molecular cloning: A Laboratory Manual, 2nd Ed., (1989), Cold Spring Harbor, N.Y. and Berger and Kimmel, Methods in Enzymology, Volume 152, Guide to Molecular Cloning Techniques (1987), Academic Press, Inc., San Diego, CA.

As used herein the term "suitable binding conditions" refer to aqueous conditions wherein a mammalian telomerase RNA component associates with its cognate protein component and forms an enzymatically active telomerase holoenzyme capable of catalytic replication, repair, and/or addition of telomeric repeats from a suitable template comprising telomeric repeats; such telomere repeat template may be present or absent. Often, suitable binding conditions can be physiological conditions. "Physiological conditions" as used herein refers to temperature, pH, ionic strength, viscosity, and like biochemical parameters which are compatible with a viable organism, and/or which typically exist intracellularly in a viable cultured mammalian cell, particularly conditions existing in the nucleus of said mammalian cell. For example, the intranuclear or cytoplasmic conditions in a mammalian cell grown under typical laboratory culture conditions are physiological conditions. Suitable in vitro reaction conditions for in vitro transcription cocktails are generally physiological conditions, and may be exemplified by a variety of art-known nuclear extracts. In general, in vitro physiological conditions can comprise 50-200 mM NaCl or KCl, pH 6.5-8.5, 20-45°C and 0.001-10 mM divalent cation (e.g., Mg⁺⁺, Ca⁺⁺); preferably about 150 mM NaCl or KCl, pH 7.2-7.6, 5 mM divalent cation, and often include 0.01-1.0 percent nonspecific protein (e.g., BSA). A non-ionic detergent (Tween, NP-40, Triton X-100) can often be present, usually at about 0.001 to 2%, typically 0.05-0.2% (v/v). Particular aqueous conditions may be selected by the practitioner according to conventional methods. For general guidance, the following buffered aqueous conditions may be applicable: 10-250 mM NaCl, 5-50 mM Tris HCl, pH 5-8, with optional addition of divalent cation(s) and/or metal chelators and/or nonionic detergents and/or membrane fractions and/or antifoam agents and/or scintillants.

As used herein, the terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or calorimetric methods). Various methods of labeling polypeptides and glycoproteins are known in the art and may be used. Examples of labels for polypeptides include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), enzymatic labels (e.g., horseradish peroxidase, β-galactosidase, luciferase, alkaline phosphatase), biotinyl groups, predetermined polypeptide epitopes recognized by a secondary reporter (e.g., leucine zipper pair sequences, binding sites for secondary antibodies, transcriptional activator polypeptide, metal binding domains, epitope tags). In some embodiments, labels are attached by spacer arms of various lengths to reduce potential steric hindrance.

As used herein, the term "statistically significant" means a result (i.e., an assay readout) that generally is at least two standard deviations above or below the mean of at least three separate determinations of a control assay readout and/or which is statistically significant as determined by Student's t-test or other art-accepted measure of statistical significance.

As used herein the terms "pathognomonic concentration", "pathognomonic amount", and "pathognomonic hybridization pattern" refer to a concentration, amount, or localization pattern, respectively, of a telomerase RNA component in a sample, that indicates the presence of a pathological (e.g., neoplastic, senescent, immunodeficient, neurodegenerative, inflammatory, etc.) condition or a predisposition to developing a neoplastic disease, such as carcinoma, sarcoma, or leukemia. A pathognomonic amount is an amount of telomerase RNA component in a cell or cellular sample that falls outside the range of normal clinical values that is established by prospective and/or retrospective statistical clinical studies. Generally, an individual having a neoplastic disease (e.g., carcinoma, sarcoma, or leukemia) will exhibit an amount of telomerase RNA component in a cell or tissue sample that is outside the range of concentrations that characterize normal, undiseased individuals; typically the pathognomonic concentration is at least about one standard deviation outside the mean normal value, more usually it is at least about two standard deviations or more above the mean normal value. However, essentially all clinical diagnostic tests produce some percentage of false positives and false negatives. The sensitivity and selectivity of the diagnostic assay must be sufficient to satisfy the diagnostic objective and any relevant regulatory requirements. In general, the diagnostic methods of the invention are used to identify individuals as disease candidates, providing an additional parameter in a differential diagnosis of disease made by a competent health professional. As used herein, the term "disease allele" refers to an allele of a gene which is capable of producing a recognizable disease. A disease allele may be dominant or recessive and may produce disease directly or when present in combination with a specific genetic background or pre-existing pathological condition. A disease allele may be present in the gene pool or may be generated de novo in an individual by somatic mutation.

The term "antineoplastic agent" is used herein to refer to agents that have the functional property of inhibiting a development or progression of a neoplasm in a human, often including inhibition of metastasis or metastatic potential.

As used herein, the term "operably linked" refers to a linkage of polynucleotide elements in a functional relationship. A nucleic acid is "operably linked" when it is placed into a functional relationship with another nucleic acid sequence. For instance, a promoter or enhancer is operably linked to a coding sequence if it affects the transcription of the coding sequence. Operably linked means that the DNA sequences being linked are typically contiguous and, where necessary to join two protein coding regions, contiguous and in reading frame. However, since enhancers generally function when separated from the promoter by several kilobases and intronic sequences may be of variable lengths, some polynucleotide elements may be operably linked but not contiguous. A structural gene (e.g., a HSV tk gene) which is operably linked to a polynucleotide sequence corresponding to a transcriptional regulatory sequence of an endogenous gene is generally expressed in substantially the same temporal and cell type-specific pattern as is the naturally-occurring gene.

As used herein, the term "transcriptional unit" or "transcriptional complex" refers to a polynucleotide sequence that comprises a structural gene (exons), a cis-acting linked promoter and other cis-acting sequences necessary for efficient transcription of the structural sequences, distal regulatory elements necessary for appropriate tissue-specific and developmental transcription of the structural sequences, and additional cis sequences important for efficient transcription and translation (e.g., polyadenylation site, mRNA stability controlling sequences).

The term "transcriptional modulation" is used herein to refer to the capacity to either enhance transcription or inhibit transcription of a structural sequence linked in cis; such enhancement or inhibition may be contingent on the occurrence of a specific event, such as stimulation with an inducer and/or may only be manifest in certain cell types.

As used herein; the term "transcription regulatory region" refers to a DNA sequence comprising a functional promoter and any associated transcription elements (e.g., enhancer, CCAAT box, TATA box, SP1 site, etc.) which are essential for transcription of a polynucleotide sequence which is operably linked to the transcription regulatory region.

The term "null allele" as used herein means that a gene locus comprises at least one mutation or structural alteration such that the null allele is substantially incapable of directing the efficient expression of a functional gene product. A "knockout cell" is a cell having at least one null allele of an endogenous gene, typically being homozygous for the null allele. Thus, for example, a knockout cell may be homozygous for null alleles at the telomerase RNA component locus, so that the knockout cell is substantially incapable of expressing a functional telomerase RNA component.

### Brief Description of the Drawings

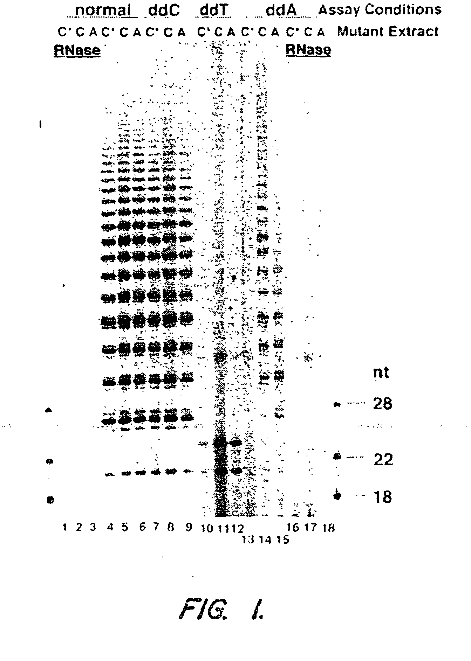

Fig. 1. Telomerase activity from cells expressing template-mutated TRC3 DEAE-sepharose fractionated extracts from mutant TRC3-expressing stable transformants were assayed for telomerase activity using conventional assays under various reaction conditions. Extracts from cells expressing MuC+17 TRC3 (lanes 1, 4, 7, 10, 13, 16 labeled C*), MuC TRC-3 (lanes 2, 5, 8, 11, 14, 17 labeled C), or MuA TRC3 (lanes 3, 6, 9, 12, 15, 18 labeled A) were assayed under normal reaction conditions (lanes 1-6), normal plus 0.5 mM ddCTP (lanes 7-9), normal minus dTTP plus 0.5 mM ddTTP (lanes 10-12), or normal minus dATP plus 0.5 mM ddATP (lanes 13-18). Assay reactions in lanes 1-9 contained 8 µM total dGTP, 1 µM of which was ³²P-dGTP (800 Ci/mmol). To facilitate mutant telomerase detection, assay reactions in lanes 10-18 contained 8 µM total dGTP, 2 µM of which was ³²P-dGTP (800 Ci/mmol). Extracts were treated with DNase-free RNase (25 µg/ml for 10 min at 30°C) prior to telomerase assays (lanes 1-3, 16-18). Flanking lanes contain DNA markers with sizes in nucleotides (nt) as indicated.

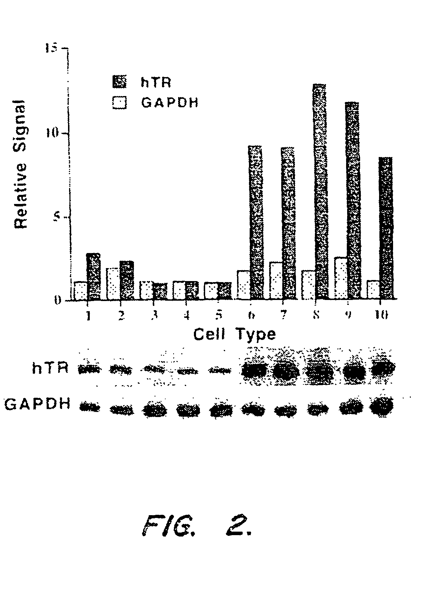

Fig. 2. The steady-state level of telomerase RNA component (hTR) and GAPDH RNA was determined using quantitative RT-PCR. Controls show that all PCR quantitations were in the linear range up to 25 cycles. RT-PCR was analyzed for five normal telomerase-negative cell lines (1-5) and five tumor telomerase-positive cells lines (6-10): 1) Primary fetal lung; 2) Primary fetal hand skin; 3) Adult primary prostate; 4) Primary sinovial fibroblasts; 5) Foreskin fibroblasts; 6) Melanoma LOX; 7) Leukemia U251; 8) NCIH23 lung carcinoma; 9) Colon tumor SW620; 10) Breast tumor MCF7. PCR products were labeled with ³²P, resolved on a 6% PAGE, and quantified using a PhosphorImager. The relative transcription is expressed in arbitrary units.

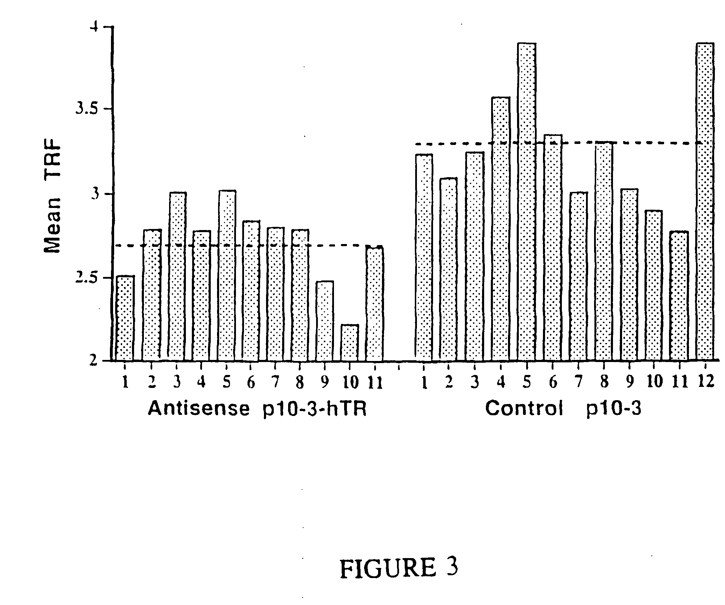

Fig. 3. Mean TRF length in telomerase RNA component antisense and vector control cells. HeTe7 cells that stably express 10-3-hTR antisense or vector control were selected in hygromycin and puromycin media and harvested at 23 PDL post transfection. Nuclear DNA was purified, cut with *Hinf*I and *Rsa*I, and run on a 0.5% agarose gel. The DNA was probed in the gel with a (TTAGGG)₃ oligonucleotide to label the telomeric terminal restriction fragments (TRF). The gel was scanned with a Molecular Dynamic's PhosphorImager and the mean TRF quantitated as described (Allsopp et al. (1992) Proc. Natl. Acad. Sci. (USA) 89: 10114). The dashed lines indicate the average mean TRF for the antisense and control groups.

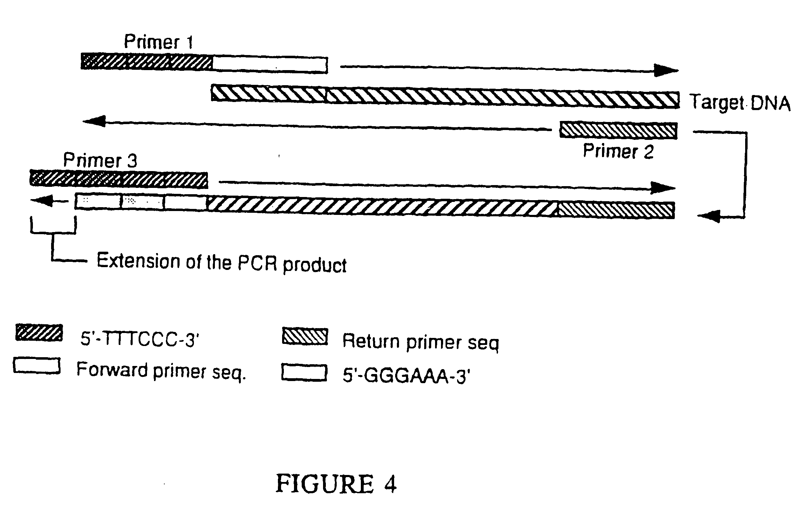

Fig. 4 Schematic representation of in situ PCR method.

### Description of the Preferred Embodiments

The present invention provides methods, reagents, genetically modified animals and cells, and pharmaceutical compositions relating to the ribonucleoprotein human telomerase.

The nomenclature used hereafter and the laboratory procedures in cell culture, molecular genetics, and nucleic acid chemistry and hybridization described below may involve well known and commonly employed procedures in the art. Standard techniques are used for recombinant nucleic acid methods, polynucleotide synthesis, and microbial culture and transformation (e.g., electroporation, lipofection). The techniques and procedures are generally performed according to conventional methods in the art and various general references (see, generally, Sambrook et al. Molecular Cloning: A Laboratory Manual, 2d ed. (1989) Cold Spring Harbor Laboratory Press, Cold Spring Harbor, N.Y. which are provided throughout this document.

Oligonucleotides can be synthesized on an Applied Bio Systems oligonucleotide synthesizer according to specifications provided by the manufacturer.

Methods for PCR amplification are described in the art (PCR Technology: Principles and Applications for DNA Amplification ed. HA Erlich, Freeman Press, New York, NY (1992); PCR Protocols: A Guide to Methods and Applications, eds. Innis, Gelfland, Snisky, and White, Academic Press, San Diego, CA (1990); Mattila et al. (1991) Nucleic Acids Res. 19: 4967; Eckert, K.A. and Kunkel, T.A. (1991) PCR Methods and Applications 1: 17; PCR, eds. McPherson, Quirkes, and Taylor, IRL Press, Oxford; and U.S. Patent 4,683,202.

### Overview

The invention in part arises out of the cloning and isolation of the RNA component of human telomerase and the gene for that RNA component, including associated transcriptional control elements. The nucleotide sequence of the RNA component of human telomerase is shown below. For convenience, the sequence is shown using the standard abbreviations for ribonucleotides (A is riboadenine, G is riboguanine, C is ribocytidine, and U is uridine). Those of skill in the art recognize that the sequence shown below also shows the sequence of the cDNA, in which the ribonucleotides are replaced by deoxyribonucleotides (with uridine being replaced by thymidine). 
50
GGGUUGCGGAGGGAGGGUGGCCUGGAGGGGUGGUGGCCAUUUUUUGUC
100
UAACCCUAACUGAGAAGGGCGUAGGCGCCGUGCUUUUGCUCCCCGCGC
150
UGUUUUUCUCGCUGACUUUCAGCGGGCGGAAAAGCCUCGGCCUGCCGCCU
200
UCCACCGUUCAUUCUAGAGCAAACAAAAAAUGUCAGCUGCUGGCCCGUUC
250
GCCCCUCCCGGACCUGCGGCGGGUCGCUGCCCAGCCCCCGAACCCCGCC
300
UGGAGGCCGCGGUCGGCCGGGGCUUCUCCGGAGGCACCCACUGCCACCGC
350
GAAGAGUUGGGCUCUGUCAGCCGCGGGUCUCUCGGGGGCGAGGGCGAGGU
400
UCACCGUUUCAGGCCGCAGGAAGAGGAACGGAGCGAGUCCCGCGCGGC
450
GCGAUUCCCUGAGCUAUGGGACGUGCACCCAGGACUCGGCUCACACAUGC
500
AGUUCGCUUUCCUGUUGGUGGGGGGAACGCCGAUCGUGCGCAUCCGUCAC
 
' ' ' ' ' 550
CCCUCGCCGGCAGUGGGGCUUGUGAACCCCCAAACCUGACUGACUGGGC
' 559
CAGUGUGCU
 The sequence above is shown in the 5'-3' direction and is numbered for reference. The template sequence of the RNA component is believed to be located within the region defined by nucleotides 50-60 (5'-CUAACCCUAAC-3'), which is complementary to a telomeric sequence composed of about one-and-two-thirds telomeric repeat units.

This sequence was derived from cDNA clones and from a genomic clone of the RNA component. When the RNA component is first transcribed from the corresponding gene, at least some of the RNA transcripts produced are much longer than the ⁻560 nucleotide sequence shown above and in fact may comprise more than 1000 nucleotides. However, a fully functional telomerase molecule can be assembled from transcripts consisting of the ⁻560 nucleotide sequence shown above. The 3'-end of the RNA component in native telomerase is believed to lie within the region defined by nucleotides 514-559 in the sequence above; one analysis indicates that the 3'-end may be the U residue at nucleotide 538. Recombinant RNA component molecules comprising less than nucleotides 1-559 of the sequence shown above can also be used to prepare active telomerase.

A genomic clone was identified and isolated from a genomic library of human DNA inserted into a lambda vector FIXII. The genomic clone, comprising the RNA component gene sequences, contained an ⁻15 kb insert and was designated clone 28-1. The gene is localized on the distal end of the q arm of chromosome 3. The sequence information obtained from a SauIIIA1 restriction enzyme recognition site at one end of the ⁻15kb insert to an internal HindIII restriction enzyme recognition site, which comprises all of the mature RNA component sequence as well as transcription control elements of the RNA component gene, of lambda clone 28-1 is shown below using the standard deoxyribonucleotide abbreviations and depicted in the 5'-3' direction. 
50
GATCAGTTAGAAAGTTACTAGTCCACATATAAAGTGCCAAGTCTTGTACT
100
CAAGATTATAAGCAATAGGAATTTAAAAAAAGAAATTATGAAAACTGACA
150
AGATTTAGTGCCTACTTAGATATGAAGGGGAAAGAAGGGTTTGAGATAAT
200
GTGGGATGCTAAGAGAATGGTGGTAGTGTTGACATATAACTCAAAGCATT
250
TAGCATCTACTCTATGTAAGGTACTGTGCTAAGTGCAATAGTGCTAAAAA
300
CAGGAGTCAGATTCTGTCCGTAAAAAACTTTACAACCTGGCAGATGCTAT
350
GAAAGAAAAAGGGGATGGAGAGAGAGAAGGAGGGAGAGAGATGGAGAGG
400
GAGATATTTTACTTTTCTTTCAGATCGAGGACCGACAGCGACAACTCCAC
450
GGAGTTTATCTAACTGAATACGAGTAAAACTTTTAAGATCATCCTGTCAT
500
TTATATGTAAAACTGCACTATACTGGCCATTATAAAAATTCGCGGCCGGG
550
TGCGGTGGCTCATACCTGTAATCCCAGCACTTTGGGAGGCCGAAGCGGGT
600
GGATCACTTGAGCCCTGGCGTTCGAGACCAGCCTGGGCAACATGGTGAAA
650
CCCCCGTCTCTACTAAAAACACAAAAACTAGCTGGGCGTGGTGGCAGGCG
700
CCTGTAATCCCAGCTACTCAGGAGGCTGAGACACGAGAATCGCTTGAACC
750
CGGGAGCAGAGGTTGCAGTGAGCCGAGATCACGCCACTAGACTCCATCCA
800
GCCTGGGCGAAAGAGCAAGACTCCGTCTCAAAAAAAAAAATCGTTACAAT
850
TTATGGTGGATTACTCCCCTCTTTTTACCTCATCAAGACACAGCACTACT
900
TTAAAGCAAAGTCAATGATTGAAACGCCTTTCTTTCCTAATAAAAGGGAG
950
ATTCAGTCCTTAAGATTAATAATGTAGTAGTTACACTTGATTAAAGCCAT
1000
CCTCTGCTCAAGGAGAGGCTGGAGAAGGCATTCTAAGGAGAAGGGGGCAG
1050
GGTAGGAACTCGGACGCATCCCACTGAGCCGAGACAAGATTCTGCTGTAG
1100
TCAGTGCTGCCTGGGAATCTATTTTCACAAAGTTCTCCAAAAAATGTGAT
1150
GATCAAAACTAGGAATTAGTGTTCTGTGTCTTAGGCCCTAAAATCTTCCT
1200
GTGAATTCCATTTTTAAGGTAGTCGAGGTGAACCGCGTCTGGTCTGCAGA
1250
GGATAGAAAAAAGGCCCTCTGATACCTCAAGTTAGTTTCACCTTTAAAGA
1300
AGGTCGGAAGTAAAGACGCAAAGCCTTTCCCGGACGTGCGGAAGGGCAAC
1350
GTCCTTCCTCATGGCCGGAAATGGAACTTTAATTTCCCGTTCCCCCCAAC
1400
CAGCCCGCCCGAGAGAGTGACTCTCACGAGAGCCGCGAGAGTCAGCTTGG
1450
 
CCAATCCGTGCGGTCGGCGGCCGCTCCCTTTATAAGCCGACTCGCCCGGC
1500
AGCGCACCGGGTTGCGGAGGGAGGGTGGGCCTGGGAGGGGTGGTGGCCAT
1550
TTTTTGTCTAACCCTAACTGAGAAGGGCGTAGGCGCCGTGCTTTTGCTCC
1600
CCGCGCGCTGTTTTTCTCGCTGACTTTCAGCGGGCGGAAAAGCCTCGGCC
1650
TGCCGCCTTCCACCGTTCATTCTAGAGCAAACAAAAAATGTCAGCTGCTG
1700
GCCCGTTCGCCCCTCCCGGGACCTGCGGCGGGTCGCTGCCCAGCCCCCGA
1750
ACCCCGCCTGGAGGCCGCGGTCGGCCGGGGCTTCTCCGGAGGCACCCACT
1800
GCCACCGCGAAGAGTTGGGCTCTGTCAGCCGCGGGTCTCTCGGGGGCGAG
1850
GGCGAGGTTCACCGTTTCAGGCCGCAGGAAGAGGAACGGAGCGAGTCCCG
1900
CGCGCGGCGCGATTCCCTGAGCTATGGGACGTGCACCCAGGACTCGGCTC
1950
ACACATGCAGTTCGCTTTCCTGTTGGTGGGGGGAACGCCGATCGTGCGCA
2000
TCCGTCACCCCTCGCCGGCAGTGGGGGCTTGTGAACCCCCAAACCTGACT
2050
GACTGGGCCAGTGTGCTGCAAATTGGCAGGAGACGTGAAGGCACCTCCAA
2100
AGTCGGCCAAAATGAATGGGCAGTGAGCCGGGGTTGCCTGGAGCCGTTCC
2150
TGCGTGGGTTCTCCCGTCTTCCGCTTTTTGTTGCCTTTTATGGTTGTATT
2200
ACAACTTAGTTCCTGCTCTGCAGATTTTGTTGAGGTTTTTGCTTCTCCCA
2250
AGGTAGATCTCGACCAGTCCCTCAACGGGGTGTGGGAGAACAGTCATTT
2300
TTTTTTGAGAGATCATTTAACATTTAATGAATATTTAATTAGAAGATCTA
2350
AATGAACATTGGAAATTGTGTTCCTTTAATGGTCATCGGTTTATGCCAGA
2400
GGTTAGAAGTTTCTTTTTTGAAAAATTAGACCTTGGCGATGACCTTGAGC
2425
AGTAGGATATAACCCCCACAAGCTT
 The RNA component sequence begins at base 1459. A variety of transcription control elements are identified in the sequence. An A/T Box consensus sequence is found at nucleotides 1431-1436; PSE consensus sequences are found at nucleotides 1406-1414 as well as nucleotides 1508-1526; a CAAT box consensus sequence is found at nucleotides 1399-1406; an SP1 consensus sequence is found at nucleotides 1354-1359; and a β/γ-interferon response element consensus sequence is found at nucleotides 1234-1245. The steady state transcription of human telomerase RNA component gene in human cells such as HT1080 (telomerase expressing) is substantially unchanged when sequences upstream of nucleotide 1159 are deleted in vectors which are stably transfected into the cells.

DNA from squirrel monkey, which is believed to be among the most genetically divergent non-human primate with respect to humans, comprises a telomerase RNA component gene which is amplifiable with PCR primers consisting of sequences which correspond to or are complementary to the disclosed human telomerase RNA component polynucleotide sequence. Other nonhuman primates are believed to possess telomerase RNA component genes which are also amplifiable with PCR primers derived from the sequence of the human telomerase RNA component gene.

### Telomerase RNA Component Polynucleotides

Disclosure of the sequences for mammalian telomerase RNA component and its gene, as shown supra for human telomerase makes possible the construction of isolated polynucleotides that comprise a sequence of at least 15 contiguous nucleotides, typically at least 20 to 25 contiguous polynucleotides, which are substantially identical to a mammalian telomerase RNA component sequence or mammalian RNA component gene sequence. Further, the mammalian telomerase RNA component (and gene) sequences make possible the construction of nucleic acid hybridization probes and PCR primers that can be used to detect RNA and DNA sequences of the cognate telomerase RNA component and/or gene in a cell, cellular sample, tissue section, reaction vessel, hybridization membrane, or the like.

Polynucleotides comprising mammalian telomerase RNA component sequences may include sequences that facilitate transcription (expression sequences), RNA stabilizing sequences, and the like. General principles for construction of such polynucleotides in view of the presently disclosed sequence information and guidance and direction of the invention is well known in the art and is described further in Maniatis et al., Molecular Cloning: A Laboratory Manual, 2nd Ed. (1989), Cold Spring Harbor, N.Y. For example, but not for limitation, such polynucleotides can include a promoter and, optionally, an enhancer for use in eukaryotic expression hosts, and, optionally, sequences necessary for replication of a vector. A typical eukaryotic expression cassette will include a polynucleotide sequence which, when transcribed, produces a mammalian telomerase RNA component transcript; such polynucleotide sequence is linked downstream (i.e., in transcription orientation 5' to 3' of a suitable promoter, such as the HSV *tk* promoter or the *pgk* (phosphoglycerate kinase) promoter, optionally linked to an enhancer.

Additionally, where expression of a functional telomerase RNA component is not desired, polynucleotides of this invention need not transcribe a functional telomerase RNA component transcript. Polynucleotides of this invention may serve as hybridization probes and/or PCR primers (amplimers) and/or LCR oligomers for detecting telomerase RNA component RNA or DNA sequences.

Alternatively, polynucleotides of this invention may serve as hybridization probes or primers for detecting RNA or DNA sequences of related genes, or telomerase RNA component gene in related species, typically mammalian species. For such hybridization and PCR applications, the polynucleotides of the invention need not transcribe a functional telomerase RNA component. Thus, polynucleotides of the invention may contain substantial deletions, additions, nucleotide substitutions and/or transpositions, so long as specific hybridization or specific amplification to a mammalian telomerase RNA component sequence is retained.

Genomic or cDNA clones of mammalian telomerase RNA component and corresponding gene sequences may be isolated from clone libraries (e.g., available from Clontech, Palo Alto, CA) using hybridization probes designed on the basis of the nucleotide sequences disclosed herein and using conventional hybridization screening methods (e.g., Benton WD and Davis RW (1977) Science 196: 180; Goodspeed et al. (1989) Gene 76: 1). Where a cDNA clone is desired, clone libraries containing cDNA derived from somatic cell RNA or other telomerase RNA component-expressing cell RNA are preferred. Alternatively, synthetic polynucleotide sequences corresponding to all or part of the sequences disclosed herein may be constructed by chemical synthesis of oligonucleotides. Additionally, polymerase chain reaction (PCR) using primers based on the sequence data disclosed herein may be used to amplify DNA fragments from genomic DNA, RNA pools, or from cDNA clone libraries. U.S. Patents 4,683,195 and 4,683,202 describe the PCR method.

Such polynucleotides have a variety of uses, including as telomerase RNA component probes, as templates for producing functional or nonfunctional telomerase RNA component in cells, as commercial diagnostic reagents for standardizing a telomerase RNA component detection assay, as gene therapy polynucleotides for administering to an animal; such polynucleotides can also be used as foodstuffs, combustible energy sources, UV-absorbing sunscreen agents, and viscosity-enhancing solutes, among other uses.

The plasmids described herein that were constructed during the cloning of the RNA component of human telomerase and the gene for the RNA component are important aspects of the present invention. These plasmids can be used to produce the RNA component of, as well as the gene for, human telomerase in substantially pure form, yet another important aspect of the present invention. In addition, those of skill in the art recognize that a variety of other plasmids, as well as non-plasmid nucleic acids in substantially pure form, that comprise all or at least a useful portion of the nucleotide sequence of the RNA component of human telomerase are useful materials provided by the present invention.

As a general point regarding the nucleic acids and preparations containing the same of the invention, those of skill in the art recognize that the nucleic acids of the invention include both DNA and RNA molecules, as well as synthetic, non-naturally occurring analogues of the same, and heteropolymers of deoxyribonucleotides, ribonucleotides, and/or analogues of either. The particular composition of a nucleic acid or nucleic acid analogue of the invention will depend upon the purpose for which the material will be used and the environment(s) in which the material will be placed. Modified or synthetic, non-naturally occurring nucleotides, have been designed to serve a variety of purposes and to remain stable in a variety of environments, such as those in which nucleases are present, as is well known in the art. Modified or synthetic non-naturally occurring nucleotides, as compared to the naturally occurring ribo- or deoxyribonucleotides, may differ with respect to the carbohydrate (sugar), phosphate linkage, or base portions, of the nucleotide, or may even contain a non-nucleotide base (or no base at all) in some cases. See, e.g., Arnold et al., PCT patent Publication No. WO 89/02439, entitled "Non-nucleotide Linking Reagents for Nucleotide Probes" incorporated herein by reference. Just as the nucleic acids of the invention can comprise a wide variety of nucleotides, so too can those nucleic acids serve a wide variety of useful functions.

### Isolation of Cognate Genes

As indicated by the foregoing description, access to purified nucleic acids comprising the sequence of the RNA component of human telomerase provides valuable diagnostic and therapeutic methods and reagents, as well as other important benefits. One important benefit of the present invention is that the methods and reagents of the invention can be used to isolate the RNA component and genes for the RNA component of telomerase from any mammalian species that has an RNA component substantially homologous to the human RNA component of the present invention. The phrase "substantially homologous" refers to that degree of homology required for specific hybridization of an oligonucleotide or nucleic acid sequence of the human RNA component to a nucleic acid sequence of an RNA component sequence of another mammalian species. Given such substantial homology, those of ordinary skill in the art can use the nucleic acids and oligonucleotide primers and probes of the invention to identify and isolate substantially homologous sequences.

For instance, one can probe a genomic or cDNA library to detect homologous sequences. One can also use primers corresponding to regions of the RNA component sequence and PCR amplification under low or moderate stringency conditions to amplify a specific homologous nucleic acid sequence from preparations of RNA or DNA from a mammalian species. By using these and other similar techniques, those of ordinary skill can readily isolate not only variant RNA component nucleic acids from human cells but also homologous RNA component nucleic acids from other mammalian cells, such as cells from primates, from mammals of veterinary interest, i.e., cattle, sheep, horse, dogs, and cats, and from rodents, i.e., rats, mice, and hamsters. In turn, these nucleic acids can be used to prepare transgenic animals of great value for screening and testing of pharmaceuticals that regulate telomerase activity. For instance, by using a plasmid of the invention, one can "knock out" the RNA component gene or replace the natural RNA component gene with a recombinant inducible gene in a mus spretus embryonic stem cell and then generate a transgenic mouse that will be useful as a model or test system for the study of age- or senescence-related disease. Example 9, below, illustrates how such methodology has been used to identify and isolate RNA component sequences of primates.

Other mammalian homologs of the human and monkey telomerase RNA components and/or the cognate genes can be identified and isolated by screening a suitable nonhuman mammal genomic or cDNA clone library, such as derived from a mouse, rat, rabbit, guinea pig, hamster, canine, bovine, ovine, lupine, porcine, or other genomic or cDNA library in a suitable vector, such as yeast artificial chromosomes, cosmids, or bacteriophage λ (e.g., λ Charon 35), with a polynucleotide probe comprising a sequence of about at least 20 contiguous nucleotides (or their complement) of a human or monkey telomerase RNA component or gene polynucleotide sequence. Typically, hybridization and washing conditions are performed at high stringency according to conventional hybridization procedures. Positive clones are isolated and sequenced. For illustration and not for limitation, a full-length polynucleotide corresponding to the 559 nucleotide sequence of the human telomerase RNA component may be labeled and used as a hybridization probe to isolate genomic clones from a non-human genomic clone library in λEMBL4 or λGEM11 (Promega Corporation, Madison, Wisconsin); typical hybridization conditions for screening plaque lifts (Benton and Davis (1978) Science 196: 180; Dunn et al. (1989) J. Biol. Chem. 264: 13057) can be: 50% formamide, 5 x SSC or SSPE, 1-5 x Denhardt's solution, 0.1-1% SDS, 100-200 µg sheared heterologous DNA or tRNA, 0-10% dextran sulfate, 1 x10⁵ to 1 x 10⁷ cpm/ml of denatured probe with a specific activity of about 1 x 10⁸ cpm/µg, and incubation at 42°C-37°C for about 6-36 hours. Prehybridization conditions are essentially identical except that probe is not included and incubation time is typically reduced. Washing conditions are typically 1-3 x SSC, 0.1-1% SDS, 45-70°C with change of wash solution at about 5-30 minutes. For isolating non-human telomerase RNA component polynucleotides with a human telomerase RNA component polynucleotide probe, it is often preferred to hybridize at a lower stringency, such as approximately 39°C and to wash sequentially at the following step temperatures: room temperature, 37°C, 39°C, 42°C, 45°C, 50°C, 55°C, 60°C, 65°C, and 70°C, stopping after each step and monitoring the background probe signal (and optionally detecting signal by autoradiogram and/or phosphor imaging, if radiolabeled probe is used) and terminating the washing steps when suitable signal/noise ratio is achieved, as determined empirically.

Polynucleotides comprising sequences of approximately at least 30-50 nucleotides, preferably at least 100 nucleotides, corresponding to or complementary to the nucleotide sequences shown herein for the human and monkey telomerase RNA component sequences can serve as PCR primers and/or hybridization probes for identifying and isolating germline genes corresponding to the disclosed gene and RNA component sequences. Such germline genes may be isolated by various methods conventional in the art, including, but not limited to, by hybridization screening of genomic libraries in bacteriophage X or cosmid libraries, or by PCR amplification of genomic sequences using primers derived from the sequences disclosed herein. Human genomic libraries are publicly available or may be constructed de novo from human DNA.

It is apparent to one of skill in the art that nucleotide substitutions, deletions, and additions may be incorporated into the polynucleotides of the invention. Nucleotide sequence variation may result from sequence polymorphisms of various alleles and the like. However, such nucleotide substitutions, deletions, and additions should not substantially disrupt the ability of the polynucleotide to hybridize to one of the full-length human or monkey telomerase RNA component polynucleotide sequences shown herein under hybridization conditions that are sufficiently stringent to result in specific hybridization.

Mammalian telomerase RNA component polynucleotides may be short oligonucleotides (e.g., 20-100 bases long), such as for use as hybridization probes and PCR (or LCR) primers. The polynucleotide sequences may also comprise part of a larger polynucleotide (e.g., a cloning vector comprising a telomerase RNA component clone) and may be fused, by polynucleotide linkage, with another polynucleotide sequence. Typically, telomerase RNA component polynucleotides comprise at least 25 consecutive nucleotides which are substantially identical to a naturally-occurring telomerase RNA component or gene sequence, more usually telomerase RNA component polynucleotides comprise at least 50 to 100 consecutive nucleotides which are substantially identical to a naturally-occurring mammalian telomerase RNA component sequence. However, it will be recognized by those of skill that the minimum length of a telomerase RNA component polynucleotide required for specific hybridization to a telomerase RNA component target sequence will depend on several factors: G/C content, positioning of mismatched bases (if any), degree of uniqueness of the sequence as compared to the population of target polynucleotides, and chemical nature of the polynucleotide (e.g., methylphosphonate backbone, polyamide nucleic acid, phosphorothiolate, etc.), among others.

If desired, PCR amplimers for amplifying substantially full-length cDNA copies may be selected at the discretion of the practitioner. Similarly, amplimers to amplify portions of the telomerase RNA component gene (monkey or human) may be selected.

Each of these sequences may be used as hybridization probes or PCR amplimers to detect the presence of telomerase RNA component, for example to diagnose a neoplastic disease characterized by the presence of an elevated or reduced telomerase RNA component level in cells, or to perform tissue typing (i.e., identify tissues characterized by the expression of telomerase RNA component), and the like. The sequences may also be used for detecting genomic telomerase RNA component gene sequences in a DNA sample, such as for forensic DNA analysis (e.g., by RFLP analysis, PCR product length(s) distribution, etc.) or for diagnosis of diseases characterized by amplification and/or rearrangements of the telomerase RNA component gene.

For example and not limitation, the following pair of oligonucleotide primers can be used to amplify telomerase RNA component polynucleotide sequences (e.g., cDNA) or as hybridization probes (e.g., as biotinylated or end-labeled oligonucleotide probes): 5'-AGCACACTGGCCCAGTCAGTCAGGTTTG-3' and 5'-GGGTTGCGGAGGGAGGGTGGGCCTGGGA-3'. Other suitable PCR primers, LCR primers, hybridization probes, and primers, and the like are apparent to those of skill in the art in view of the telomerase RNA component sequences disclosed herein and which can be obtained therewith. Human telomerase RNA component polynucleotides and their complements may serve as hybridization probes or primers for detecting RNA or DNA sequences of mammalian telomerase RNA component. For such hybridization and PCR applications may contain substantial deletions, additions, nucleotide substitutions and/or transpositions, so long as specific hybridization or specific amplification of a human telomerase RNA component sequence is retained. However, such nucleotide substitutions, deletions, and additions should not substantially disrupt the ability of the polynucleotide to hybridize to a telomerase RNA component or gene sequence under hybridization conditions that are sufficiently stringent to result in specific hybridization.

For example and not limitation, a human telomerase RNA component polynucleotide can comprise the sequence from nucleotide 48 to nucleotide 209, which is believed sufficient for reconstituting human telomerase holoenzyme in the presence of telomerase protein component: 
5'-UCUAACCCUAACUGAGAAGGGCGUAGGCGCCGUGCUUUUGCUCCCCGCGCGC
UGUUUUUCUCGCUGACUUUCAGCGGGCGGAAAAGCCUCGGCCUGCCGCCUUCCAC
CGUUCAUUCUAGAGCAAACAAAAAAUGUCAGCUGCUGGCCCGUUCGCCCCUCCC-3'
 or as DNA: 
5'-TCTAACCCTAACTGAGAAGGGCGTAGGCGCCGTGCTTTTGCTCCCCGCGCGC
TGTTTTTCTCGCTGACTTTCAGCGGGCGGAAAAGCCTCGGCCTGCCGCCTTCCAC
CGTTCATTCTAGAGCAAACAAAAAATGTCAGCTGCTGGCCCGTTCGCCCCTCCC-3'.
 Also, a human telomerase RNA component polynucleotide can comprise or consist of nucleotides 1-559, and may include terminal additions of other nucleotides or nucleotide sequences. A template-scrambled variant consisting of nucleotides 48-209, but wherein the telomere repeat template sequence is altered, is able to compete with a truncated RNA component consisting of wild-type sequence 48-209 for binding to telomerase protein component and reconstituting telomerase holoenzyme.

Structural analysis of the human telomerase RNA component indicates regions having a propensity to form secondary structures, such as hairpin loops. For example, the region from approximately nucleotide 200 to nucleotide 350 of the human telomerase RNA component has a substantial hairpin loop character. Other portions of the telomerase RNA component have significant secondary structure character as well. In view of this noted secondary structure, other nucleotide sequences which are predicted by computer analysis to adopt similar secondary structure formations can be substituted by skilled artisans. Although a variety of computer programs are suitable for determining nucleotide sequences which adopt substantially equivalent secondary structures, the UWGCG Sequence Analysis Software Package programs FOLD, SQUIGGLES, CIRCLES, DOMES, NOUNTAINS, and STEMLOOP, and the like can be used. Similarly, non-nucleotide structural mimetics, such as peptide nucleic acids, and the like, can be designed by molecular modeling programs as mimetics of the characteristic secondary structure of the human telomerase RNA component region(s) desired to be mimicked. Such structural mimetics can be used therapeutically or for various uses (e.g., competitive antagonist, etc.).

### Antisense

One especially useful type of nucleic acid of the invention is an antisense oligonucleotide that can be used in vivo or in vitro to inhibit the activity of human telomerase. Antisense oligonucleotides comprise a specific sequence of from about 10 to about 25 to 200 or more (i.e., large enough to form a stable duplex but small enough, depending on the mode of delivery, to administer in vivo, if desired) nucleotides complementary to a specific sequence of nucleotides in the RNA component of human telomerase. The mechanism of action of such oligonucleotides can involve binding of the RNA component either to prevent assembly of the functional ribonucleoprotein telomerase, to prevent the RNA component from serving as a template for telomeric DNA synthesis, to destabilize the telomerase RNA component and reduce its half-life, and/or to inhibit transcription of the telomerase RNA component gene.

Illustrative antisense oligonucleotides of the invention that serve to inhibit telomerase activity in vivo and/or in vitro include the oligonucleotides mentioned above in connection with the tests to determine whether clone pGRN7 comprised the cDNA for the RNA component of human telomerase. Three such oligonucleotides, as noted above, were used to demonstrate inhibition of telomerase activity in vitro. The sequence of each of these oligonucleotides is shown below. T3 5'-CTCAGTTAGGGTTAGACAAA-3' P3    5'-CGCCCTTCTCAGTTAGGGTTAG-3' TA3 5'-GGCGCCTACGCCCTTCTCAGTT-3' These oligonucleotides can also be used to inhibit telomerase activity in human cells.

Those of skill in the art will recognize that the present invention provides a wide variety of antisense oligonucleotides able to inhibit telomerase activity. Another useful antisense oligonucleotides of the invention is oligonucleotide Tel-AU, which has the sequence 5'-CAGGCCCACCCTCCGCAACC-3', and which, like any of the antisense oligonucleotides of the invention, can be synthesized using phosphorothioate nucleotides, chiral-methyl phoshponates, naturally occurring nucleotides, or mixtures of the same to impart stability and the desired Tₘ. Those of skill in the art recognize that a wide variety of modified nucleotide analogues, such as O-methyl ribonucleotides, phosphorothioate nucleotides, and methyl phosphonate nucleotides, can be used to produce nucleic acids of the invention with more desired properties (i.e., nuclease-resistant, tighter-binding, etc.) than those produced using naturally occurring nucleotides. Other techniques for rendering oligonucleotides nuclease-resistant include those described in PCT patent publication No. 94/12633.

Additional embodiments directed to modulation of telomerase activity include methods that employ specific antisense polynucleotides complementary to all or part of the human telomerase RNA component (hTR) sequences, such as antisense polynucleotides to the human telomerase RNA component gene or its transcribed RNA, including truncated forms which may be associated with telomerase holoenzyme. Such complementary antisense polynucleotides may include nucleotide substitutions, additions, deletions, or transpositions, so long as specific binding to the relevant target sequence corresponding to telomerase RNA component or its gene is retained as a functional property of the polynucleotide. Complementary antisense polynucleotides include soluble antisense RNA or DNA oligonucleotides which can hybridize specifically to telomerase RNA component species and prevent transcription of the telomerase RNA component gene (Ching et al. (1989) Proc. Natl. Acad. Sci. U.S.A. 86: 10006; Broder et al. (1990) Ann. Int. Med. 113: 604; Loreau et al. (1990) FEBS Letters 274: 53; Holcenberg et al., WO91/11535; US 5,256,643 (U.S.S.N. 07/530,165); WO91/09865; WO91/04753; WO90/13641; and EP 386563. The antisense polynucleotides therefore inhibit production of functional telomerase RNA component. Since telomerase RNA component expression (transcription rate and/or RNA stability) is associated with activation and enzymatic activity of telomerase holoenzyme, antisense polynucleotides that prevent transcription of RNA corresponding to telomerase RNA component and/or the interaction of telomerase RNA component to the protein component of human telomerase and/or the interaction of telomerase RNA component to telomeric sequences may inhibit telomerase activity and/or reverse a phenotype, such as immortalization or neoplastic transformation, of cells expressing telomerase activity in the absence of antisense polynucleotides. Compositions containing a therapeutically effective dosage of telomerase RNA component antisense polynucleotides may be administered for treatment of diseases which require telomerase activity for cellular pathogenesis (e.g., neoplasia) or to inhibit gamete production or maintenance (i.e., as a contraceptive), if desired. Antisense polynucleotides of various lengths may be produced, although such antisense polynucleotides typically comprise a sequence of about at least 25 consecutive nucleotides which are substantially complementary to a naturally-occurring telomerase RNA component polynucleotide sequence, and typically which are perfectly complementary to a human telomerase RNA component sequence, often being complementary to the sequence of telomerase RNA component which is complementary to the telomere repeat sequence, or complementary to a portion of the telomerase RNA component which contacts the telomerase polypeptide subunit.

Antisense polynucleotides may be produced from a heterologous expression cassette in a transfectant cell or transgenic cell. The heterologous expression cassette may be part of a gene therapy vector, such as an adenoviral or adeno-associated viral vector, or other gene therapy vector. The heterologous cassette may be on a polynucleotide which is not capable of independent replication and which is transferred into cells by any of a variety of suitable methods know to those skilled in the art (e.g., lipofection, biolistics, liposomes, immunoliposomes, electroporation, etc.). Alternatively, the antisense polynucleotides may comprise soluble oligonucleotides that are administered to the external milieu, either in the culture medium in vitro or in interstitial spaces and bodily fluids (e.g., blood, CSF) for application in vivo. Soluble antisense polynucleotides present in the external milieu have been shown to gain access to the cytoplasm and inhibit specific RNA species. In some embodiments the antisense polynucleotides comprise methylphosphonate moieties, C-5 propenyl moieties, 2' fluororibose sugars, or are polyamide nucleic acids (PNAs) (Egholm et al. (1992) J. Am. Chem. Soc. 114: 1895; Wittung et al. (1994) Nature 368: 561; Egholm et al. (1993) Nature 365: 566; Hanvey et al. (1992) Science 258: 1481. For general methods relating to antisense polynucleotides, see Antisense RNA and DNA, (1988), D.A. Melton, Ed., Cold Spring Harbor Laboratory, Cold Spring Harbor, NY).

In addition to the antisense oligonucleotides of the invention, one can construct oligonucleotides that will bind to duplex nucleic acid either in the folded RNA component or in the gene for the RNA component, forming a triple helix-containing or triplex nucleic acid to inhibit telomerase activity. Such oligonucleotides of the invention are constructed using the base-pairing rules of triple helix formation and the nucleotide sequence of the RNA component (Cheng et al. (1988) J. Biol. Chem. 263: 15110; Ferrin and camerini-otero (1991) Science 354: 1494; Ramdas et al. (1989) J. Biol. Chem. 264: 17395; Strobel et. al. (1991) Science 254: 1639; Hsieh et al. (1990) op.cit.; Rigas et al. (1986) Proc. Natl. Acad. Sci. (U.S.A.) 83: 9591. Such oligonucleotides can block telomerase activity in a number of ways, including by preventing transcription of the telomerase gene or by binding to a duplex region of the RNA component of telomerase in a manner that prevents the RNA component either from forming a functional ribonucleoprotein telomerase or from serving as a template for telomeric DNA synthesis. Typically, and depending on mode of action, the triplex-forming oligonucleotides of the invention comprise a specific sequence of from about 10 to about 25 to 200 or more (i.e., large enough to form a stable triple helix but small enough, depending on the mode of delivery, to administer in vivo, if desired) nucleotides "complementary" (in this context, complementary means able to form a stable triple helix) to a specific sequence in the RNA component of telomerase or the gene for the RNA component of telomerase.

In addition to the antisense and triple helix-forming oligonucleotides of the invention, sense oligonucleotides identical in sequence to at least a portion of the RNA component of human telomerase can also be used to inhibit telomerase activity. Oligonucleotides of the invention of this type are characterized in comprising either (1) less than the complete sequence of the RNA component needed to form a functional telomerase enzyme or (2) the complete sequence of the RNA component needed to form a functional telomerase enzyme as well as a substitution or insertion of one or more nucleotides that render the resulting RNA non-functional. In both cases, inhibition of telomerase activity is observed due to the "mutant" RNA component binding the protein components of human telomerase to form an inactive telomerase molecule. The mechanism of action of such oligonucleotides thus involves the assembly of a non-functional ribonucleoprotein telomerase or the prevention of assembly of a functional ribonucleoprotein telomerase. An example can be a template-scrambled variant consisting of nucleotides 48-209, but wherein the telomere repeat template sequence is altered. Such template-scrambled variants are able to compete for binding to telomerase protein component. Sense oligonucleotides of the invention of this type typically comprise a specific sequence of from about 20, 50, 100, 200, 400, 500, or more nucleotides identical to a specific sequence of nucleotides in the RNA component of human telomerase.

In addition, antisense polynucleotides can comprise a derivatized substituent which is substantially non-interfering with respect to hybridization to the RNA component of a mammalian telomerase. Antisense polynucleotides that have been modified with appended chemical substituents may be introduced into a metabolically active eukaryotic cell to hybridize with a telomerase RNA component of telomerase in the cell. Typically such antisense polynucleotides are derivatized, and additional chemical substituents are attached, either during or after polynucleotide synthesis, respectively, and are thus localized to a complementary sequence in the telomerase RNA component where they produce an alteration or chemical modification to a local DNA sequence and/or to the telomerase protein component. Preferred attached chemical substituents include: europium (III) texaphyrin, cross-linking agents, psoralen, metal chelates (e.g., iron/EDTA chelate for iron catalyzed cleavage), topoisomerases, endonucleases, exonucleases, ligases, phosphodiesterases, photodynamic porphyrins, chemotherapeutic drugs (e.g., adriamycin, doxirubicin), intercalating agents, base-modification agents, immunoglobulin chains, and oligonucleotides. Iron/EDTA chelates are particularly preferred chemical substituents where local cleavage of a polynucleotide sequence is desired (Hertzberg et al. (1982) J. Am. Chem. Soc. 104: 313; Hertzberg and Dervan (1984) Biochemistry 23: 3934; Taylor et al. (1984) Tetrahedron 40: 457; Dervan, PB (1986) Science 232: 464). Preferred attachment chemistries include: direct linkage, e.g., via an appended reactive amino group (Corey and Schultz (1988) Science 238: 1401, and other direct linkage chemistries, although streptavidin/biotin and digoxigenin/anti-digoxigenin antibody linkage methods may also be used. Methods for linking chemical substitutents are provided in U.S. Patents 5,135,720, 5,093,245, and 5,055,556. Other linkage chemistries may be used at the discretion of the practitioner. Polynucleotides which correspond to all or a substantial portion of a mammalian telomerase RNA component (i.e., "sense" polynucleotides) may also be derivatized and used to react with telomeric repeat sequences in the genome and produce adducts or other modification of the chemical environment at telomere regions of the chromosomes.

### Mismatch Templates

Thus, another useful oligonucleotide of the invention comprises an altered or mutated sequence of the RNA component of human telomerase. Yu et al., 1990, Nature 344: 126, shows that a mutated form of the RNA component of *Tetrahymena* telomerase can be incorporated into the telomerase of *Tetrahymena* cells and that the incorporation has deleterious effects on those cells. Incorporation of mutated forms of the RNA component of human telomerase may have similar effects on human cells that otherwise have telomerase activity without affecting normal human cells that do not have telomerase activity. Such mutated forms include those in which the sequence 5'-CTAACCCTA-3' is mutated to 5'-CAAACCCAA-3', 5'-CCAACCCCAA-3', or 5'-CTCACCCTCA-3'. Each of these altered RNA component sequences alters the telomeric repeat units incorporated into the chromosomal DNA, thus affecting chromosome structure and function. Such oligonucleotides can be designed to contain restriction enzyme recognition sites useful in diagnostic methods for the presence of the altered RNA component via restriction enzyme digestion of telomeric DNA or an extended telomerase substrate.

To illustrate this aspect of the invention, site-specific mutagenesis was carried out using a plasmid (designated pGRN33, available from the American Type Culture Collection under accession No. ATCC 75926) that comprises an ⁻2.5 kb HindIII-SacI fragment from lambda clone 28-1 (see Example 7, below) as well as the SV40 origin of replication (but no promoter activity). The resulting plasmids, designated pGRN34 (comprising 5'-CAAACCCAA-3'), pGRN36 (comprising 5'-CCAACCCCAA-3'), and pGRN37 (comprising 5'-CTCACCCTCA-3'), were transformed into eukaryotic host cells (a 293-derived cell line expressing SV40 large T antigen), and telomerase assays were conducted using cell extracts from the transformants.

The assays showed that the telomerase activity in the cells resulted in the formation of nucleic acids comprising the altered sequences, indicating that the genomic clone comprised a functional RNA component gene and that the plasmids comprised an altered but functional RNA component gene. These results illustrate how the present invention provides recombinant telomerase preparations and methods for producing such preparations. The present invention provides a recombinant human telomerase that comprises the protein components of human telomerase in functional association with a recombinant RNA component of the invention. Such recombinant RNA component molecules of the invention include those that differ from naturally occurring RNA component molecules by one or more base substitutions, deletions, or insertions, as well as RNA component molecules identical to a naturally occurring RNA component molecule that are produced in recombinant host cells. The method for producing such recombinant telomerase molecules comprises transforming a eukaryotic host cell that expresses the protein components of telomerase with a recombinant expression vector that encodes an RNA component molecule of the invention, and culturing said host cells transformed with said vector under conditions such that the protein components and RNA component are expressed and assemble to form an active telomerase molecule capable of adding sequences (not necessarily the same sequence added by native telomerase) to telomeres of chromosomal DNA. Other useful embodiments of such recombinant DNA expression vectors (or plasmids) include plasmids that comprise the gene for the RNA component of human telomerase with a deletion, insertion, or other modification that renders the gene non-functional. Such plasmids are especially useful for human gene therapy to "knock-out" the endogenous RNA component gene, although a highly efficient transformation and recombination system is required, to render the treated cells irreversibly mortal.

### Ribozyme Embodiments

Other oligonucleotides of the invention called ribozymes can also be used to inhibit telomerase activity. Unlike the antisense and other oligonucleotides described above, which bind to an RNA, a DNA, or a telomerase protein component, a ribozyme not only binds but also specifically cleaves and thereby potentially inactivates a target RNA, such as the RNA component of human telomerase. Such a ribozyme can comprise 5'- and 3'-terminal sequences complementary to the telomerase RNA. Depending on the site of cleavage, a ribozyme can render the telomerase enzyme inactive. See PCT patent publication No. 93/23572, supra. Those in the art upon review of the RNA sequence of the human telomerase RNA component will note that several useful ribozyme target sites are present and susceptible to cleavage by, for example, a hammerhead motif ribozyme. Illustrative ribozymes of the invention of this type include the ribozymes below, which are RNA molecules having the sequences indicated: 1: 5'-UAGGGUUACUGAUGAGUCCGUGAGGACGAAACAAAAAAU-3'; 2:    5'-UUAGGGUCUGAUGAGUCCGUGAGGACGAAAGACAAAA-3'; 3:    5'-UCUCAGUCUGAUGAGUCCGUGAGGACGAAAGGUUA-3'; and 4:     5'-CCCGAGACUGAUGAGUCCGUGAGGACGAAACCCGCG-3'. Other optimum target sites for ribozyme-mediated inhibition of telomerase activity can be determined as described by Sullivan et al., PCT patent publication No. 94/02595 and Draper et al., PCT patent publication No. 93/23569.

As described by Hu et al. PCT patent publication No. 94/03596, antisense and ribozyme functions can be combined in a single oligonucleotide. Moreover, ribozymes can comprise one or more modified nucleotides or modified linkages between nucleotides, as described above in conjunction with the description of illustrative antisense oligonucleotides of the invention. In one aspect, a catalytic subunit of RNase P (human or E. coli) is modified (see, Altman S (1995) Biotechnology 13: 327) to generate a guide sequence which corresponds to the portion of mammalian telomerase RNA component which basepairs to the telomere repeat sequence; such RNase P variants can cleave telomere sequences. In one aspect, a catalytic subunit of RNase P (human or E. coli) is modified to generate a guide sequence which is complementary to a portion of telomerase RNA component such that the RNase P variant can cleave telomerase RNA component. Such engineered ribozymes can be expressed in cells or can be transferred by a variety of means (e.g., liposomes, immunoliposomes, biolistics, direct uptake into cells, etc.). Other forms of ribozymes (group I intron ribozymes (Cech T (1995) Biotechnology 13; 32-3); hammerhead ribozymes (Edgington SM (1992) Biotechnology 10: 256) can be engineered on the basis of the disclosed telomerase RNA component sequence information to catalyze cleavage of human telomerase RNA component and/or human telomere repeat sequences.

Thus, the invention provides a wide variety of oligonucleotides to inhibit telomerase activity. Such oligonucleotides can be used in the therapeutic methods of the invention for treating disease, which methods comprise administering to a patient a therapeutically effective dose of a telomerase inhibitor or activator of the invention. One can measure telomerase inhibition or activation to determine the amount of an agent that should be delivered in a therapeutically effective dose using the assay protocols described in PCT patent publication No. 93/23572 noted above. As noted in those applications and discussed above, inhibition of telomerase activity renders an immortal cell mortal, while activation of telomerase activity can increase the replicative lifespan of a cell. Telomerase inhibition therapy is an effective treatment against cancers involving the uncontrolled growth of immortal cells, and telomerase activation is an effective treatment to prevent cell senescence. Delivery of agents that inhibit or block telomerase activity, such as an antisense oligonucleotide, a triple helix-forming oligonucleotide, a ribozyme, or a plasmid that drives expression of a mutant RNA component of telomerase can prevent telomerase action and ultimately leads to cell senescence and cell death of treated cells.

### Therapeutic and Prophylactic Aspects

In addition, the present invention provides therapeutic methods that ensure that normal cells remain mortal; for instance, the RNA component can be modified using standard genetic engineering procedures to delete all or a portion of a natural gene encoding the component (e.g., by in vitro mutagenesis) by genetic recombination. Such cells will then be irreversibly mortal. This procedure is useful in gene therapy, where normal cells modified to contain expression plasmids are introduced into a patient, and one wants to ensure cancerous cells are not introduced or, if such cells are introduced, then those cells have been rendered irreversibly mortal.

Because telomerase is active only in tumor, germline, and certain stem cells of the hematopoietic system, other normal cells are not affected by telomerase inhibition therapy. Steps can also be taken to avoid contact of the telomerase inhibitor with germline or stem cells, although this may not be essential. For instance, because germline cells express telomerase activity, inhibition of telomerase may negatively impact spermatogenesis and sperm viability, suggesting that telomerase inhibitors may be effective contraceptives or sterilization agents. This contraceptive effect may not be desired, however, by a patient receiving a telomerase inhibitor of the invention for treatment of cancer. In such cases, one can deliver a telomerase inhibitor of the invention in a manner that ensures the inhibitor will only be produced during the period of therapy, such that the negative impact on germline cells is only transient.

Other therapeutic methods of the invention employ the telomerase RNA nucleic acid of the invention to stimulate telomerase activity and to extend replicative cell life span. These methods can be carried out by delivering to a cell a functional recombinant telomerase ribonucleoprotein of the invention to the cell. For instance, the ribonucleoprotein can be delivered to a cell in a liposome, or the gene for the RNA component of human telomerase (or a recombinant gene with different regulatory elements) can be used in a eukaryotic expression plasmid (with or without sequences coding for the expression of the protein components of telomerase) to activate telomerase activity in various normal human cells that otherwise lack detectable telomerase activity due to low levels of expression of the RNA component or a protein component of telomerase. If the telomerase RNA component is not sufficient to stimulate telomerase activity, then the RNA component can be transfected along with genes expressing the protein components of telomerase to stimulate telomerase activity. Thus, the invention provides methods for treating a condition associated with the telomerase activity within a cell or group of cells by contacting the cell(s) with a therapeutically effective amount of an agent that alters telomerase activity in that cell.

Cells that incorporate extra copies of the telomerase RNA gene can exhibit an increase in telomerase activity and an associated extended replicative life span. Such therapy can be carried out ex vivo on cells for subsequent introduction into a host or can be carried out in vivo. The advantages of stabilizing or increasing telomere length by adding exogenous telomerase genes ex vivo to normal diploid cells include: telomere stabilization can arrest cellular senescence and allow potentially unlimited amplification of the cells; and normal diploid cells with an extended life span can be cultured in vitro for drug testing, virus manufacture, or other useful purposes. Moreover, ex vivo amplified stem cells of various types can be used in cell therapy for particular diseases, as noted above.

Telomere stabilization can also suppress cancer incidence in replicating cells by preventing telomeres from becoming critically short as cells near crisis. During crisis, massive genomic instability is generated as the protective effect of the telomeric cap is lost. The "genetic deck" is reshuffled, and almost all cells die. The rare cells that emerge from this process are typically aneuploid with many gene rearrangements and end up reestablishing stability in their telomeres by expressing telomerase. If crisis can be prevented by keeping telomeres long, then the genomic instability associated with crisis can also be prevented, limiting the chances that an individual cell will suffer the required number of genetic mutations needed to spawn a metastatic cancer.

Cells that can be targeted for telomerase gene therapy (therapy involving increasing the telomerase activity of a target cell) include but are not limited to hematopoietic stem cells (AIDS and post-chemotherapy), vascular endothelial cells (cardiac and cerebral vascular disease), skin fibroblasts and basal skin keratinocytes (wound healing and burns), chondrocytes (arthritis), brain astrocytes and microglial cells (Alzheimer's Disease), osteoblasts (osteoporosis), retinal cells (eye diseases), and pancreatic islet cells (Type I diabetes).

Typically, the therapeutic methods of the invention involve the administration of an oligonucleotide that functions to inhibit or stimulate telomerase activity under in vivo physiological conditions and will be stable under those conditions. As noted above, modified nucleic acids may be useful in imparting such stability, as well as for ensuring delivery of the oligonucleotide to the desired tissue, organ, or cell. Methods useful for delivery of oligonucleotides for therapeutic purposes are described in Inouye et al., U.S. Patent 5,272,065.

While oligonucleotides can be delivered directly as a drug in a suitable pharmaceutical formulation, one can also deliver oligonucleotides using gene therapy and recombinant DNA expression plasmids of the invention. One such illustrative plasmid is described in Example 8, below. In general, such plasmids will comprise a promoter and, optionally, an enhancer (separate from any contained within the promoter sequences) that serve to drive transcription of an oligoribonucleotide, as well as other regulatory elements that provide for episomal maintenance or chromosomal integration and for high-level transcription, if desired. Adenovirus-based vectors are often used for gene therapy and are suitable for use in conjunction with the reagents and methods of the present invention. See PCT patent publication Nos. 94/12650; 94/12649; and 94/12629. Useful promoters for such purposes include the metallothionein promoter, the constitutive adenovirus major late promoter, the dexamethasone-inducible MMTV promoter, the SV40 promoter, the MRP polIII promoter, the constitutive MPSV promoter, the tetracycline-inducible CMV promoter (such as the human immediate-early CMV promoter), and the constitutive CMV promoter. A plasmid useful for gene therapy can comprise other functional elements, such as selectable markers, identification regions, and other genes. Recombinant DNA expression plasmids can also be used to prepare the oligonucleotides of the invention for delivery by means other than by gene therapy, although it may be more economical to make short oligonucleotides by in vitro chemical synthesis.

In related aspects, the invention features pharmaceutical compositions including a therapeutically effective amount of a telomerase inhibitor or telomerase activator of the invention. Pharmaceutical compositions of telomerase inhibitors of the invention include a mutant RNA component of human telomerase, an antisense oligonucleotide or triple helix-forming oligonucleotide that binds the RNA component or the gene for the same of human telomerase, or a ribozyme able to cleave the RNA component of human telomerase, or combinations of the same or other pharmaceuticals in a pharmaceutically acceptable carrier or salt. Other pharmaceutical compositions of the invention comprise a telomerase activator preparation, such as purified human telomerase or mRNA for the protein components of telomerase and the RNA component of telomerase, and are used to treat senescence-related disease. In an aspect, a mutated sense mammalian telomerase RNA component is administered to a cell population; said mutated sense telomerase RNA component comprises at least one base mismatch with respect to the human telomerase repeat sequence, but is capable of exhibiting telomerase activity in conjunction with human telomerase polypeptide component, producing misincorporation at selected nucleotide positions in the human telomerase repeat, thereby generating telomeres which rely on the continued presence of the mutated sense telomerase RNA component for substantial replication. A therapeutic method is provided wherein a mutated sense telomerase RNA component is administered to a cell population for a sufficient period to introduce telomere sequences which are substantially non-functional as templates for naturally occurring mammalian telomerase RNA component, followed by withdrawal of the mutated sense telomerase RNA component which results in rapid loss of average telomere length in the cell population and enhanced senescence or cell mortality.

The therapeutic agent can be provided in a formulation suitable for parenteral, nasal, oral, or other mode of administration. See PCT patent publication No. 93/23572, supra.

### Diagnostic Methods

The present invention provides diagnostic methods and reagents in addition to the pharmaceutical formulations and therapeutic methods described above. The invention provides diagnostic methods for determining the level, amount, or presence of the RNA component of human telomerase, telomerase, or telomerase activity in a cell, cell population, or tissue sample. In a related aspect, the present invention provides useful reagents for such methods, optionally packaged into kit form together with instructions for using the kit to practice the diagnostic method. As noted above in connection with the tests conducted to determine that clone pGRN7 contained the cDNA for the RNA component of human telomerase, the levels of the RNA component are elevated in tumor cells. Thus, detection of the RNA component is a useful diagnostic for tumor cells.

In addition, probes or primers that bind specifically to the RNA component of human telomerase (or either strand of the gene for the same) can be used in diagnostic methods to detect the presence of telomerase nucleic acid in a sample. Primers and probes are oligonucleotides that are complementary, and so will bind, to a target nucleic acid. Although primers and probes can differ in sequence and length, the primary differentiating factor is one of function: primers serve to initiate DNA synthesis, as in PCR amplification, while probes are typically used only to bind to a target nucleic acid. Typical lengths for a primer or probe can range from 8 to 20 to 30 or more nucleotides. A primer or probe can also be labeled to facilitate detection (i.e., radioactive or fluorescent molecules are typically used for this purpose) or purification/separation (i.e., biotin or avidin is often used for this purpose).

An especially preferred diagnostic method of the invention involves the detection of telomerase RNA component sequences in cell or tissue samples taken from patients suspected to be at risk for cancer. Such methods will typically involve binding a labelled probe or primer to an RNA component sequence under conditions such that only perfectly matched (complementary) sequences bind (hybridize) to one another. Detection of labelled material bound to RNA. in the sample will correlate with the presence of telomerase activity and the presence of cancer cells. Some cells may express the RNA component of telomerase but remain telomerase-negative due to lack of expression of the protein components of telomerase. If one desired to detect the presence of telomerase activity in such cells, then one could first isolate protein and then determine whether the protein fraction contains the telomerase RNA component, which would signal the presence of telomerase activity. The diagnostic methods of the invention may be especially useful in detecting the presence of telomerase activity in tissue biopsies and histological sections in which the method is carried out in situ, typically after amplification of telomerase RNA component using specific PCR primers of the invention.

The invention also provides telomerase RNA component polynucleotide probes for diagnosis of disease states (e.g., neoplasia or preneoplasia) by detection of a telomerase RNA component, or rearrangements or amplification of the telomerase RNA component gene, in cells explanted from a patient, or detection of a pathognomonic telomerase RNA component allele (e.g., by RFLP or allele-specific PCR analysis). Typically, the detection will be by in situ hybridization using a labeled (e.g., ³²P, ³⁵S, ¹⁴C, ³H, fluorescent, biotinylated, digoxigeninylated) telomerase RNA component polynucleotide, although Northern blotting, dot blotting, or solution hybridization on bulk RNA or poly A⁺ RNA isolated from a cell sample may be used, as may PCR amplification using telomerase RNA component-specific primers. Cells which contain an altered amount of telomerase RNA component as compared to non-neoplastic cells of the same cell type(s) will be identified as candidate diseased cells. Similarly, the detection of pathognomonic rearrangements or amplification of the telomerase RNA component gene locus or closely linked loci in a cell sample will identify the presence of a pathological condition or a predisposition to developing a pathological condition (e.g., cancer, genetic disease). The polynucleotide probes are also used for forensic identification of individuals, such as for paternity testing or identification of criminal suspects or unknown decedents.

Within the human population there can be minor alterations in the basic primary sequence of telomerase RNA component, including allelic variants, restriction site polymorphisms, and congenital telomerase RNA component disease alleles associated with genetic disease.

If desired, PCR amplimers for amplifying substantially full-length telomerase RNA component copies may be selected at the discretion of the practitioner. Similarly, amplimers to amplify portions of the telomerase RNA component gene or RNA may be selected.

### Telomerase RNA Component Expression

Depending on the length and intended function of the primer, probe, or other nucleic acid comprising sequences from the RNA component of human telomerase, expression plasmids of the invention may be useful. For instance, recombinant production of the full-length RNA component of the invention can be carried out using a recombinant DNA expression plasmid of the invention that comprises a nucleic acid comprising the nucleotide sequence of the RNA component positioned for transcription under the control of a suitable promoter. Host cells for such plasmids can be either prokaryotic or eukaryotic, and the promoter, as well as the other regulatory elements and selectable markers chosen for incorporation into the expression plasmid will depend upon the host cell used for production.

The intact RNA component gene, i.e., the promoter, which includes any regulatory sequences in the 5'-region of the gene, and RNA component coding region, can be used to express the RNA component in human cells, including human cells that have been immortalized by viral transformation or cancer. The promoter of the RNA component gene may be regulated, however, and for this and other reasons, one may want to express the RNA component under the control of a different promoter. On the other hand, the promoter of the RNA component gene can be used independently of the RNA component coding sequence to express other coding sequences of interest. For instance, one could study the transcriptional regulation of the RNA component gene by fusing the promoter of the RNA component gene to a coding sequence for a reporter coding sequence, such as the coding sequence for beta-galactosidase or another enzyme or protein the expression of which can be readily monitored. Thus, the promoter and other regulatory elements of the gene for the RNA component of human telomerase can be used not only to express the RNA component but also protein components of human telomerase, antisense or other oligonucleotides, as well as other gene products of interest in human cells. Expression plasmids comprising the intact gene for the RNA component of human telomerase can be especially useful for a variety of purposes, including gene therapy. Those of skill in the art recognize that a wide variety of expression plasmids can be used to produce useful nucleic acids of the invention and that the term "plasmid", as used herein, refers to any type of nucleic acid (from a phage, virus, chromosome, etc.) that can be used to carry specific genetic information into a host cell and maintain that information for a period of time.

### Isolation of Telomerase Protein Component

The reagents of the present invention also allow the cloning and isolation of nucleic acids encoding the protein components of human as well as other mammalian telomerase enzymes, which have not previously been available. Access to such nucleic acids provide complementary benefits to those provided by the nucleic acids comprising nucleic acid sequences of the RNA component of human telomerase. For instance, and as noted above, the therapeutic benefits of the present invention can be enhanced, in some instances, by use of purified preparations of the protein components of human telomerase and by access to nucleic acids encoding the same. The nucleic acids of the invention that encode the RNA component of human telomerase can be used to isolate the nucleic acid encoding the protein components of human telomerase, allowing access to such benefits. Thus, the invention provides methods for isolating and purifying the protein components of human telomerase, as well as for identifying and isolating nucleic acids encoding the protein components of human telomerase. In related aspects, the present invention provides purified human telomerase, purified nucleic acids that encode the protein components of human telomerase, recombinant expression plasmids for the protein components of human telomerase. The invention also provides pharmaceutical compositions comprising as an active ingredient either the protein components of human telomerase or a nucleic acid that either encodes those protein components or interacts with nucleic acids that encode those protein components, such as antisense oligonucleotides, triple helix-forming oligonucleotides, ribozymes, or recombinant DNA expression plasmids for any of the foregoing.

The cloned RNA component of human telomerase can be used to identify and clone nucleic acids encoding the protein components of the ribonucleoprotein telomerase enzyme. Several different methods can be employed to achieve identification and cloning of the protein components. For instance, one can use affinity capture of the enzyme or partially denatured enzyme using as an affinity ligand either (1) nucleotide sequences complementary to the RNA component to bind to the RNA component of the intact enzyme; or (2) the RNA component to bind the protein components of a partially or fully denatured enzyme. The ligand can be affixed to a solid support or chemically modified (e.g., biotinylated) for subsequent immobilization on the support. Exposure of cell extracts containing human telomerase, followed by washing and elution of the telomerase enzyme bound to the support, provides a highly purified preparation of the telomerase enzyme. The protein components can then be optionally purified further or directly analyzed by protein sequencing. The protein sequence determined can be used to prepare primers and probes for cloning the cDNA or identifying a clone in a genomic bank comprising nucleic acids that encode a protein component of telomerase.

Affinity capture of telomerase utilizing an engineered RNA component can also be conducted using in vitro transcribed telomerase RNA and a system for the reconstitution of telomerase enzyme activity. See Autexier and Greider, 1994, Genes & Development 8:563-575. The RNA is engineered to contain a tag, similar to epitope tagging of proteins. The tag can be an RNA sequence to which a tightly binding ligand is available, e.g., an RNA sequence-specific antibody, a sequence-specific nucleic acid binding protein, or an organic dye that binds tightly to a specific RNA sequence. The tolerance of telomerase for the tag sequence and position can be tested using standard methods. Synthesis of the altered RNA component and the reconstitution step of this method can also be carried out in vivo. Affinity capture using the immobilized ligand for the RNA tag can then be used to isolate the enzyme.

Expression screening can also be used to isolate the protein components of the telomerase enzyme. In this method, cDNA expression libraries can be screened with labeled telomerase RNA, and cDNAs encoding proteins that bind specifically to telomerase RNA can be identified. A molecular genetic approach using translational inhibition can also be used to isolate nucleic acids encoding the protein components of the telomerase enzyme. In this method, telomerase RNA sequences will be fused upstream of a selectable marker. When expressed in a suitable system, the selectable marker will be functional. When cDNA encoding a telomerase RNA binding protein is expressed, the protein will bind to its recognition sequence thereby blocking translation of the selectable marker, thus allowing for identification of the clone encoding the protein. In other embodiments of this method, the blocked translation of the selectable marker will allow transformed cells to grow. other systems that can be employed include the "interaction trap system" described in PCT patent publication No. WO 94/10300; the "one-hybrid" system described in Li and Herskowitz, 17 Dec. 1993, Science 262:1870-1874, and Zervos et al., 29 Jan. 1993, Cell 72:223-232; and the "two-hybrid" system commercially available from Clontech.

Telomerase RNA binding or telomerase activity assays for detection of specific binding proteins and activity can be used to facilitate the purification of the telomerase enzyme and the identification of nucleic acids that encode the protein components of the enzyme. For example, nucleic acids comprising RNA component sequences can be used as affinity reagents to isolate, identify, and purify peptides, proteins or other compounds that bind specifically to a sequence contained within the RNA component, such as the protein components of human telomerase. Several different formats are available, including gel shift, filter binding, footprinting, Northwestern (RNA probe of protein blot), and photocrosslinking, to detect such binding and isolate the components that bind specifically to the RNA component. These assays can be used to identify binding proteins, to track purification of binding proteins, to characterize the RNA binding sites, to determine the molecular size of binding proteins, to label proteins for preparative isolation, and for subsequent immunization of animals for antibody generation to obtain antibodies for use in isolating the protein or identifying a nucleic acid encoding the protein in a coupled transcription/translation system.

### Purification of Mammalian Telomerase Protein Component

Mammalian telomerase protein component can be purified by conventional biochemical methods from telomerase-expressing cells, such as HT1080 cells, 293 cells, and other suitable immortalized cell lines. For example and not limitation, human telomerase can be purified from cell extracts. Mammalian telomerase extracts can be stripped of the telomerase RNA component by treatment with an RNAse activity or other suitable means to dissociate and/or degrade the telomerase RNA component while leaving telomerase protein component substantially intact and capable of reconstitution with addition of exogenous telomerase RNA component, such as may be produced recombinantly or the like. The telomerase protein component thus purified, and optionally stripped of endogenous telomerase RNA component, can be used in the agent screening assays described herein, and for other uses.

### Gene Therapy

Transferring exogenous genetic material into cells (i.e., DNA-mediated transfection) is an essential method for basic research in cell biology and molecular genetics, as well as the basis for developing effective methods for human gene therapy. So far, the majority of the approved gene transfer trials in the United States rely on replication-defective retroviral vectors harboring a therapeutic polynucleotide sequence as part of the retroviral genome (Miller et al. (1990) Mol. Cell. Biol. 10: 4239; Kolberg R (1992) J. NIH Res. 4: 43; Cornetta et al. (1991) Hum. Gene Ther. 2: 215). Adenoviral vectors have also been described for potential use in human gene therapy (Rosenfeld et al. (1992) Cell 68: 143).

The other gene transfer method that has been approved for use in humans is physical transfer of plasmid DNA in liposomes directly into tumor cells in situ. Unlike viral vectors which must be propagated in cultured cells, plasmid DNA can be purified to homogeneity and thus reduces the potential for pathogenic contamination. In some situations (e.g., tumor cells) it may not be necessary for the exogenous DNA to stably integrate into the transduced cell, since transient expression may suffice to kill the tumor cells. Liposome-mediated DNA transfer has been described by various investigators (Wang and Huang (1987) Biochem. Biophys. Res. Commun. 147: 980; Wang and Huang (1989) Biochemistry 28: 9508; Litzinger and Huang (1992) Biochem. Biophys. Acta 1113: 201; Gao and Huang (1991) Biochem. Biophys. Res. Commun. 179: 280; Felgner WO91/17424; WO91/16024).

Immunoliposomes have also been described as carriers of exogenous polynucleotides (Wang and Huang (1987) Proc Natl. Acad. Sci. (U.S.A.) 84: 7851; Trubetskoy et al. (1992) Biochem. Biophys. Acta 1131: 311). Immunoliposomes hypothetically might be expected to have improved cell type specificity as compared to liposomes by virtue of the inclusion of specific antibodies which presumably bind to surface Antigens on specific cell types. Behr et al. (1989) Proc. Natl. Acad. Sci. (U.S.A.) 86: 6982 report using lipopolyamine as a reagent to mediate transfection itself, without the necessity of any additional phospholipid to form liposomes.

Accordingly, a polynucleotide substantially identical to at least 25 nucleotides, preferably 50 to 100 nucleotides or more, of the telomerase RNA component sequence or its complement, is operably linked to a heterologous promoter to form a transcription unit capable of expressing an telomerase RNA component or an antisense telomerase RNA component polynucleotide in a human cell. Such a transcription unit may be comprised in a transgene, adenoviral vector, or other gene therapy modality for delivery into human cells, such as for therapy of a telomerase-related disease (e.g., neoplasia). Suitable delivery methods of the telomerase RNA component sense or antisense expression construct will be selected by practitioners in view of acceptable practices and regulatory requirements.

### Transgenic Animal Embodiments

Genomic clones of mammalian telomerase RNA component, particularly of telomerase RNA component genes which are substantially identical to mouse telomerase RNA component, may be used to construct homologous targeting constructs for generating cells and transgenic nonhuman animals having at least one functionally disrupted telomerase RNA component allele. Guidance for construction of homologous targeting constructs may be found in the art, including: Rahemtulla et al. (1991) Nature 353: 180; Jasin et al. (1990) Genes Devel. 4: 157; Koh et al. (1992) Science 256: 1210; Molina et al. (1992) Nature 357: 161; Grusby et al. (1991) Science 253: 1417; Bradley et al. (1992) Bio/Technology 10: 534. Homologous targeting can be used to generate so-called "knockout" mice, which are heterozygous or homozygous for an inactivated telomerase RNA component allele. Such mice may be sold commercially as research animals for investigation of immune system development, neoplasia, spermatogenesis, may be used as pets, may be used for animal protein (foodstuff), and other uses.

Chimeric targeted mice are derived according to Hogan, et al., Manipulating the Mouse Embryo: A Laboratory Manual, Cold Spring Harbor Laboratory (1986) and Teratocarcinomas and Embryonic Stem Cells: A Practical Approach, E.J. Robertson, ed., IRL Press, Washington, D.C., (1987). Embryonic stem cells are manipulated according to published procedures (Teratocarcinomas and Embryonic Stem Cells: A Practical Approach, E.J. Robertson, ed., IRL Press, Washington, D.C. (1987); Zjilstra et al. (1989) Nature 342:435; and Schwartzberg et al. (1989) Science 246: 799.

Additionally, a telomerase RNA component cDNA or genomic gene copy may be used to construct transgenes for expressing telomerase RNA component at high levels and/or under the transcriptional control of transcription control sequences which do not naturally occur adjacent to the telomerase RNA component gene. For example but not limitation, a constitutive promoter (e.g., a HSV-*tk* or *pgk* promoter) or a cell-lineage specific transcriptional regulatory sequence (e.g., a CD4 or CD8 gene promoter/enhancer) may be operably linked to a telomerase RNA component polynucleotide sequence to form a transgene (typically in combination with a selectable marker such as a neo gene expression cassette). Such transgenes can be introduced into cells (e.g., ES cells, hematopoietic stem cells) and transgenic cells and transgenic nonhuman animals may be obtained according to conventional methods. Transgenic cells and/or transgenic nonhuman animals may be used to screen for antineoplastic agents and/or to screen for potential carcinogens, as overexpression of telomerase RNA component or inappropriate expression of telomerase RNA component may result in a preneoplastic or neoplastic state.

### Agent Screening Assays

Telomerase RNA component polynucleotides, telomerase protein component preparations, telomere repeat templates, and binding reactions and the like are practiced with reference to the Experimental Examples. Generally, telomerase protein components are obtained by conventional purification from telomerase-expressing mammalian cells and are stripped of associated RNA component prior to their use in the assays described herein. Particular aqueous conditions may be selected by the practitioner according to conventional methods. For general guidance, the following buffered aqueous conditions may be used: 10-250 mM NaCl, 5-50 mM Tris HCl, pH 5-8, with optional addition of divalent cation(s) and/or metal chelators and/or nonionic detergents and/or membrane fractions. It is appreciated by those in the art that additions, deletions, modifications (such as pH) and substitutions (such as KCl substituting for NaCl or buffer substitution) may be made to these basic conditions. Modifications can be made to the basic binding reaction conditions so long as specific telomerase holoenzyme formation and/or telomerase activity occurs in the control reaction(s). Conditions that do not permit specific binding and cleavage in control reactions (no agent included) are not suitable for use in binding assays.

Preferably, for determining binding of telomerase RNA component to immobilized telomerase protein component, the telomerase RNA component species is labeled with a detectable marker, typically a biotinyl group, a fluorescent moiety, or a radiolabeled incorporated nucelotide. Suitable labeling for telomerase protein component includes, but is not limited to, radiolabeling by incorporation of a radiolabeled amino acid (e.g., ¹⁴C-labeled leucine, ³H-labeled glycine, ³⁵S-labeled methionine), radiolabeling by post-translational radioiodination with ¹²⁵I or ¹³¹I (e.g., Bolton-Hunter reaction and chloramine T), labeling by post-translational phosphorylation with ³²P (e.g., phosphorylase and inorganic radiolabeled phosphate) fluorescent labeling by incorporation of a fluorescent label (e.g., fluorescein or rhodamine), or labeling by other conventional methods known in the art. In embodiments where one of the polypeptide species is immobilized by linkage to a substrate, the other component is generally labeled with a detectable marker.

Labeled telomerase RNA or protein component is contacted with immobilized telomerase protein or RNA component, respectively, and the capacity of an agent to alter the amount of labeled component which becomes immobilized (i.e., which binds the cognate telomerase component and is captured). The time and temperature of incubation of a binding reaction may be varied, so long as the selected conditions permit specific binding to occur in a control reaction where no agent is present. Preferable embodiments employ a reaction temperature of about at least 15 degrees Centigrade, more preferably 35 to 42 degrees Centigrade, and a time of incubation of approximately at least 15 seconds, although longer incubation periods are preferable so that, in some embodiments, a binding equilibrium is attained. Binding kinetics and the thermodynamic stability of bound complexes determine the latitude available for varying the time, temperature, salt, pH, and other reaction conditions. However, for any particular embodiment, desired binding reaction conditions can be calibrated readily by the practitioner using conventional methods in the art, which may include binding analysis using Scatchard analysis, Hill analysis, and other methods (Proteins, Structures and Molecular Principles, (1984) Creighton (ed.), W.H. Freeman and Company, New York).

Specific binding of labeled telomerase protein component to immobilized telomerase RNA component, respectively, is determined by including unlabeled competitor protein (e.g., albumin) and/or unlabeled RNA. After a binding reaction is completed, the amount of labeled species that is/are specifically bound to the immobilized species is detected. For example and not for limitation, after a suitable incubation period for binding, the aqueous phase containing non-immobilized labelled telomerase protein component is removed and the substrate containing the immobilized bound telomerase holoenzyme species and any labeled telomerase protein component bound to it is washed with a suitable buffer, optionally containing unlabeled blocking agent(s), and the wash buffer(s) removed. After washing, the amount of detectable label remaining specifically bound to the immobilized labeled component is determined (e.g., by optical, enzymatic, autoradiographic, or other radiochemical methods).

In some embodiments, addition of unlabeled blocking agents that inhibit non-specific binding are included. Examples of such blocking agents include, but are not limited to, the following: calf thymus DNA, salmon sperm DNA, yeast RNA, mixed sequence (random or pseudorandom sequence) oligonucleotides of various lengths, bovine serum albumin, nonionic detergents (NP-40, Tween, Triton X-100, etc.), nonfat dry milk proteins, Denhardt's reagent, polyvinylpyrrolidone, Ficoll, and other blocking agents. Practitioners may, in their discretion, select blocking agents at suitable concentrations to be included in binding assays.

In embodiments where a telomerase RNA component or telomerase protein component is immobilized, covalent or noncovalent linkage to a substrate may be used. Covalent linkage chemistries include, but are not limited to, well-characterized methods known in the art (Kadonaga and Tijan (1986) Proc. Natl. Acad. Sci. (U.S.A.) 83: 5889). One example, not for limitation, is covalent linkage to a substrate derivatized with cyanogen bromide (such as CNBr-derivatized Sepharose 4B). It may be desirable to use a spacer to reduce potential steric hindrance from the substrate. Noncovalent bonding of proteins to a substrate include, but are not limited to, bonding of the protein to a charged surface and binding with specific antibodies.

In one embodiment, candidate therapeutic agents are identified by their ability to block the binding of a telomerase protein component to a telomerase RNA component.

Typically, a telomerase RNA component used in these methods comprises a naturally occurring mammalian telomerase RNA component sequence (e.g., a human RNA component), although mutant telomerase RNA component sequences are sometimes used if the mutant telomerase RNA component binds to the telomerase protein component under control assay conditions (e.g., physiological conditions).

In an embodiment, candidate telomerase modulating agents are identified by their ability to produce a statistically significant reduction or increase in transcription of a reporter polynucleotide sequence (e.g., β-galactosidase gene, luciferase gene, HPRT gene) operably linked to a transcriptional regulatory sequence of a mammalian telomerase RNA component gene, preferably a human telomerase RNA component gene, in a metabolically active mammalian cell. A reporter gene (e.g., HPRT) can be inserted into a restriction site or blunt-ended site of a ds cDNA of a mammalian telomerase RNA component so as to generate a homologous targeting construct or transgene wherein, in a viable mammalian cell, the reporter gene is placed under the transcriptional control of the endogenous telomerase RNA component gene promoter and related transcription control elements. Agents which produce a dose-dependent transcriptional modulation of the reporter gene are thereby identified as candidate telomerase modulating agents.

In a variation, an endogenous telomerase RNA component gene in a mammalian cell is targeted with a homologous targeting construct to place a reporter polynucleotide sequence in operable linkage to the upstream transcription regulatory sequence (e.g., promoter) of the endogenous telomerase RNA component gene in the chromosomal locus of the endogenous gene. In an alternative variation, an exogenous polynucleotide comprising a reporter polynucleotide is operably linked to a mammalian telomerase RNA component gene transcription regulatory region (e.g., promoter and upstream transcription factor binding sites); the exogenous polynucleotide is transferred into a mammalian cell wherein it may integrate non-homologously into a chromosomal location and/or is maintained or replicated as an episomal polynucleotide. Agents which produce a statistically significant transcriptional modulation of the reporter polynucleotide in cells treated with the agent are thereby identified as candidate mammalian telomerase modulating agents.

Telomerase-modulating agents which reduce the cell's capacity to repair telomere damage or inhibit telomere replication (e.g., by competitively inhibiting endogenous naturally-occurring telomerase) are candidate antineoplastic agents or sensitizing agents which sensitize cells (e.g., neoplastic cells) to telomere damaging agents (e.g., alkylating agents and ionizing radiation).

Candidate antineoplastic agents are then tested further for antineoplastic activity in assays which are routinely used to predict suitability for use as human antineoplastic drugs. Examples of these assays include, but are not limited to: (1) ability of the candidate agent to inhibit the ability of anchorage-independent transformed cells to grow in soft agar, (2) ability to reduce tumorigenicity of transformed cells transplanted into nu/nu mice, (3) ability to reverse morphological transformation of transformed cells, (4) ability to reduce growth of transplanted tumors in nu/nu mice, (5) ability to inhibit formation of tumors or preneoplastic cells in animal models of spontaneous or chemically-induced carcinogenesis, and (6) ability to induce a more differentiated phenotype in transformed cells to which the agent is applied.

Assays for detecting the ability of agents to inhibit or augment the telomerase protein component:telomerase RNA component binding and/or enzymatic activity of telomerase provide for facile high-throughput screening of agent banks (e.g., compound libraries, peptide libraries, and the like) to identify telomerase antagonists or agonists. Such antagonists and agonists may modulate telomerase activity and thereby modulate telomere repair competence and replicative potential.

Administration of an efficacious dose of an agent capable of specifically inhibiting telomerase activity to a patient can be used as a therapeutic or prophylactic method for treating pathological conditions (e.g., cancer, inflammation, lymphoproliferative diseases, autoimmune disease, neurodegenerative diseases, and the like) which are effectively treated by modulating telomerase activity and DNA repair and replication.

As will be apparent to those of skill in the art upon reading of this disclosure, the present invention provides valuable reagents relating to human telomerase, as well as a variety of useful therapeutic and diagnostic methods. The above description of necessity provides a limited sample of such methods, which should not be construed as limiting the scope of the invention. Other features and advantages of the invention will be apparent from the following examples and claims.

The following examples describe specific aspects of the invention to illustrate the invention and provide a description of the methods used to isolate and identify the RNA component of human telomerase for those of skill in the art. The examples should not be construed as limiting the invention, as the examples merely provide specific methodology useful in understanding and practice of the invention.

### EXPERIMENTAL EXAMPLES

### Overview

The cloning of the RNA component of human telomerase required a novel method involving negative selection and cycles of positive selection, described below. Initially, however, an attempt was made to clone the RNA component using reverse transcription and a method for cloning the ends of cDNA called '5-RACE PCR amplification. The reverse transcription reaction was initiated with a primer identical to the repeat unit in the single-strand portion of human telomeric DNA and thus complementary to a sequence believed to be present in the RNA component of human telomerase. The primer also comprised, at its 5'-end, a sequence corresponding to a restriction enzyme recognition site. However, when the cDNA produced by the reverse transcription reaction and PCR amplification was examined by gel electrophoresis and nucleotide sequence analysis of the bands of nucleic acid present in the gel, only ribosomal RNA sequences were detected. Similar problems were encountered when variations of this 5'-RACE approach were attempted using nested primers.

The successful cloning effort began with the preparation of cDNA from purified preparations of human telomerase as well as from cell lines that have human telomerase activity and from cell lines that do not have detectable human telomerase activity. The method used to prepare the cDNA is described in detail in Example 1, below. Two negative selection steps and successive cycles of positive selection were used in conjunction with the cDNA preparations from the two human cell lines to lower the concentration of unwanted sequences and to raise the concentration of the desired RNA component sequences.

The negative selection steps involved the preparation of biotinylated PCR product from cDNA prepared from a human cell line that does not have detectable telomerase activity. The biotinylated PCR product was denatured and then rehybridized in a solution comprising a much lower concentration of non-biotinylated PCR product (100 biotinylated product:1 non-biotinylated product) from cDNA prepared from a human cell line that does have telomerase activity. Given the possibility that the telomerase negative cell line might contain some low amount of the RNA component, the hybridization step was conducted to discriminate or select against only RNA expressed abundantly in both cell lines. After hybridization to a Cₒt selected to allow hybridization of the most abundantly expressed RNA, the unwanted material was removed by binding to streptavidinylated magnetic particles; the supernatant remaining after particle collection contained the desired cDNA for the RNA component of human telomerase. The process for PCR amplification of cDNA is described in Example 2, below.

This material was further enriched for the desired cDNA by successive cycles of positive selection. In the positive selection step, a biotinylated probe complementary to the predicted template sequence in the RNA component of human telomerase was hybridized to PCR product from an enriched (by negative selection) sample of the PCR-amplified cDNA from a human cell line that has telomerase activity. After hybridization, the probe/target complexes were bound to avidinylated magnetic beads, which was then collected and used as a source of nucleic acid enriched in RNA component sequences in further cycles of positive selection. The positive selection process is described in more detail in Examples 3 and 4, below.

After the third cycle of positive selection, the amplification products were separated by gel electrophoresis, and sections of the gel corresponding to nucleic acids ⁻200 bp in size were removed. The nucleic acids were then eluted from the gel sections and amplified by PCR. The PCR amplification products were digested with restriction enzyme NotI and then inserted by ligation into the NotI site of plasmid pBluescriptIISK+, commercially available from Stratagene. The resulting plasmids were transformed into *E. coli* host cells, and individual colonies were isolated and used as a source of nucleic acid for further analysis and DNA sequencing. A number of clones positive by this test were then analyzed by DNA sequencing and a variety of other tests.

These other tests included the following: (1) determination whether antisense oligonucleotides complementary to the putative RNA component would inhibit telomerase activity in human cell extracts known to contain telomerase; (2) determination whether PCR primers specific for a putative RNA component clone sequence could be used to amplify a nucleic acid present in a telomerase sample and whether the product observed, if any, would track telomerase activity during purification of telomerase; and (3) determination whether PCR primers specific for a putative RNA component clone sequence could be used to amplify a nucleic acid present in greater abundance in cell extracts from cells in which telomerase activity is known to be high (i.e., tumor cells) than in cell extracts from cells known to produce no or only low amounts of telomerase activity. One clone, designated plasmid pGRN7, produced results in these tests consistent with the determination that the plasmid comprised cDNA corresponding to the RNA component of human telomerase.

Thus, antisense oligonucleotides corresponding to sequences of the putative RNA component sequence of pGRN7 exhibited inhibition of telomerase activity in vitro. Likewise, when telomerase was purified from cell extracts by a process involving (1) DEAE chromatography; (2) Sephadex S300 chromatography; and (3) either glycerol gradient, SP sepharose, or phenyl sepharose separation and fractions collected, PCR primers specific for the putative RNA component sequence of pGRN7 amplified a nucleic acid of the appropriate size, and the amount of amplification product correlated well with the amount of telomerase activity observed in the fractions collected. Finally, cell extracts from normal (no detectable telomerase activity) and cancer (telomerase activity present), as well as testis (telomerase activity present), cells showed corresponding amounts of PCR product upon reverse transcription and PCR amplification (RT-PCR) with primers specific for the putative RNA component comprised in pGRN7. The protocol for the RT-PCR is described in Examples 5 and 6, below.

The above results provided convincing evidence that the RNA component of human telomerase had been cloned, so plasmid pGRN7 was then used to isolate a genomic clone for the RNA component from a human cell line, as described in Example 7, below. The genomic clone was identified in and isolated from a genomic library of human DNA inserted into a lambda vector FIXII purchased from Stratagene. The desired clone comprising the RNA component gene sequences contained an ⁻15 kb insert and was designated clone 28-1. This clone has been deposited with the American Type Culture Collection and is available under the accession No. 75925. Various restriction fragments were subcloned from this phage and sequenced. The gene has also been localized to the distal end of the q arm of chromosome 3. The sequence information obtained from a SauIIIA1 restriction enzyme recognition site at one end of the ⁻15kb insert to an internal HindIII restriction enzyme recognition site, which comprises all of the mature RNA component sequence as well as transcription control elements of the RNA component gene, of lambda clone 28-1 is shown supra.

### Example 1

### Preparation of PCR-amplifiable cDNA

RNA was obtained from 293 and IMR-90 cells by guanidine-thiocyanate extraction or from purified telomerase fractions by phenol/chloroform extractions. The total RNA from 293 cells was size fractionated on a 2% agarose gel, and the RNA sized less than 500 bp was isolated.

First strand cDNA synthesis was performed with Superscript™ II reverse transcriptase obtained from Bethesda Research Laboratories (BRL). About 0.5 to 1.0 µg RNA was mixed with about 40 ng of random primer (6 mer; pdN₆) in water at a total volume of 11 µl. The solution was heated for 10 min. at 95°C and then cooled on ice for 5-10 min. The denatured nucleic acid was collected by centrifugation. The denatured RNA and primer mixture were then resuspended by adding, in the order shown: 4 µl 5x 1st strand synthesis buffer; 2 µl 0.1 M dithiothreitol (DTT); 1 µl RNAsin (Pharmacia); and 1 µl dNTP (2.5 mM each stock solution). The reaction mixture was incubated at 42°C for 1 min., and then, 1 µl (200 units) of Superscript™ II RTase (BRL) was added and mixed into the reaction, which was then incubated for 60 min. at 42°C. The resulting reaction mixture, containing the newly synthesized cDNA was placed on ice until second strand synthesis was performed.

Second strand cDNA synthesis was performed as follows. About 20 µl of the reaction mixture from the first strand cDNA synthesis reaction mixture (from above) was mixed with, in the order shown, the following components: 111.1 µl of water; 16 µl of 10x *E. coli* DNA ligase buffer; 3 µl of dNTP (2.5 mM each stock); 1.5 µl of *E*. *coli* DNA ligase (15 units from BRL); 7.7 µl of *E*. *coli* DNA polymerase (40 units from Pharmacia); and 0.7 µl of *E. coli* RNase H (BRL). The resulting solution was gently mixed and incubated for 2 hours at 16°C, at which time 1 µl (10 units) of T4 DNA polymerase was added to the reaction tube and incubation continued for 5 min. at the same temperature (16°C). The reaction was stopped, and the nucleic acid was collected by extracting the reaction with phenol/chloroform twice, precipitating the nucleic acid with ethanol, and centrifuging the reaction mixture to pellet the nucleic acid.

The cDNA pellet collected by centrifugation was resuspended in 20 µl of TE buffer and ligated to a double-stranded oligonucleotide called "NotAB" composed of two oligonucleotides (NH2 is an amino blocking group): NotA:    5'-pATAGCGGCCGCAAGAATTCA-NH2 NotB:    5'-TGAATTCTTGCGGCCGCTAT-3' The double-stranded oligonucleotide was made by mixing 50 µl of NotA oligonucleotide (100 pmol) with 50 µl of NotB oligonucleotide (100 pmol) in 46.25 µl of water, heating the resulting solution for 5 min. at 95°C, and adding 3.75 µl of 20x SSC buffer while the solution was still hot. The tube containing the mixture was then placed in a beaker containing hot water (at a temperature of about 70 to 75°C), the temperature of which was allowed to drop slowly to below 15°C, so that the two oligonucleotides could hybridize to form the double-stranded oligonucleotide NotAB. The resulting nucleic acid was collected by precipitation and centrifugation.

The double-stranded NotAB oligonucleotide was resuspended in about 30 µl TE buffer and then ligated to the cDNA in a reaction mixture containing 10 µl of the cDNA preparation described above, about 50 pmol (calculated by OD260) of NotAB; 2 µl of 10x T4 DNA ligase buffer; 1.2 µl of T4 DNA ligase; 0.2 µl of 10 mM ATP; and water in a total volume of 20 µl by incubating the reaction mixture at 16°C overnight. The reaction was then heat-inactivated by heating the reaction mixture for 10 min. at 65°C. About 1 to 2 µl of the resulting mixture was typically used for PCR amplification; one can amplify the ligation mixture for 10 to 15 cycles (94°C, 45 seconds; 60°C, 45 seconds; and 72°C, 1.5 min.) and save as a stock, as described in Example 2.

### Example 2

### PCR Amplification of cDNA

The cDNA was routinely amplified by preparing an amplification reaction mixture composed of 5 µl of 10X PCR buffer (500 mM KCl; 100 mM Tris, pH=8.3; and 20 mM MgCl₂; 5-8 µl of dNTP (2.5 mM each); 1 µl of Tag polymerase (Boehringer-Mannheim); 0.1 µl of gene 32 protein (Boehringer-Mannheim); 6 µl of Not B primer (20 µM stock); 2 µl of the cDNA (prepared as described in Example 1), and water to 50 µl. This mixture was then overlayed with 50 to 100 µl of mineral oil, and PCR amplification was performed for 10 to 15 cycles of 94°C, 45 seconds; 60°C, 45 seconds; and 72°C, 1.5 min. After amplification, the reaction mixture was extracted with phenol/chloroform, and the amplified nucleic acid was precipitated with ethanol and collected by centrifugation. The precipitate was then dissolved in 100 µl of TE buffer to prepare a stock solution.

### Example 3

### PCR Amplification for Subtractive Hybridization and Cyclic Selection

To make PCR product for both subtractive hybridization and cyclic selection, about 1 µl of a stock solution prepared as described in Example 2 was amplified in 50 µl of PCR reaction mixture prepared as described in Example 2, except that 21-24 cycles of primer annealing, extension, and denaturation of product were conducted. After amplification, reaction mixtures were extracted with phenol/chloroform, precipitated with ethanol, and collected by centrifugation. Product yield was estimated by staining with ethidium bromide after agarose gel electrophoresis of a small aliquot of the reaction mixture. For subtraction hybridization, a cDNA library from telomerase negative cells (IMR-90) was amplified in the presence of biotinylated dUTP. An approximately 100 to 1 ratio of cDNA from telomerase negative cells (IMR-90) to cDNA from telomerase positive cells (293) was used to perform subtraction hybridization. Standard conditions were used at 65°C for 48-72 hours. Typically, about 2 µg of the nucleic acid product from partially purified cDNA and negatively selected material was used for at least two rounds of cyclic selection.

After cyclic selection, described in Example 4, about 1 to 2 µl of the selected "pull-down" products (out of a total volume of 20 µl) were PCR amplified as described in Example 2 for 22 cycles, precipitated with ethanol, and collected by centrifugation in preparation for further cyclic selection.

### Example 4

### Positive Selection of PCR-amplified cDNA

For the positive selection step of the cyclic selection process used to clone the RNA component of human telomerase, about 2 µg of the PCR-amplified cDNA were diluted into 25 µl of TE buffer and then mixed with 1.25 µl of 20X SSC and the resulting solution heated to 95°C for 3 min. The temperature was lowered to 60°C for 5 min., and one µl (0.1 µg/µl) of the R2 or R4 biotinylated probe was added. The sequences of these probes are shown below. The probes are O-methyl-RNA probes, so U is O-methyl-uridine, A is O-methyl-riboadenine, G is O-methyl-riboguanine, and I is inosine. R2: 5'-UUAGGGUUAGII-biotin R4: 5'-AUUGGGUUAUII-biotin The R2 probe is specific for the telomere repeat, and the R4 probe is specific for RNase P, which was used to track the effectiveness and efficiency of the cyclic selection process. By carrying out a cyclic selection simultaneously but separately for RNase P RNA, a molecule of known sequence, one can have greater confidence that the cyclic selection process is functioning properly with respect to the molecule of interest, in this case the RNA component of human telomerase.

After either the R2 or R4 probe was added to the mixture at 65°C, the temperature of the hybridization reaction mixture was lowered to 30°C by incubating the mixture at that temperature for 5 min., and then the reaction mixtures were further lowered to a temperature of 14°C by incubating at that temperature for 60 min. Finally, the mixture was incubated at 4°C for 2-12 hours.

The entire hybridization reaction mixture for each sample (R2 or R4) was added to 400 µl of 0.5X SSC at 4°C and then added to a tube of ice-cold magnetic beads, which were purchased from Promega and pre-washed four times with 0.5x SSC before use. The resulting mixture was incubated 30 min. at 4°C to ensure complete binding to the magnetic beads. Each reaction tube was then incubated briefly at room temperature on the magnetic stand (Promega) to pull down the beads. The beads were resuspended in cold 0.5X SSC (600 µl) and placed (in a tube) on ice. The samples were washed three more times with 0.5X SSC in this manner. Nucleic acid was eluted from the beads by resuspending the beads in 100 µl of water and incubating for 2 min. at 65°C before placing the beads back on the magnetic stand for collection. This process was repeated three more times; the last time, the resuspended beads were incubated for 5 min. at 65°C before placing the beads on the magnetic stand for collection. All of the 100 µl supernatants (for each sample) were pooled and dried down to 20 µl in a SpeedVac™ centrifuge. The recovered DNA was then PCR amplified for another round of amplification and selection. After each amplification, the PCR products were phenol-chloroform extracted twice, ethanol precipitated, and resuspended in 20 µl of TE buffer.

Typically, PCR amplifications were verified by agarose gel electrophoresis. In addition, a variety of controls were used to monitor the cyclic selection process. As one control, PCR "arms" (oligonucleotides of defined sequence that serve as primer hybridization sites) were placed on a nucleic acid that comprised a neomycin resistance-conferring gene. The resulting nucleic acid was mixed with the PCR-amplified cDNA and monitored at each selection by quantitative PCR. As another control, RNase P was followed in both the RNase P selected and the telomerase RNA component selected libraries.

### Example 5

### RT-PCR Protocol

The first strand cDNA was made in substantial accordance with the procedure described in Example 1. Basically, RNA was purified from each telomerase fraction containing 0.1 to 1 µg RNA; typical, about one-third to one-fifth of the RNA made from a 300 µl fraction was used. The RNA was mixed with 40 to 80 ng random hexamer in 10 µl, denatured for 10 min. at 95°C (using a thermal-cycling instrument), and chilled on ice. The denatured RNA and 6-mer were added to a reaction mixture containing 4 µl of 5x 1st strand synthesis buffer supplied by the manufacturer of the reverse transcriptase (RTase, purchased from BRL), 2 µl of 0.1 M DTT, 1 µl of 10 mM dNTP (each), 1 µl of RNase inhibitor (Pharmacia), and water to a total volume of 9 µl. The combined mixture was placed into a 42°C water bath. After 1-2 min. incubation, 1 µl of Superscript™ II RTase (BRL) was added to the mixture. The incubation was continued for 60 min. at 42°C. The reaction was stopped by heating the tube for 10 min. at 95-98°C. The first strand cDNA was collected by brief centrifugation, aliquoted to new tubes, quickly frozen on dry ice, and stored at -80°C or used immediately.

### Example 6

### PCR Amplification of cDNA with a Specific Primer Set

For a 20 µl PCR reaction with radioactively labeled nucleotides, 1 µl of the cDNA prepared in accordance with the procedure of Example 5 was mixed with 20 pmol of primer 1, 20 pmol of primer 2, 2.5 µl of 2.5 mM dNTP, 5 µCi of alpha-³²P-dATP, 2 units of Taq polymerase (Boehringer-Mannheim), 0.2 µg of T4 gene 32 protein (Boehringer-Mannheim), 2 µl of 10x buffer (500 mM KCl, 100 mM Tris-HCl-pH8.3, and 20 mM MgCl₂), and water to a total volume of 20 µl. One drop of mineral oil was then added to the tube.

The PCR amplification conditions for the telomerase RNA component clone were: 94°C for 45 sec., 60°C for 45 sec., 72°C for 1.5 min. The number of cycles differed depending on the type of purified materials used for RNA preparation but typically range from 18 to 25 cycles. As for all quantitative RT-PCR, several reactions with differing cycles were run for each sample to determine when the PCR amplification became saturated and non-linear.

For the RNase P used as a control, the PCR amplification conditions were: 94°C for 45 sec., 50°C for 45 sec., and 72°C for 1.5 min. Again, the number of cycles ranged from 15 to 22 cycles, depending on the nature of the samples. The sequences of the primers used for RNase P amplification are shown below: P3: 5'-GGAAGGTCTGAGACTAG-3' P4: 5'-ATCTCCTGCCCAGTCTG-3' The PCR product obtained with these two primers is about 110 bp in size.

After PCR, the products (5 to 10 µl of the reaction mixture) were loaded onto a 6% native polyacrylamide gel and electrophoresed. After electrophoresis, the gel was dried and exposed to a PhosphorImager™ cassette or to autoradiographic film for analysis.

### Example 7

### Cloning the Gene for the RNA Component of Human Telomerase

The procedures used to clone the gene for the RNA component of human telomerase were carried out as generally described in Maniatis et al., Laboratory Molecular Cloning Manual. A genomic DNA library of DNA from the human lung fibroblast cell line WI-38 inserted into phage lambda vector FIXII was purchased from Stratagene. The phage were plated at a concentration of about 25,000 plaques per plate onto three sets of 15 (150 mm) plates. The plates were made with NZY agar and NZY top agarose; the cells used for the phage transformation were XL1BlueMRAP2 cells; and the transformants were grown overnight for about 16 hours at 37°C. The plates were then chilled at 4°C for about an hour, and then the plaques were "lifted" onto C/P nylon circles (filter paper from Bio Rad). This process was repeated to produce a duplicate set of lifted filters. The filters (in duplicate) were denatured, neutralized, equilibrated in 6x SSC buffer, exposed to UV irradiation to cross-link the nucleic acid to the filter, and then dried on blotter paper.

Prehybridization was conducted for one hour at 37°C in 50% formamide buffer. The filters were probed with an ⁻218 bp, radioactively-labeled, NotI fragment from clone pGRN7, which had been isolated by electroelution from a 5% polyacrylamide gel after separation by electrophoresis and then nick-translated with alpha-³²P-dCTP using a nick-translation kit from Boehringer-Mannheim Biochemicals in accordance with the manufacturer's instructions. About 25 ng (⁻10 µCi label) of the probe were used per filter, and hybridization was conducted overnight at 37°C in 50% formamide hybridization buffer. After hybridization, the filters were washed at room temperature six times; the first three washes were with 6x SSC containing 0.1% SDS, and the last three washes were with 6x SSC alone. After an initial exposure of several duplicate filters in a PhosphorImager™ cassette to check hybridization efficiency and signal strength, the filters were washed at 65°C in 0.5x SSC. The filters were then placed under Kodak XAR5 film using two intensifier screens and allowed to expose the film for about 100 hours at -70°C.

One strong signal emanated from the filter containing a phage, later designated 28-1, comprising the gene for the RNA component of human telomerase. The plaque corresponding to the signal observed on the filter was used to make secondary plates, so that an isolated plaque (confirmed by probing with labeled pGRN7 nucleic acid) could be cultured for large-scale isolation of the phage DNA. Phage 28-1, available from the American Type Culture Collection under accession No.75925, comprises an ~15 kb insert and comprises several restriction fragments that contain sequences that hybridize with RNA component sequences on pGRN7: a 4.2 kb EcoRI restriction enzyme fragment; a 4.2 kb ClaI restriction enzyme fragment, and a 2.5 kb HindIII-SacI restriction enzyme fragment. The latter fragment comprises the entire ⁻560 nucleotide sequence of the RNA component shown above and is believed to comprise the complete gene for the RNA component. The plasmid comprising the 2.5 kb HindIII-SacI restriction enzyme fragment in the pBluescript vector was designated plasmid pGRN33 and is available from the American Type Culture Collection under the accession No. ATCC 75926. To the extent the human gene may comprise sequences other than those on the 2.5 kb fragment, those sequences can be isolated from phage 28-1 or from other phage clones identified by probing with the 2.5 kb fragment (or another probe of the invention). The restriction enzyme fragments noted above were prepared in separate restriction enzyme digests; the products of the digests were separated by electrophoresis on a 0.7% agarose gel or, for the ⁻2.5 kb fragment only, a 3% polyacrylamide gel; and the desired bands were cut from the gel and prepared for subcloning either by using the GeneClean™ Kit II (from Bio101, Inc.) or by electroelution into Spectropor #2 dialysis tubing in 0.1x TBE at 100 V for two hours (for the ⁻2.5 kb fragment only).

These restriction enzyme fragments were subcloned into *E. coli* expression/mutagenesis plasmids derived from pUC-based plasmids or from pBluescriptII plasmids that also comprise an SV40 origin of replication (but no SV40 promoter activity). The resulting plasmids can be used to prepare altered (mutated) RNA component nucleic acids for introduction into human or other eukaryotic cells for a variety of purposes, as described above in the Description of the Preferred Embodiments.

### Example 8

### Antisense Plasmids for the RNA Component of Human Telomerase

Antisense expression plasmids were prepared by PCR amplification of RNA component cDNA using the following primer sets: (1) NotB and G1, which produces an antisense nucleic acid that is smaller than the cDNA insert in the plasmid; and (2) NotB and R3C, which produces a full-length (relative to the insert in the plasmid) antisense nucleic acid. The nucleotide sequence of NotB is shown in Example 1, above; the nucleotide sequences of the G1 and R3C primers are shown below. G1:        5'-GAGAAAAACAGCGCGGGAGCAAAAGCA-3' R3C: 5'-GTTTGCTCTAGAATGAACGGTGGAAG-3' After PCR amplification, the amplified fragments were cloned into an ⁻10 kb expression plasmid at a PmlI site; the plasmid comprises puromycin resistance-conferring, DHFR, and hygromycin B resistance-conferring genes as selectable markers, the SV40 origin of replication; the inducible human metallothionein gene promoter positioned for expression of the antisense strand of the gene for the RNA component of human telomerase (one could also use a stronger promoter to get higher expression levels), and the SV40 late poly A addition site.

The resulting plasmids (designated pGRN42 for the NotB/G1 product and pGRN45 for the NotB/R3C product) were transfected by the calcium phosphate procedure (see Maniatis et al., supra) into the fibrosarcoma cell line HT1080. HT1080 cells are normally immortal; expression of the antisense RNA for the RNA component of human telomerase should prevent the RNA component of human telomerase from association with the protein components, blocking the formation of active telomerase and rendering the cells mortal.

### Example 9

### Identification and Isolation of RNA Component Nucleic Acids from Non-human Mammals

To illustrate how the reagents of the invention can be used to identify and isolate substantially homologous nucleic acids from other mammalian species, PCR primers complementary to human RNA component sequences were used to amplify homologous sequences in a PCR. An illustrative primer pair used to demonstrate this aspect of the invention is composed of primer +10, which has the sequence 5'-CACCGGGTTGCGGAGGGAGG-3', and primer R7, which has the sequence 5'-GGAGGGGCGAACGGGCCAGCA-3'. Genomic DNA was prepared from chimpanzee, squirrel monkey, rhesus monkey, and baboon tissue and dissolved in TE buffer at a concentration of about 0.5 - 4 mg/ml.

For each tissue type, a PCR mixture was prepared, which mixture comprised: 1 µL of genomic DNA, 48 µL of Master Mix (Master Mix is composed of 1x TaqExtender™ buffer from Stratagene, 200 µM of each dNTP, and 0.5 µM of each primer), and 0.5 µL of a 1:1 mixture of Taq polymerase (5 Units/µL, Boehringer Mannheim):Tth polymerase (TaqExtender™ polymerase, from Stratagene). The reaction tubes were loaded onto a thermal cycler, which was programmed to first heat the reaction mixture at 94°C for 5 minutes and then to perform 27 cycles of incubations at 94°C for 30 sec., 63°C for 10 sec., and 72°C for 45 sec. After the amplification reaction was complete, about 10 µL of each reaction mixture were loaded onto a 2% agarose gel for electrophoresis. After electrophoresis, staining of the gel, and UV irradiation, one could observe that each reaction mixture contained a band of the predicted (~200 bp) size. Nucleic acids from these bands can be cloned and sequenced and the remainder of the RNA component genes from each of these mammalian species can be cloned as described above for the gene for the RNA component of human telomerase. Example 14, infra, describes a specific example of squirrel monkey telomerase RNA gomonent gene sequencing.

### Example 10

### Mutated Sense Human Telomerase RNA Component Sequences

This example demonstrates that changing the telomerase RNA component sequence at the template region alters the sequence synthesized by human telomerase, leading to changes in the chromosomal telomerase repeats.

To determine if re-programming the TRC3 template region would produce leads to corresponding changes in the telomere repeat synthesized in human telomerase activity, a gene fragment that expresses the entire telomerase RNA component (TRC3) gene sequence was cloned and mutagenized. Southern blot analysis showed that TRC3 hybridized to a single-copy gene in the human genome. A genomic copy of TRC3 was isolated from a lambda phage library and shown to have the same restriction map as that observed on genomic Southern blots probed with TRC3. A 2.6 kb *Hind*III-*Sac*1 fragment was isolated and subcloned into a modified version of pBluescript, generating the genomic TRC3 plasmid pGRN33. The 5' and 3' ends of the RNA were mapped using primer extension, RACE PCR, and RT-PCR. The size of the RNA transcript was ≈550 bases, consistent with its migration on Northern blots. The transcribed region for TRC3 was central to the genomic clone with 1.4 kb of upstream sequence.

RNA was extracted from purified telomerase preparations and was then used in the following experiments. 5' RACE: First strand cDNA was made using antisense TRC3 primers (R3B: 5'-CAACGAACGGCCA GCAGCTGACATT) in the presence of ³²P-dATP. Extension products were resolved by PAGE and were identified by autoradiography, excised and extracted. An oligonucleotide (NotA: 5'-pATAGCGGCCGCTT GCCTTCA-NH₂) was ligated to these first strand products using T4 RNA ligase and PCR was then performed using a nested primer, R3c (5'-GTTTGCTCTAGAATGAACGGTGGAAG), and an oligonucleotide (NotB: 5'-TGAATTCTTGCGGCCGCTAT) complementary to the NotA oligonucleotide. The PCR products were resolved on an agarose gel and bands were excised and sequenced directly, using oligonucleotide G1 (5'-AGAAAAACAGCGCGCGGGGAGCAAAGCA) as a primer. 3' end mapping: Attempts to perform 3' RACE were unsuccessful. Subsequently, an RT-PCR strategy was employed in which a series of antisense primers complementary to the genomic DNA sequence were used to prime first strand cDNA synthesis. The primers in this series were spaced at approximately 150 bp intervals starting from the 5' end of the transcript, proceeding towards the 3' end. PCR was then performed using the first strand primer and a primer whose sequence was internal to the known TRC3 transcript (F3b: 5'-TCTAACCCTAACTGAGAAGGGCGTAG). Reverse transcriptase-sensitive PCR bands of the predicted size were seen when using cDNA made with all primers designed to the interval +100 to +450 (numbering relative to 5' end) but not with any of the primers designed to +558 to +950. This placed the putative 3' end of the mature TRC3 transcript between position +450 and +558. Subsequent rounds of primer design and RT-PCR narrowed this interval to between +545 and +558.

The predicted template sequence of TRC3 was altered from CUAACCCUA to CCAACCCCA (MuC) or CAAACCCAA (MuA) by *in vitro* mutagenesis (performed substantially according to Perez et al. (1994) J. Biol. Chem. 269: 22485) of the genomic plasmid pGRN33. If incorporated into functional telomerase, these mutant RNAs should template the synthesis of TTGGGG (MuC) or TTTGGG (MuA) rather than wild-type repeat TTAGGG. These mutant telomerase activities could be easily distinguished from wild-type activity since they would no longer require dATP for activity and only the wild-type activity should be sensitive to termination by ddATP. A double mutant (MuC+17) was also prepared in which a 17 bp insertion was present at +180 bp in addition to the MuC template. This mutant allowed specific detection of the altered RNA with a probe to the 17 bp insertion or by size.

The 2.6 kb of genomic sequence was sufficient for expression of TRC3 *in vivo.* Cells were transiently transfected with the MuC+17 plasmid and RNA expressed from the transfected DNA was detected by RT PCR using the 17 bp insert sequence as the primer in the reverse transcription step. The RNA was detected in MuC+17-transfected cells but not in mock transfected cells, indicating that the 2.6 kb genomic clone was sufficient for TRC3 expression. Stable transformants were then derived from each of these three mutant plasmids by calcium phosphate transfection of HT1080 cells along with pCMV*neo*. Resistant clones were selected in G418 and expression of the MuC+17 RNA was verified by RT-PCR (Irving et al. (1992) Exp. Cell Res. 202: 161).

To test for mutant telomerase activity, the extracts were assayed from untransfected cells and from three stable transformants with integrated MuC*, MuC, or MuA vectors (C*, C, or A in Fig. 1). Since the mutant extracts were expected to contain both wild-type and mutant telomerase activities, various assay conditions were employed to distinguish between them. Under normal reaction conditions (dTTP, ³²P-dGTP and dATP), all three extracts from the mutant construct series showed wild-type telomerase activity that was sensitive to RNase (Fig. 1, lanes 1-6). As expected, this activity was unaffected when ddCTP was included in the reactions (Fig. 1, lanes 7-9) and was abolished by ddTTP (lanes 10-12). In contrast, when ddATP was substituted for dATP, the C (lane 14) and A (lane 15) extracts still displayed RNase-sensitive (lanes 17 and 18) telomerase activity, while C* (lane 13) and control extract did not. These results indicate that the ddATP-resistant activities represent telomerase was reprogrammed with MuC or MuA TRC3 RNA. In contrast, the 17 bp insertion in C* inhibits reconstitution, indicating that telomerase reconstitution is very specific for the TRC3 sequence.

To confirm that the sequence synthesized by the mutant MuA was (GGGTTT)n, we modified the existing PCR methodology for amplifying telomerase repeats added onto a unique telomerase primer (Kim et al. (1994) Science 266: 2011). Using synthetic primers, we identified reaction conditions where a 3' primer with the sequence d(CCCAAACCCAAACCCAA) would only amplify (TTTGGG)n repeats and would not amplify (TTAGGG)n containing repeats.

To distinguish between telomeric repeats added by wild-type versus mutated telomerase, a two-step assay was combined with a strategy for limiting the availability of nucleotides for the telomerase reaction. Since MuA and MuC would generate telomeric repeat sequences of (TTTGGG)ₙ and (TTGGGG)ₙ, respectively, cell extracts were first incubated with the TS substrate in the presence of only dTTP and dGTP for 10 min at room temperature to allow for the addition of telomeric repeats. Residual telomerase activity was then destroyed by boiling the extracts for 5 min. Telomerase products with specific DNA sequence were then' detected by PCR amplification using the appropriate reverse primers and in the presence of all 4 dNTP's and trace amounts of ³²P-dCTP as described (Kim et al. (1994) op.cit). To detect the MuA products, the reverse primer was (ACCCAA)₄ and the PCR conditions were 94°C, 10 sec, 60°C, 30 sec and 72°C, 30 sec for 20 cycles. To detect the MuC products, three reverse primers were used: (CCCCAA)₃, (CCAACC)₃ and (CCAACC)₃, respectively, which gave PCR products with the corresponding mobility shifts consistent with the expected position of annealing to the telomerase products. The PCR conditions used were the same as above, except that the annealing temperature was 50°C. Under the same conditions, no telomerase products were generated from extracts of parental cells or cells transfected with the wild-type TRC-3 gene. In tests of the specificity of our PCR amplification conditions, synthetic oligonucleotides containing (TTTGGG)ₙ and (TTGGGG)ₙ generated the appropriate 6 nt ladder PCR products with (ACCCAA)₄ or (CCCCAA)₃ reverse primer respectively, whereas (TTAGGG)ₙ oligonucleotides did not produce any PCR products with the (ACCCAA)₄ or (CCCCAA)₃ reverse primer

Using these conditions, extracts from MuA but not from MuC or wild type cells generated products in the modified telomerase assay, indicating that telomerase from MuA containing cells generated (TTTGGG)n repeats. Similar methods were used to analyze the MuC mutant, which synthesized (TTGGGG)n repeats. Together, the above data constitute strong evidence that the TRC3 gene encodes the RNA component of human telomerase, and thus we have renamed TRC3 as hTR for human Telomerase RNA.

### Example 11

### Telomerase RNA Component in Mortal and Immortal Cells

Most cancer cells express high levels of telomerase activity, while in most normal somatic human cells, telomerase is not detected (Kim et al. (1994) op.cit). To determine if telomerase RNA component levels are elevated in immortal cancer cell lines, telomerase RNA component and GAPDH transcript levels were analyzed using RT-PCR in five mortal primary cell strains, which lacked detectable telomerase activity, and five immortal cancer cell lines with high levels of telomerase activity. The steady state levels of telomerase RNA component transcripts was 3- to 12-fold higher in the tumor cell lines than in primary cells when compared to the levels of GAPDH (Fig. 2A). While telomerase RNA component levels were increased in the immortal cancer cells expressing high levels of telomerase, it is interesting that low but readily detectable levels of telomerase RNA component were also present in mortal primary cells with no detectable telomerase activity (lanes 1-5).

The telomerase RNA component levels were also examined in a variety of normal human tissues by Northern blot analysis. Testes and ovary had the highest level of telomerase RNA component, which was expected since these tissues express high levels of telomerase activity. However, a number of other tissues also expressed telomerase RNA component (Figs. 2B and 2C). These include normal kidney, prostate, and adult liver, all of which lack detectable levels of telomerase activity. These results confirm the data from cell lines (Fig. 2A) and suggest that telomerase RNA may be present but inactive in a number of human tissues. Similar tissue-specific differdntes in RNA expression are seen in mouse tissues; however, many normal mouse tissues are positive for telomerase activity. The apparent increased repression of telomerase activity in human cells may help explain why mouse cells spontaneously immortalize in culture while human cells do not.

### Example 12

### Expression of antisense hTR (human telomerase RNA component) transcripts in HeLa cells leads to cell crisis and cell death

To examine the function of telomerase in an immortalized cell, antisense hTR expression constructs were introduced into HeLa cells. A 200 bp EcoRI DNA fragment containing 1 to 193 bp of a human telomerase RNA component cDNA clone, TRC3, was inserted into the EcoRI site of p10-3 and pBBS212 to generate plasmids p10-3-hTR and pBBBhTR, respectively. Plasmid p10-3-hTR expresses the antisense of telomerase RNA component under the transcriptional control of the tetracycline-regulated CMV minimal promoter relative to the five Tet-operators upstream in two different orientations as described (Gossen et al. (1992) Proc. Natl. Acad. Sci. (USA) 89: 5547). Plasmid pBBS-hTR expresses the antisense of hTR under the control of the MPSV promoter (Lin et al. (1994) Gene 147: 287). In parallel, control expression vectors lacking the antisense hTR coding sequence were also electroporated into HeLa cells. Clones containing antisense or control plasmids were selected in three separate experiments. Initially, the 41 cultures expressing antisense hTR grew identically to cells with the control vector. However, at 23 to 26 population doubling levels (PDL) post-transfection, 33 out of 41 antisense expressing cultures underwent crisis (Table I). Cell crisis in these cultures was characterized by a marked inhibition in cell growth from 20 to 26 PD, followed by the rounding up and detachment of cells from the plate over a period of a week. In 28 out of 33 cases in which the cells underwent crisis, rare (< 1%) revertant colonies were observed within three weeks after most of the cells had died. The revertant cells may represent variants that escape the inhibiting effect of the antisense hTR construct. In contrast to the antisense clones, none of the vector control cell lines had any change in growth or mortality over 50 doublings.

**Table I**

| Antisense hTR leads to shorter mean TRF and cell crisis | | | | | |
|---|---|---|---|---|---|
| Three independent experiments were carried out in which HeTe7 cells (HeLa cells that express high levels of the tetracycline repressor-VP16 chimeric protein) were transfected by electroporation with either plasmid p10-3hTR, expressing antisense hTR under control of the tetracycline-VP16 induced CMV minimal promoter or pBBS-hTR, expressing antisense hTR under the control of the MPSV promoter (54). [In these experiments no regulation by tetracycline was observed. The experiments with p10-3-hTR were typically carried out in the absence of tetracycline, because control experiments with luciferase or antisense hTR under the control of the tetracycline-VP16-induced CMV minimal promoter demonstrated that the presence or absence of tetracycline had little effect on luciferase or antisense hTR expression in HeTe7 cells. Indeed, in early experiments with antisense hTR constructs in HeTe7 cells, the cells still underwent crisis at 23 to 26 PDL in the presence of tetracycline. ]. As a control, HeTe7 cells were also transfected with the parental vector under the same conditions. Eleven to eighteen stable clones were isolated from each transfection series. Clones from all isolates displayed identical morphology and growth profiles until 20 PDL, at which time the growth of most of the antisense-expressing clones slowed markedly (p10-3-hTR and pBBS-hTR). By PDL 23-26, these cells underwent crisis, characterized by the appearance of enlarged and rounded cells. However, not all of the clones generated from the pBBS-hTR transfected HeTe7 cells underwent crisis; eight clones expressing antisense hTR continued growing similar to the control cultures. Cells from all clones were harvested at PDL 23 and the mean TRF lengths were determined. The P values were calculated by the method of unpaired t-test. For each series, the proportion of clones that underwent crisis is indicated. | | | | | |

| | Plasmid | Cell Crisis | Mean TRF | P Value | Crisis/Total |
|---|---|---|---|---|---|
| 1 | p10-3 | no | ND | | 0 / 7 |
| | P10-3-hTR | yes | ND | | 18 / 18 |
| 2 | p10-3 | no | 3.27 ± 0.10 | | 0 / 12 |
| | p10-3-hTR | yes | 2.72 ± 0.07 | 0.0003 | 11 / 11 |
| 3 | pBBS | no | 3.22 ± 0.11 | | 0 / 13 |
| | pBBS-hTRa | yes | 2.39 ± 0.10 | 0.0008 | 4 / 4 |
| | pBBS-hTRb | no | 3.03 ± 0.20 | 0.3551 | 0 / 8 |
| | HeTe7 cells | no | 3.15 ± 0.09 | | Parental cells |

To determine if telomere length and telomerase inhibition correlated with cell crisis in the antisense expressing clones, telomere length and telomerase activity in several precrisis control and experimental colonies were assayed at 23 PDL. All colonies containing control vector had mean TRF lengths (3.22 and 3.27 kb) similar to the parent cell line (3.15 kb), whereas clones containing the antisense vector constructs that underwent crisis had mean TRF lengths between 2.39 and 2.72 kb or 17 to 26% shorter than those of the parental line (Fig. 3). These data are consistent with telomere repeats being lost in the antisense containing clones due to the inhibition of telomerase activity. To test this directly, telomerase activity was assayed in 14 of the clones. Telomerase activity was generally low but detectable in many of the antisense clones, although the shortened telomeres suggest that the level was not sufficient to maintain telomere length since mean TRF fell from 3.22 to 2.39 kb (P=0.0008). In the eight clones containing the antisense vector (pBBS-hTRb) that did not undergo crisis, telomere length was not significantly changed (3.03 versus 3.33, P=0.355), and telomerase activity was similar to that of controls. Taken together, these results are evidence that telomere loss leads to crisis and cell death once telomeres reach a critical length.

The induction of cell crisis in HeLa cells expressing antisense telomerase RNA component provide further support that telomerase inhibition can provide a specific and effective therapeutic against human cancer.

### Example 13

### In Situ Amplification and Detection

### In situ detection of telomerase RNA

### A. Fluorescent in situ hybridization (FISH)

Identification of telomerase positive cells in a mixed population of cells or tissues can be performed by in situ hybridization of labeled probe targeted at the RNA component of telomerase. A tissue of cell samples fixed onto a microscopic glass slide and permeabilized sufficiently and used for in situ hybridization. An example of in situ hybridization for human telomerase RNA (hTR) consist of first denaturing the nucleic acids by immersing the slides in 70% deionized formamide/ 2X SSC solution pre-warmed to 70-74°C for 2-3 min. The slides are then transferred to ice-cold 70% EtOH, and then to 95% EtOH, and then to 100% EtOH (4 min. in each solutions). 100-200 ng (per slide) of the labeled htRNA probe (-500 bp DNA probe labeled with biotin, digoxigenin, radioisotope, fluorescent tag) is dried, resuspended in 10 µl of 100% deionized formamide, denatured by incubation at 75°C for 8 min., and immediately cooled on ice. Add 10 µl of 2X hybridization buffer (4X SSC; 4X Denhardt's solution; 20% dextran sulfate; 100 mM Tris, pH 7.5) to the 10 µl of resuspended probe. Add the probe/hybridization mix (20 µl) to the fixed sample, overlay with a coverslip, and seal the coverslip with rubber cement or nail polish. Incubate the sample at 37°C for 8-48 hr. After the hybridization, remove the coverslip, wash the sample twice in 2X SSC/50% deionized formamide at 37°C, and then wash twice in 2X SSC at 37°C (5 min. per wash). The sample is then viewed under a microscope.

### B. Primed in situ labeling (PRINS)

Another variation to the traditional in situ hybridization detection method is Primed in situ labeling (PRINS). Detection of the hTR by PRINS consist of initial cDNA synthesis of the hTR by reverse transcriptase (RT) followed by PRINS detection using hTR-specific oligonucleotide probe and chain elongation incorporating labeled nucleotides. RT reaction of the hTR can be performed by a variety of methods. One example of the RT reaction is by using GeneAmp Thermostable rTth Reverse Transcriptase RNA PCR kit (Perkin Elmer). In this method, 10 µl of RT mix (10 mM Tris-HCI pH 8.3; 90 mM KCl; 1 mM MnCl₂; 200 µM dNTPs; 2.5U rTth DNA polymerase; 0.4 µM return primer [e.g., R7G: 5'-GGAGGGGCGAACGGGCCAGCAG-3']) is placed on the fixed and permeabilized sample, sealed with coverslip, anchored with nail polish, overlayed with mineral, and incubated at 70°C for 30 min. Afterwards, the mineral oil is removed by washing for 5 min in xylene, and then 5 min in 100% EtOH. The coverslip is taken off, washed briefly with DepC water, then with 100% EtOH, and air dried for 5 min. Then 10 µl of PRINS mixture (5% (v/v) glycerol; 10 mM Tris-HCl, pH .3; 100 mM KCl; 0.05% (w/v) Tween 20; 0.75 mM EGTA; 2.5 mM MgCl₂; 0.4 µM forward primer [e.g., U3b: 5'-GCCTGGGAGGGGTGGTGGCTATTTTTTG-3']; 200 µM dA, dG, dCTP; 110 µM dTTP; 90 µM labeled dUTP) is placed on the sample. Sealed with coverslip, anchored with nail polish, overlayed with mineral oil, and incubated at 70°C for 30 min to 3 hr. The sample is then washed 3 times in the wash buffer (4X SSC; 0.05% Tween 20) heated to 70°C, for 2 min. The signal is then observed.

### C. in situ RT-PCR

RT-PCR detection of hTR consist of cDNA synthesis of the target RNA by reverse transcriptase reaction (viral reverse transcriptase, or by the intrinsic RT activity of thermostable DNA polymerases), followed by in situ PCR amplification of the target cDNA. Various RT reactions can be used in the cDNA synthesis of the hTR including the RT protocol discussed in Sec. 11.B. Furthermore, the same buffer condition and the primers used in the PRINS detection methods can also be used for RT-PCR, but instead of a final incubation at 70°C for 30 min to 3 hr, the sample is amplified in a thermocycler for 30-40 cycles of 94°C/ 40 sec, 55°C/90 sec (see Sec. 11B). Following PCR, the sample is washed 3 times in the wash buffer (4X SSC; 0.05% Tween 20) heated to 70°C, for 2 min. The signal is then observed.

Another alternative is to amplify the cDNA generated from the initial RT reaction by using the GeneAmp in situ PCR system 1000 and GeneAmp in situ PCR core kit (Perkin Elmer).

One step in situ RT-PCR on a fixed and permeabilized sample can be performed using GeneAmp EZ rTth RNA PCR protocol in combination with GeneAmp in situ PCR system 1000 (Perkin Elmer). This method consists of placing 40-50 µl of EZ RNA PCR buffer mix (50 mM Bicine; 115 mM potassium acetate; 8% (w/v) glycerol, pH 8.2; 300 µM dA, dG, dCTP; 165 µM dTTP; 135 µM labeled dUTP; 5-10U of rTth DNA polymerase; 2.5 mM Mn(OAc)₂; 0.45-1 µM of htRNA-specific primers e.g., R7 and U3b onto a fixed and permeabilized sample on a microscopic slide, and sealing it with the silicone gasket and clip following the manufacturer's protocol (GeneAmp in situ PCR system 1000, Perkin Elmer). The sample is then placed in GeneAmp in situ PCR machine and heated for 120 sec at 94°C, and then amplified for 30-40 cycles of 94°C/45 sec, and 60°C/45 sec. After the amplification, the sample is washed and visualized as discussed supra.

To reduce the background signals that can arise from direct incorporation of fluorescent tags during the PCR amplification, indirect detection that consist of PCR amplification using non-tagged dNTPs followed by in situ hybridization utilizing a tagged hybridization probe specific for the amplified product can be used. In this method, the signal is amplified by any of the RT-PCR method described above without labeled dNTPs or primers, and the amplified product is detected by in situ hybridization.

### D. Application of product extension primer to in situ PCR

The success of in situ PCR depends on preventing leakage of the amplified products inside the cellular matrix out of the cell. Therefore, it is generally true that PCR products smaller than 500 bp are not desirable for in situ PCR. In order to prevent the leakage of the PCR products smaller than 500 bp from the cellular matrix, incorporation of "bulky" dNTPs (e.g., biotin, fluorescent tag, digoxigenin labeled dUTP) into the PCR product is commonly used. Another way to prevent the leakage of a small product in situ PCR would be to incorporate product extension primer into the in situ PCR protocol.

The method comprises using a primer that contains 3-4 6 bp repetitive sequence (e.g. [5'-TTTCCC-3']₃₋₄] at 5' end, followed by a sequence that is specific for the target (see Fig. 4, primer 1), in combination with appropriate return primer (primer 2), and a third primer that consist solely of the repetitive sequence (primer 3, e.g. [5'-TTTCCC-3']₄) to amplify the specific target in situ PCR. The presence of the primer 3 will elongate the PCR product due to the staggered-binding of the primer 3 to 3'-end of the target PCR product. The elongation of the PCR products can be induced by decreasing the annealing temperature of the initial PCR condition.

For example, if the annealing temperature of the target sequence in the primer 1 is 60°C, the sample will be initially amplified for 15-20 cycles of 94°C/45°C and 60°C/45°C, then it will be amplified for 15-20 cycles of 94°C/45°C and 50°C/45°C. Lowered annealing temperature in the second PCR step will favor the generation of the elongated PCR products by increasing the chance of stagger-binding of primer 3 to the repetitive sequences. The resulting elongated PCR products will be less prone to leakage through the cellular matrix, thus resulting in a better signal retention in situ PCR analysis.

### Example 14

### Non-Human Primate Telomerase RNA Component

In order to demostrate that other mammalian telomerase RNA component sequences can be obtained using primers based on the sequence of the human RNA component, human sequence PCR primers were used to amplify telomerase RNA component sequences from genomic DNA of squirrel monkey, a species believed to be one of the species of non-human primates which is the most evolutionarily divergent in comparison to humans.

Squirrel monkey genomic DNA was amplified with primers F3b (5'-TCTAACCCTAACTGAGAAGGGCGTAG-3') and H3+20 (5'-CTCAAGGTCATCGCCAAGGT-3') as described. A single DNA band was observed and was subsequently cloned into the vector pBluescriptII SK (Stratagene, San Diego, CA). The resulting clones were partially sequenced using the PCR method with the reverse universal primer (5'-AACAGCTATGACCATG-3'), primer - 210-3' (5'-TCACGTCTCCTGCCAATTTGC-3'), or primer H3+20. The squirrel monkey telomerase RNA component partial sequences obtained were: 
5'-GGCGCGCTTCCCTGAGCTGTGGACGTGCACCAGGACTCGGCTCACACATGCAGTTCG
CTTTCCTGCTGGTGGGGGACGCCGATCGTGGCCATCCGTCACCCCTCGCCGGCAGTGGG
GGCTTGTGAATCCCTAAATCTGACTGA-3'
 and 
5'-TTTTGAGAGATCATTAAGTATTTAATGAATATTTAGCTAGAAGATCTAAACGAAAATT
CAAGTTGTGTTCCTTTAGTGGTCATCGGTTTATGCCGAAGGTTACAAATTTCTTCTTTGAA
AAATTAGACCATTGGCGATGATCCTTGA-3'
 When aligned to the human telomerase RNA component gene sequence, the following alignment was obtained, using the numbering convention of the human telomerase RNA component gene (hyphens represent gaps introduced to optimize sequence alignment); 
1900
human   CGCGCGGCGCGATTCCCTGAGCTATGGACGTGCACCCAGGACTCGGCTC
monkey      GGCGCGCTTCCCTGAGCTGT-GGACGTGCACC-AGGACTCGGCTC
 
1950
human  ACACATGCAGTTCGCTTTCCTGTTGGTGGGGGGAACGCCGATCGTGCGCA
monkey ACACATGCAGTTCGCTTTCCTGCTGGT-GGGGGGACGCCGATCGTGGCCA
 
2000
human  TCCGTCACCCCTCGCCGGCAGTGGGGGCTTGTGAACCCCCAAACCTGACT
monkey TCCGTCACCCCTCGCCGGCAGTGGGGGCTTGTGAATCCCTAAATCTGACT
 
2050
human GACTGGGCCAGTGTGCTGCAAATTGGCAGGAGACGTGAAGGCACCTCCAA
monkey GA
 and

2300
human   TTTTTTGAGAGATCATTTAACATTTAATGAATATTTAATTAGAAGATCTA
monkey    TTTTGAGAGATCATTAAGTATTTAATGAATATTTAGCTAGAAGATCTA

2350
human  AATGAACATTGGAAATTGTGTTCCTTTAATGGTCATCGGTTTATGCCAGA
monkey AACGAAAATT-CAAGTTGTGTTCCTTTAGTGGTCATCGGTTTATGCCGAA

2400
human   GGTTAGAAGTTTCTTTTTGAAAAATTAGACC-TTGGCGATGA-CCTTGAGC
monkey  GGTTACAAATTTCTTCTTTGAAAAATTAGACCATTGGCGATGATCCTTGA

The foregoing examples describe various aspects of the invention and how certain nucleic acids of the invention were made. The examples are not intended to provide an exhaustive description of the many different embodiments of the invention encompassed by the following claims.

## Claims

1. A substantially pure ribonucleic acid capable of associating with a mammalian telomerase protein component to form an enzymatically active telomerase holoenzyme, the oligoribonucleotide comprising a sequence encoded by the coding region of the ∼2.5 kb HindIII-SacI nsert of plasmid GRN33 (ATCC 75926), or by a sequence that has at least 80% or 95% identity with at least 50 contiguous nucleotides of said coding region.

2. A ribonucleic acid as claimed in claim 1, wherein the sequence is: 
GGGUUGCGGAGGGAGGGUGGGCCUGGGAGGGGUGGUGGCCAUUUUUUGUC
UAACCCUAACUGAGAAGGGCGUAGGCGCCGUGCUUUUGCUCCCCGCGCGC
UGUUUUUCUCGCUGACUUUCAGCGGGCGGAAAAGCCUCGGCCUGCCGCCU
UCCACCGUUCAUUCUAGAGCAAACAAAAAAUGUCAGCUGCUGGCCCGUUC
GCCCCUCCCGGGACCUGCGGCGGGUCGCUGCCCAGCCCCCGAACCCCGCC
UGGAGGCCGCGGUCGGCCGGGGCUUCUCCGGAGGCACCCACUGCCACCGC
GAAGAGUUGGGCUCUGUCAGCCGCGGGUCUCUCGGGGGCGAGGGCGAGGU
UCACCGUUUCAGGCCGCAGGAAGAGGAACGGAGCGAGUCCCGCGCGCGGC
GCGAUUCCCUGAGCUAUGGACGUGCACCCAGGACUCGGCUCACACAUGC
AGUUCGCUUUCCUGUUGGUGGGGGGAACGCCGAUCGUGCGCAUCCGUCAC
CCCUCGCCGGCAGUGGGGGCUUGUGAACCCCCAAACCUGACUGACUGGGC
CAGUGUGCU,
 or a sequence that has at least 80% or at least 95% identity with at least 50 contiguous nucleotides of said sequence.

3. A ribonucleic acid as claimed in claim 1 or claim 2 being a human telomerase RNA component.

4. A ribonucleic acid as claimed in any of claims 1 to 3 comprising the sequence:

5' –UCUAACCCUAACUGAGAAGGGCGUAGGCGCCGUGCUUUUGCUCCC
CGCGCGCUGUUUUUCUCGCUGACUUUCAGCGGGCGGAAAAGCCUCGG
CCUGCCGCCUUCCACCGUUCAUUCUAGAGCAAACAAAAAAUGUCAGCU
GCUGGCCCGUUCGCCCCUCCC-3'

5. An isolated nucleic acid molecule encoding a ribonucleic acid of any of claims 1 to 4.

6. A recombinant plasmid comprising a nucleotide sequence that encodes a ribonucleic acid of any of claims 1 to 4.

7. A host cell transformed with a plasmid of claim 6.

8. An oligonucleotide comprising more than 10 contiguous nucleotides complementary to contiguous nucleotides of a ribonucleic acid of any of claims 1 to 4 and which inhibits mammalian telomerase enzyme activity.

9. An oligonucleotide as claimed in claim 8, wherein the oligonucleotide comprises at least 15, optionally at least 20 contiguous nucleotides complementary to contiguous nucleotides of a ribonucleic acid of claims 1 to 4.

10. An oligonucleotide as claimed in claim 8 consisting of said more than 10 contiguous nucleotides complementary to contiguous nucleotides of a ribonucleic acid of any of claims 1 to 4.

11. An oligonucleotide as claimed in claim 9 consisting of said at least 15, optionally of said at least 20, contiguous nucleotides complementary to contiguous nucleotides of a ribonucleic acid of any of claims 1 to 4.

12. An oligonucleotide as claimed in claim 8 or claim 9 having the sequence: 5'-CTCAGTTAGGGTTAGACAAA-3'; 5'-CGCCCTTCTCAGTTAGGGTTAG-3'; 5'-GGCGCCTACGCCCTTCTCAGTT-3'; or

5'-CAGGCCCACCCTCCGCAACC-3'

13. An oligonucleotide as claimed in any of claims 8 to 11 being a ribozyme that specifically cleaves the RNA component of telomerase.

14. An oligonucleotide as claimed in any of claims 8 to 12 comprising modified nucleotide analogs, e.g. O-methyl ribonucleotides, phosphorothioate nucleotides or methyl phosphonate nucleotides.

15. A pharmaceutical formulation comprising an oligonucleotide of any of Claims 7 to 14.

16. An oligonucleotide as claimed in any of claims 8 to 14 for use as a pharmaceutical.

17. Use of an oligonucleotide as claimed in any of claims 8 to 14 for the manufacture of a medicament to treat neoplasia.

18. A method of inhibiting telomerase activity in a cell *in vitro*, comprising contacting the cell with an oligonucleotide of any of claims 8 to 14.

19. An isolated, recombinant or synthesized nucleic acid comprising a sequence identical or complementary to more than 10 consecutive nucleotides of a ribonucleic acid of any of claims 1-4, or complement thereof, and which is selected from:
a) a nucleotide probe that specifically hybridizes with telomerase RNA or its coding sequence in a mammalian, preferably human, sample, optionally comprising a detectable label; or
b) a nucleotide primer capable of priming an amplification reaction specific for telomerase RNA or its coding sequence in a mammalian, preferably human, sample.

20. A nucleic acid as claimed in claim 19, wherein the nucleic acid comprises a sequence identical or complementary to at least 20 consecutive nucleotides of a ribonucleic acid of any of claims 1-4, or complement thereof.

21. The use of an oligonucleotide comprising a sequence identical or complementary to more than 10 consecutive nucleotides of a ribonucleic acid of any of claims 1 to 4, or complement thereof, as:
a) a nucleotide probe specific for telomerase RNA or its coding sequence in a mammalian, preferably human, sample, optionally comprising a detectable label; or
b) a nucleotide primer capable of priming an amplification reaction specific for telomerase RNA or its coding sequence.

22. A use as claimed in claim 21, wherein the oligonucleotide comprises a sequence identical or complementary to at least 20 consecutive nucleotides of a ribonucleic acid of any of claims 1 to 4, or complement thereof.

23. A method for determining the level, amount or presence of telomerase RNA component in a mammalian, preferably human, sample, comprising either:
a) combining the sample with an oligonucleotide probe as defined in claim 19 or claim 20 under conditions where the oligonucleotide will specifically hybridize with telomerase RNA in the sample, and detecting any hybrid that forms as a result; or
b) combining the sample with a pair of oligonucleotide primers as defined in claim 19 or claim 20, performing an amplification reaction and detecting any amplification product that forms as a result.

24. A method as claimed in claim 23, further comprising quantifying the hybrid or amplification product so as to determine the concentration of telomerase RNA component in the sample.

25. A method for detecting telomerase RNA component in a sample obtained from a patient, comprising binding a labelled oligonucleotide probe or primer as defined in claim 19 or claim 20 to a sample obtained from the patient, and detecting the presence of labelled material bound to the RNA in the sample.

26. A method for detecting the presence of cancer cells in a sample, the method comprising determining the level of telomerase RNA component in the sample according to claim 23 or claim 24 and correlating the presence of a pathognomic level of telomerase RNA component with the presence of cancer cells.

27. A kit for determining the level, amount or presence of human telomerase RNA component in a sample, said kit comprising an oligonucleotide probe or primer as defined in claim 19 or claim 20 and optionally comprising a set of instructions.

28. A method for isolating telomerase holoenzyme, comprising capturing the holoenzyme protein using an affinity agent comprising more than 10 consecutive nucleotides of an oligonucleotide complementary to a ribonucleic acid of any of claims 1 to 4, and then recovering the protein from the affinity agent.

29. A method as claimed in claim 28, wherein the affinity agent is attached to a solid support or is chemically modified for subsequent immobilization on a solid support.

30. A method of identifying a telomerase RNA component of a non-human mammalian species comprising contacting nucleic acid from tissue of said species with an oligonucleotide primer as defined in claim 17 or claim 18.

31. An isolated, recombinant or synthesized nucleic acid comprising a transcription regulatory region having at least one of the following properties:
(a) it comprises the following sequence, or its complement: 
5'-GATCAGTTAGAAAGTTACTAGTCCACATATAAAGTGCCAAGTCTTGT
ACTCAAGATTATAAGCAATAGGAATTTAAAAAAGAAATTATGAAAACTG
ACAAGATTTAGTGCCTACTTAGATATGAAGGGGAAAGAAGGGTTTGAGAT
AATGTGGGATGCTAAGAGAATGGTGGTAGTGTTGACATATAACTCAAAGC
ATTTAGCATCTACTCTATGTAAGGTACTGTGCTAAGTGCAATAGTGCTAA
AAACAGGAGTCAGATTCTGTCCGTAAAAAACTTTACAACCTGGCAGATGC
TATGAAAGAAAAAGGGGATGGAGAGAGAGAAGGAGGGAGAGAGATGGAG
AGGGAGATATTTTACTTTTCTTTCAGATCGAGGACCGACAGCGACAACTC
CACGGAGTTTATCTAACTGAATACGAGTAAAACTTTTAAGATCATCCTGT
CATTTATATGTAAAACTGCACTATACTGGCCATTATAAAAATTCGCGGCC
GGGTGCGGTGGCTCATACCTGTAATCCCAGCACTTTGGGAGGCCGAAGCG
GGTGGATCACTTGAGCCCTGGCGTTCGAGACCAGCCTGGGCAACATGGTG
AAACCCCCGTCTCTACTAAAAACACAAAAACTAGCTGGGCGTGGTGGCAG
GCGCCTGTAATCCCAGCTACTCAGGAGGCTGAGACACGAGAATCGCTTGA
ACCCGGGAGCAGAGGTTGCAGTGAGCCGAGATCACGCCACTAGACTCCAT
CCAGCCTGGGCGAAAGAGCAAGACTCCGTCTCAAAAAAAAAAATCGTTAC
AATTTATGGTGGATTACTCCCCTCTTTTTACCTCATCAAGACACAGCACT
ACTTTAAAGCAAAGTCAATGATTGAAACGCCTTTCTTTCCTAATAAAAGG
GAGATTCAGTCCTTAAGATTAATAATGTAGTAGTTACACTTGATTAAAGC
CATCCTCTGCTCAAGGAGAGGCTGGAGAAGGCATTCTAAGGAGAAGGGG
CAGGGTAGGAACTCGGACGCATCCCACTGAGCCGAGACAAGATTCTGCTG
TAGTCAGTGCTGCCTGGGAATCTATTTTCACAAAGTTCTCCAAAAAATGT
GATGATCAAAACTAGGAATTAGTGTTCTGTGTCTTAGGCCCTAAAATCTT
CCTGTGAATTCCATTTTTAAGGTAGTCGAGGTGAACCGCGTCTGGTCTGC
AGAGGATAGAAAAAAGGCCCTCTGATACCTCAAGTTAGTTTCACCTTTAA
AGAAGGTCGGAAGTAAAGACGCAAAGCCTTTCCCGGACGTGCGGAAGGGC
AACGTCCTTCCTCATGGCCGGAAATGGAACTTTAATTTCCCGTTCCCCCC
AACCAGCCCGCCCGAGAGAGTGACTCTCACGAGAGCCGCGAGAGTCAGCT
TGGCCAATCCGTGCGGTCGGCGGCCGCTCCCTTTATAAGCCGACTCGCCC
GGCAGCGCACCGGGTTGCGGAGGGAGGGTGGGCCTGGGAGGGGTGGTGGC
CATTTTTTGTCTAACCCTAACTGAGAAGGGCGTAGGCGCCGTGCTTTTGC
TCCCCGCGCGCTGTTTTTCTCGCTGACTTTCAGCGGGCGGAAAAGCCTCG
GCCTGCCGCCTTCCACCGTTCATTCTAGAGCAAACAAAAAATGTCAGCTG
CTGGCCCGTTCGCCCCTCCCGGACCTGCGGCGGGTCGCTGCCCAGCCCC
CGAACCCCGCCTGGAGGCCGCGGTCGGCCGGGGCTTCTCCGGAGGCACCC
ACTGCCACCGCGAAGAGTTGGGCTCTGTCAGCCGCGGGTCTCTCGGGGGC
GAGGGCGAGGTTCACCGTTTCAGGCCGCAGGAAGAGGAACGGAGCGAGTC
CCGCGCGCGGCGCGATTCCCTGAGCTATGGGACGTGCACCCAGGACTCGG
CTCACACATGCAGTTCGCTTTCCTGTTGGTGGGGGGAACGCCGATCGTGC
GCATCCGTCACCCCTCGCCGGCAGTGGGGGCTTGTGAACCCCCAAACCTG
ACTGACTGGGCCAGTGTGCTGCAAATTGGCAGGAGACGTGAAGGCACCTC
CAAAGTCGGCCAAAATGAATGGGCAGTGAGCCGGGGTTGCCTGGAGCCGT
TCCTGCGTGGGTTCTCCCGTCTTCCGCTTTTTGTTGCCTTTTATGGTTGT
ATTACAACTTAGTTCCTGCTCTGCAGATTTTGTTGAGGTTTTTGCTTCTC
CCAAGGTAGATCTCGACCAGTCCCTCAACGGGGTGTGGGAGAACAGTCA
TTTTTTTTTGAGAGATCATTTAACATTTAATGAATATTTAATTAGAAGAT
CTAAATGAACATTGGAAATTGTGTTCCTTTAATGGTCATCGGTTTATGCC
AGAGGTTAGAAGTTTCTTTTTTGAAAATTAGACCTTGGCGATGACCTTG
AGCAGTAGGATATAACCCCCACAAGCTT-3',
(b) it comprises a sequence that is identical to at least 25 consecutive nucleotides of the sequence in (a);
(c) it comprises a sequence that is at least 80% or 95% identical to at least 100 consecutive nucleotides of the sequence in (a), or its complement;
(d) it specifically hybridizes to the -2.5kb HindIII-SacI insert of plasmid pGRN33 (ATCC 75926); or
(e) it specifically hybridizes to a SauIIIA1 - HindIII insert of lambda clone 28-1 (ATCC 75925)

32. A nucleic acid as claimed in claim 31, wherein the transcription regulatory region controls transcription of a gene sequence to which it is operably linked.

33. A recombinant plasmid comprising a nucleotide sequence as claimed in claim 31.

34. A host cell transformed with a plasmid as claimed in claim 33.

35. A transgenic non-human mammal having at least one functionally disrupted mammalian telomerase RNA component allele which has been disrupted by homologous targetting.

## Patentansprüche

1. Eine im Wesentlichen reine Ribonukleinsäure, welche in der Lage ist, mit einer Säugetier-Telomerase-Proteinkomponente zu assozileren, um ein enzymatisch aktives Telomerase-Holoenzym zu bilden, wobei das Oligoribonukleotid eine Sequenz umfasst, welche durch den Codierungsbereich des HindIII-SacI-Inserts mit ~2,5 kb des Plasmids GRN33 (ATCC 75926) oder durch eine Sequenz, die wenigstens 80% oder 95% Identität mit wenigstens 50 aneinander angrenzenden Nukleotiden dieses Codierungsbereichs aufweist, codiert wird.

2. Eine Ribonukleinsäure wie in Anspruch 1 beansprucht, wobei die Sequenz die folgende: 
GGGUUGCGGAGGGAGGGUGGGCCUGGGAGGGGUGGUGGCCAUUUUUUGUC
UAACCCUAACUGAGAAGGGCGUAGGCGCCGUGCUUUUGCUCCCCGCGCGC
UGUUUUUCUCGCUGACUUUCAGCGGGCGGAAAAGCCUCGGCCUGCCGCCU
UCCACCGUUCAUUCUAGAGCAAACAAAAAAUGUCAGCUGCUGGCCCGUUC
GCCCCUCCCGGGACCUGCGGCGGGUCGCUGCCCAGCCCCCGAACCCCGCC
UGGAGGCCGCGGUCGGCCGGGGCUUCUCCGGAGGCACCCACUGCCACCGC
GAAGAGUUGGGCUCUGUCAGCCGCGGGUCUCUCGGGGGCGAGGCGAGGU
UCACCGUUUCAGGCCGCAGGAAGAGGAACGGAGCGAGUCCCGCGCGCGGC
GCGAUUCCCUGAGCUAUGGACGUGCACCCAGGACUCGGCUCACACAUGC
AGUUCGCUUUCCUGUUGGUGGGGGAACGCCGAUCGUGCGCAUCCGUCAC
CCCUCGCCGGCAGUGGGGGCUUGUGAACCCCCAAACCUGACUGACUGGGC
CAGUGUGCU,
 oder eine Sequenz ist, die wenigstens 80% oder wenigstens 95% Identität mit wenigstens 50 aneinander angrenzenden Nukleotiden dieser Sequenz aufweist.

3. Eine Ribonukleinsäure wie in Anspruch 1 oder Anspruch 2 beansprucht, welche eine menschliche Telomerase-RNA-Komponente ist.

4. Eine Ribonukleinsäure wie in irgendeinem der Ansprüche 1 bis 3 beansprucht, welche die folgende Sequenz umfasst:

5' –UCUAACCCUAACUGAGAAGGGCGUAGGCGCCGUGCUUUUGCUCCC
CGCGCGCUGUUUUUCUCGCUGACUUUCAGCGGGCGGAAAAGCCUCGG
CCUGCCGCCUUCCACCGUUCAUUCUAGAGCAAACAAAAAAUGUCAGCU
GCUGGCCCGUUCGCCCCUCCC-3' .

5. Ein isoliertes Nukleinsäuremolekül, welches eine Ribonukleinsäure nach irgendeinem der Ansprüche 1 bis 4 codiert.

6. Ein rekombinantes Plasmid, welches eine Nukleotidsequenz umfasst, die eine Ribonukleinsäure nach irgendeinem der Ansprüche 1 bis 4 codiert.

7. Eine Wirtszelle, welche mit einem Plasmid nach Anspruch 6 transformiert ist.

8. Ein Oligonukleotid, welches mehr als 10 aneinander angrenzende Nukleotide umfasst, die zu aneinander angrenzenden Nukleotiden einer Ribonukleinsäure nach irgendeinem der Ansprüche 1 bis 4 komplementär sind, und welches die Aktivität eines Säugetier-Telomerase-Enzyms hemmt.

9. Ein Oligonukleotid wie in Anspruch 8 beansprucht, wobei das Oligonukleotid wenigstens 15, gegebenenfalls wenigstens 20 aneinander angrenzende Nukleotide umfasst, die zu aneinander angrenzenden Nukleotiden einer Ribonukleinsäure der Ansprüche 1 bis 4 komplementär sind.

10. Ein Oligonukleotid wie in Anspruch 8 beansprucht, bestehend aus den mehr als 10 aneinander angrenzenden Nukleotiden, welche zu aneinander angrenzenden Nukleotiden einer Ribonukleinsäure nach irgendeinem der Ansprüche 1 bis 4 komplementär sind.

11. Ein Oligonukleotid wie in Anspruch 9 beansprucht, bestehend aus den wenigstens 15, gegebenenfalls den wenigstens 20 aneinander angrenzenden Nukleotiden, welche zu aneinander angrenzenden Nukleotiden einer Ribonukleinsäure nach irgendeinem der Ansprüche 1 bis 4 komplementär sind.

12. Ein Oligonukleotid wie in Anspruch 8 oder Anspruch 9 beansprucht mit der Sequenz: 5'-CTCAGTTAGGGTTAGACAAA-3'; 5'-CGCCCTTCTCAGTTAGGGTTAG-3'; 5'-GGCGCCTACGCCCTTCTCAGTT-3'; oder

5′-CAGGCCCACCCTCCGCAACC-3′.

13. Ein Oligonukleotid wie in irgendeinem der Ansprüche 8 bis 11 beansprucht, welches ein Ribozym ist, das spezifisch die RNA-Komponente der Telomerase spaltet.

14. Ein Oligonukleotid wie In irgendeinem der Ansprüche 8 bis 12 beansprucht, welches modifizierte Nukleotidanaloge, z.B. O-Methyl-Ribonukleotide, Phosphorothioat-Nukleotide oder Methylphosphonat-Nukleotide, umfasst.

15. Eine pharmazeutische Formulierung, welche ein Oligonukleotid nach irgendeinem der Ansprüche 7 bis 14 umfasst.

16. Ein Oligonukleotid wie in irgendeinem der Ansprüche 8 bis 14 beansprucht zur Verwendung als ein Pharmazeutikum.

17. Verwendung eines Oligonukleotids wie in irgendeinem der Ansprüche 8 bis 14 beansprucht zur Herstellung eines Medikaments, um Neoplasie zu behandeln.

18. Ein Verfahren zum Hemmen der Telomeraseaktivität in einer Zelle *in vitro*, umfassend das Inkontaktbringen der Zelle mit einem Oligonukleotid nach Irgendeinem der Ansprüche 8 bis 14.

19. Eine isolierte, rekombinante oder synthetisierte Nukleinsäure, umfassend eine Sequenz, die mit mehr als 10 aufeinander folgenden Nukleotiden einer Ribonukleinsäure nach irgendeinem der Ansprüche 1-4 identisch oder zu diesen komplementär ist, oder das Komplementäre davon und welche ausgewählt ist aus:
a) einer Nukleotidsonde, die spezifisch mit Telomerase-RNA oder ihrer codierenden Sequenz in einer Säugetierprobe, vorzugsweise einer aus Menschen, hybridisiert, wobei diese gegebenenfalls eine nachweisbare Markierung umfasst; oder
b) einem Nukleotidprimer, der in der Lage ist, eine Amplifizierungsreaktion zu primen, die für Telomerase-RNA oder ihre codierende Sequenz In einer Säugetierprobe, vorzugsweise einer aus Menschen, spezifisch ist.

20. Eine Nukleinsäure wie in Anspruch 19 beansprucht, wobei die Nukleinsäure eine Sequenz umfasst, die mit wenigstens 20 aufeinander folgenden Nukleotiden einer Ribonukleinsäure nach irgendeinem der Ansprüche 1-4 identisch oder zu diesen komplementär ist, oder das Komplementäre davon.

21. Die Verwendung eines Oligonukleotids, umfassend eine Sequenz, die mit mehr als 10 aufeinander folgenden Nukleotiden einer Ribonukleinsäure nach irgendeinem der Ansprüche 1 bis 4 identisch oder zu diesen komplementär ist, oder eines Komplementären davon als:
a) eine Nukleotidsonde, die spezifisch ist für Telomerase-RNA oder ihre codierende Sequenz in einer Säugetierprobe, vorzugsweise einer aus Menschen, wobei diese gegebenenfalls eine nachweisbare Markierung umfasst; oder
b) einen Nukleotidprimer, der in der Lage ist, eine Amplifizierungsreaktion zu primen, die für Telomerase-RNA oder ihre codierende Sequenz spezifisch ist.

22. Eine Verwendung wie in Anspruch 21 beansprucht, wobei das Oligonukleotid eine Sequenz umfasst, die mit wenigstens 20 aufeinander folgenden Nukleotiden einer Ribonukleinsäure nach irgendeinem der Ansprüche 1 bis 4 identisch oder zu diesen komplementär ist, oder das Komplementäre davon.

23. Ein Verfahren zur Bestimmung des Spiegels, der Menge oder des Vorliegens einer Telomerase-RNA-Komponente in einer Säugetierprobe, vorzugsweise einer aus Menschen, umfassend entweder:
a) das Kombinieren der Probe mit einer Oligonukleotidsonde wie in Anspruch 19 oder Anspruch 20 definiert unter Bedingungen, wo das Oligonukleotid spezifisch mit Telomerase-RNA in der Probe hybridisiert, und das Nachweisen irgendeines Hybrids, das sich als ein Ergebnis bildet; oder
b) das Kombinieren der Probe mit einem Paar von Oligonukleotidprimern wie in Anspruch 19 oder Anspruch 20 definiert, das Durchführen einer Amplifizierungsreaktion und das Nachweisen irgendeines Ampllfizierungsprodukts, das sich als ein Ergebnis bildet.

24. Ein Verfahren wie in Anspruch 23 beansprucht, welches weiterhin das Quantifizieren des Hybrids oder Amplifizierungsprodukts umfasst, um so die Konzentration der Telomerase-RNA-Komponente in der Probe zu bestimmen.

25. Ein Verfahren zum Nachweisen einer Telomerase-RNA-Komponente in einer Probe, die aus einem Patienten erhalten wird, umfassend das Binden einer markierten Oligonukleotidsonde oder eines Primers wie in Anspruch 19 oder Anspruch 20 definiert an eine Probe, die aus dem Patienten erhalten wird, und das Nachweisen des Vorliegens von markiertem Material, das an die RNA in der Probe gebunden ist.

26. En Verfahren zum Nachweisen des Vorliegens von Krebszellen in einer Probe, wobei das Verfahren das Bestimmen des Spiegels der Telomerase-RNA-Komponente in der Probe gemäß Anspruch 23 oder Anspruch 24 und das Korrelieren des Vorliegens eines pathognomen Spiegels der Telomerase-RNA-Komponente mit dem Vorliegen von Krebszellen umfasst.

27. Ein Kit zum Bestimmen des Spiegels, der Menge oder des Vorliegens einer menschlichen Telomerase-RNA-Komponente in einer Probe, wobei der Kit eine Oligonukleotidsonde oder einen Primer wie in Anspruch 19 oder Anspruch 20 definiert umfasst und welcher gegebenenfalls einen Satz Instruktionen umfasst.

28. Ein Verfahren zum Isolieren des Telomerase-Holoenzyms, umfassend das Einfangen des Holoenzymproteins unter Verwendung eines Affinitätsmittels, das mehr als 10 aufeinander folgende Nukleotide eines Oligonukleotids umfasst, welche zu einer Ribonukleinsäure nach irgendeinem der Ansprüche 1 bis 4 komplementär sind, und dann das Gewinnen des Proteins aus dem Affinitätsmittel.

29. Ein Verfahren wie in Anspruch 28 beansprucht, wobei das Affinitätsmittel an einem festen Träger befestigt ist oder zur anschließenden Immobilisierung an einem festen Träger chemisch modifiziert ist.

30. Ein Verfahren zum Identifizieren einer Telomerase-RNA-Komponente von einer nicht-menschlichen Säugetierspezies, umfassend das Inkontakbringen von Nukleinsäure aus Gewebe von dieser Spezies mit einem Oligonukleotidprimer, wie er in Anspruch 17 oder Anspruch 18 definiert ist.

31. Eine isolierte, rekombinante oder synthetisierte Nukleinsäure, umfassend einen transkriptionsregulatorischen Bereich mit wenigstens einer der folgenden Eigenschaften:
(a) er umfasst die folgende Sequenz oder deren Komplementäres: 
5'-GATCAGTTAGAAAGTTACTAGTCCACATATAAAGTGCCAAGTCTTGT
ACTCAAGATTATAAGCAATAGGAATTTAAAAAAAGAAATTATGAAAACTG
ACAAGATTTAGTGCCTACTTAGATATGAAGGGGAAAGAAGGGTTTGAGAT
AATGTGGATGCTAAGAGAATGGTGGTAGTGTTGACATATAACTCAAAGC
ATTTAGCATCTACTCTATGTAAGGTACTGTGCTAAGTGCAATAGTGCTAA
AAACAGGAGTCAGATTCTGTCCGTAAAAAACTTTACAACCTGGCAGATGC
TATGAAAGAAAAAGGGGATGGAGAGAGAGAAGGAGGGAGAGAGATGGAG
AGGGAGATATTTTACTTTTCTTTCAGATCGAGGACCGACAGCGACAACTC
CACGGAGTTTATCTAACTGAATACGAGTAAAACTTTTAAGATCATCCTGT
CATTTATATGTAAAACTGCACTATACTGGCCATTATAAAAATTCGCGGCC
GGGTGCGGTGGCTCATACCTGTAATCCCAGCACTTTGGGAGGCCGAAGCG
GGTGGATCACTTGAGCCCTGGCGTTCGAGACCAGCCTGGGCAACATGGTG
AAACCCCCGTCTCTACTAAAAACACAAAAACTAGCTGGGCGTGGTGGCAG
GCGCCTGTAATCCCAGCTACTCAGGAGGCTGAGACACGAGAATCGCTTGA
ACCCGGGAGCAGAGGTTGCAGTGAGCCGAGATCACGCCACTAGACTCCAT
CCAGCCTGGGCGAAAGAGCAAGACTCCGTCTCAAAAAAAAAAATCGTTAC
AATTTATGGTGGATTACTCCCCTCTTTTTACCTCATCAAGACACAGCACT
ACTTTAAAGCAAAGTCAATGATTGAAACGCCTTTCTTTCCTAATAAAGG
GAGATTCAGTCCTTAAGATTAATAATGTAGTAGTTACACTTGATTAAAGC
CATCCTCTGCTCAAGGAGAGGCTGGAGAAGGCATTCTAAGGAGAAGGGGG
CAGGGTAGGAACTCGGACGCATCCCACTGAGCCGAGACAAGATTCTGCTG
TAGTCAGTGCTGCCTGGGAATCTATTTTCACAAAGTTCTCCAAAAAATGT
GATGATCAAAACTAGGAATTAGTGTTCTGTGTCTTAGGCCCTAAAATCTT
CCTGTGAATTCCATTTTTAAGGTAGTCGAGGTGAACCGCGTCTGGTCTGC
AGAGGATAGAAAAAAGGCCCTCTGATACCTCAAGTTAGTTTCACCTTTAA
AGAAGGTCGGAAGTAAAGACGCAAAGCCTTTCCCGGACGTGCGGAAGGGC
AACGTCCTTCCTCATGGCCGGAAATGGAACTTTAATTTCCCGTTCCCCCC
AACCAGCCCGCCCGAGAGAGTGACTCTCACGAGAGCCGCGAGAGTCAGCT
TGGCCAATCCGTGCGGTCGGCGGCCGCTCCCTTTATAAGCCGACTCGCCC
GGCAGCGCACCGGGTTGCGGAGGGAGGGTGGGCCTGGGAGGGGTGGTGGC
CATTTTTTGTCTAACCCTAACTGAGAAGGGCGTAGGCGCCGTGCTTTTGC
TCCCCGCGCGCTGTTTTTCTCGCTGACTTTCAGCGGGCGGAAAAGCCTCG
GCCTGCCGCCTTCCACCGTTCATTCTAGAGCAAACAAAAAATGTCAGCTG
CTGGCCCGTTCGCCCCTCCCGGACCTGCGGCGGGTCGCTGCCCAGCCCC
CGAACCCCGCCTGGAGGCCGCGGTCGGCCGGGGCTTCTCCGGAGGCACCC
ACTGCCACCGCGAAGAGTTGGGCTCTGTCAGCCGCGGGTCTCTCGGGGGC
GAGGGCGAGGTTCACCGTTTCAGGCCGCAGGAAGAGGAACGGAGCGAGTC
CCGCGCGCGGCGCGATTCCCTGAGCTATGGGACGTGCACCCAGGACTCGG
CTCACACATGCAGTTCGCTTTCCTGTTGGTGGGGGGAACGCCGATCGTGC
GCATCCGTCACCCCTCGCCGGCAGTGGGGGCTTGTGAACCCCCAAACCTG
ACTGACTGGGCCAGTGTGCTGCAAATTGGCAGGAGACGTGAAGGCACCTC
CAAAGTCGGCCAAAATGAATGGGCAGTGAGCCGGGGTTGCCTGGAGCCGT
TCCTGCGTGGGTTCTCCCGTCTTCCGCTTTTTGTTGCCTTTTATGGTTGT
ATTACAACTTAGTTCCTGCTCTGCAGATTTTGTTGAGGTTTTTGCTTCTC
CCAAGGTAGATCTCGACCAGTCCCTCAACGGGGTGTGGGGAGAACAGTCA
TTTTTTTTTGAGAGATCATTTAACATTTAATGAATATTTAATTAGAAGAT
CTAAATGAACATTGGAAATTGTGTTCCTTTAATGGTCATCGGTTTATGCC
AGAGGTTAGAAGTTTCTTTTTGAAAAATTAGACCTTGGCGATGACCTTG
AGCAGTAGGATATAACCCCCACAAGCTT-3',
(b) er umfasst eine Sequenz, die mit wenigstens 25 aufeinander folgenden Nukleotiden der Sequenz in (a) identisch ist;
(c) er umfasst eine Sequenz, die zu wenigstens 80% oder 95% mit wenigstens 100 aufeinander folgenden Nukleotiden der Sequenz in (a) identisch ist, oder deren Komplementäres;
(d) er hybridisiert spezifisch mit dem HindIII-SacI-Insert von ~2,5 kb des Plasmids pGRN33 (ATCC 75926); oder
(e) er hybridisiert spezifisch mit einem SauIIIA1-HindIII-Insert des Lambda-Klons 28-1 (ATCC 75925).

32. Eine Nukleinsäure wie in Anspruch 31 beansprucht, wobei der transkriptionsregulatorische Bereich die Transkription einer Gensequenz, mit welcher dieser funktionsfähig verknüpft ist, steuert.

33. Ein rekombinantes Plasmid, welches eine Nukleotidsequenz wie in Anspruch 31 beansprucht umfasst.

34. Eine Wirtszelle, welche mit einem Plasmid wie in Anspruch 33 beansprucht transformiert ist.

35. Ein transgenes nichtmenschliches Säugetier mit wenigstens einem funktional unterbrochenen Allel einer Säugetier-Telomerase-RNA-Komponente, welches durch homologes Targetting unterbrochen wurde.

## Revendications

1. Acide ribonucléique sensiblement pur capable de s'associer à un composant protéïque d'une télomérase de mammifère pour former une holoenzyme de télomérase active du point de vue enzymatique, l'oligoribonucléotide comprenant une séquence codée par la région codante de l'insert HindIII-Sacl d'environ 2,5 kb du plasmide GRN33 (ATCC 75926), ou par une séquence qui a au moins 80 % ou 95 % d'identité d'avec au moins 50 nucléotides contigus de ladite région codante.

2. Acide ribonucléique selon la revendication 1, dans lequel la séquence est : 
GGGUUGCGGAGGGAGGGUGGCCUGGGAGGGGUGGUGGCCAUUUUUGUC
UAACCCUAACUGAGAAGGGCGUAGGCGCCGUGCUUUUGCUCCCCGCGCGC
UGUUUUUCUCGCUGACUUUCAGCGGGCGGAAAAGCCUCGGCCUGCCGCCU
UCCACCGUUCAUUCUAGAGCAAACAAAAAAUGUCAGCUGCUGGCCCGUUC
GCCCCUCCCGGGACCUGCGGCGGGUCGCUGCCCAGCCCCCGAACCCCGCC
UGGAGGCCGCGGUCGGCCGGGGCUUCUCCGGAGGCACCCACUGCCACCGC
GAAGAGUUGGGCUCUGUCAGCCGCGGGUCUCUCGGGGGCGAGGGCGAGGU
UCACCGUUUCAGGCCGCAGGAAGAGGAACGGAGCGAGUCCCGCGCGCGGC
GCGAUUCCCUGAGCUAUGGACGUGCACCCAGGACUCGGCUCACACAUGC
AGUUCGCUUUCCUGUUGGUGGGGGAACGCCGAUCGUGCGCAUCCGUCAC
CCCUCGCCGGCAGUGGGGGCUUGUGAACCCCCAAACCUGACUGACUGGGC
CAGUGUGCU,
 ou une séquence qui a au moins 80 % ou au moins 95 % d'identité avec au moins 50 nucléotides contigus de ladite séquence.

3. Acide ribonucléique selon la revendication 1 ou la revendication 2, celui-ci étant un composant ARN d'une télomérase humaine.

4. Acide ribonucléique selon l'une quelconque des revendications 1 à 3 comprenant la séquence :

5' –UCUAACCCUAACUGAGAAGGGCGUAGGCGCCGUGCUUUUGCUCCC
CGCGCGCUGUUUUUCUCGCUGACUUUCAGCGGGCGGAAAAGCCUCGG
CCUGCCGCCUUCCACCGUUCAUUCUAGAGCAAACAAAAAAUGUCAGCU
GCUGGCCCGUUCGCCCCUCCC-3'

5. Molécule d'acide nucléique isolé codant un acide ribonucléique selon l'une quelconque des revendications 1 à 4.

6. Plasmide recombinant comprenant une séquence nucléotidique qui code un acide ribonucléique selon l'une quelconque des revendications 1 à 4.

7. Cellule hôte transformée avec un plasmide selon la revendication 6.

8. Oligonucléotide comprenant plus de 10 nucléotides contigus complémentaires à des nucléotides contigus d'un acide ribonucléique selon l'une quelconque des revendications 1 à 4 et qui inhibe l'activité enzymatique d'une télomérase de mammifère.

9. Oligonucléotide selon la revendication 8, dans lequel l'oligonucléotide comprend au moins 15, éventuellement au moins 20 nucléotides contigus complémentaires à des nucléotides contigus d'un acide ribonucléique selon les revendications 1 à 4.

10. Oligonucléotide selon la revendication 8 constitué desdits plus de 10 nucléotides contigus complémentaires à des nucléotides contigus d'un acide ribonucléique selon l'une quelconque des revendications 1 à 4.

11. Oligonucléotide selon la revendication 9 constitué desdits au moins 15, éventuellement desdits au moins 20, nucléotides contigus complémentaires à des nucléotides contigus d'un acide ribonucléique selon l'une quelconque des revendications 1 à 4.

12. Oligonucléotide selon la revendication 8 ou la revendication 9 ayant la séquence : 5'-CTCAGTTAGGGTTAGACAAA-3'; 5'-CGCCCTTCTCAGTTAGGGTTAG-3'; 5'-GGCGCCTACGCCCTTCTCAGTT-3'; ou

5'-CAGGCCCACCCTCCGCAACC-3'

13. Oligonucléotide selon l'une quelconque des revendications 8 à 11, celui-ci étant un ribozyme qui clive spécifiquement le composant ARN d'une télomérase.

14. Oligonucléotide selon l'une quelconque des revendications 8 à 12 comprenant des analogues de nucléotides modifiés, par exemple des ribonucléotides O-méthylés, des nucléotides phosphorothioate ou des nucléotides phosphonates de méthyle.

15. Formulation pharmaceutique comprenant un oligonucléotide selon l'une quelconque des revendications 7 à 14.

16. Oligonucléotide selon l'une quelconque des revendications 8 à 14 pour l'utilisation en tant que produit pharmaceutique.

17. Utilisation d'un oligonucléotide selon l'une quelconque des revendications 8 à 14 pour la fabrication d'un médicament pour traiter une néoplasie.

18. Procédé d'inhibition d'une activité télomérase dans une cellule *in vitro*, comprenant la mise en contact de la cellule avec un oligonucléotide selon l'une quelconque des revendications 8 à 14.

19. Acide nucléique recombinant ou synthétique isolé comprenant une séquence identique ou complémentaire à plus de 10 nucléotides consécutifs d'un acide ribonucléique selon l'une quelconque des revendications 1 à 4, ou un complément de celle-ci, et qui est choisi parmi :
a) une sonde nucléotidique qui s'hybride spécifiquement à un ARN d'une télomérase ou sa séquence codante dans un échantillon de mammifère, de préférence humain, comprenant éventuellement un marqueur détectable ; ou
b) une amorce nucléotidique capable d'amorcer une réaction d'amplification spécifique d'un ARN d'une télomérase ou sa séquence codante dans un échantillon de mammifère, de préférence humain.

20. Acide nucléique selon la revendication 19, dans lequel l'acide nucléique comprend une séquence identique ou complémentaire à au moins 20 nucléotides consécutifs d'un acide ribonucléique selon l'une quelconque des revendications 1 à 4, ou un complément de celle-ci.

21. Utilisation d'un oligonucléotide comprenant une séquence identique ou complémentaire à plus de 10 nucléotides consécutifs d'un acide ribonucléique selon l'une quelconque des revendications 1 à 4, ou un complément de celle-ci, tel que :
a) une sonde nucléotidique spécifique d'un ARN d'une télomérase ou sa séquence codante dans un échantillon de mammifère, de préférence humain, comprenant éventuellement un marqueur détectable ; ou
b) une amorce nucléotidique capable d'amorcer une réaction d'amplification spécifique pour un ARN d'une télomérase ou sa séquence codante.

22. Utilisation selon la revendication 21, dans laquelle l'oligonucléotide comprend une séquence identique ou complémentaire à au moins 20 nucléotides consécutifs d'un acide ribonucléique selon l'une quelconque des revendications 1 à 4, ou un complément de celle-ci.

23. Procédé de détermination du niveau, de la quantité ou de la présence d'un composant ARN d'une télomérase dans un échantillon de mammifère, de préférence humain, comprenant les étapes consistant à:
a) combiner l'échantillon avec une sonde oligonucléotidique telle que définie dans la revendication 19 ou la revendication 20 dans des conditions dans lesquelles l'oligonucléotide s'hybridera spécifiquement à un ARN de télomérase dans l'échantillon, et détecter tout hybride qui se forme comme résultat ; ou
b) combiner l'échantillon avec une paire d'amorces oligonucléotidiques telles que définies dans la revendication 19 ou la revendication 20, effectuer une réaction d'amplification et détecter tout produit d'amplification qui se forme comme résultat.

24. Procédé selon la revendication 23, comprenant en outre la quantification de l'hybride ou du produit d'amplification de manière à déterminer la concentration du composant ARN d'une télomérase dans l'échantillon.

25. Procédé de détection d'un composant ARN d'une télomérase dans un échantillon provenant d'un patient, comprenant la liaison d'une sonde ou d'une amorce oligonucléotidique marquée telles que définies dans la revendication 19 ou la revendication 20 à un échantillon provenant du patient, et la détection de la présence d'un matériau marqué lié à l'ARN dans l'échantillon.

26. Procédé de détection de la présence de cellules cancéreuses dans un échantillon, le procédé comprenant la détermination du niveau du composant ARN d'une télomérase dans l'échantillon selon la revendication 23 ou la revendication 24 et la corrélation de la présence d'un niveau pathognomique du composant ARN de télomérase en présence de cellules cancéreuses.

27. Trousse de détermination du niveau, de la quantité ou de la présence d'un composant ARN d'une télomérase humaine dans un échantillon, ladite trousse comprenant une sonde ou une amorce oligonucléotidique telles que définies dans la revendication 19 ou la revendication 20 et comprenant éventuellement une série d'instructions.

28. Procédé d'isolement d'une holoenzyme de télomérase, comprenant la capture de la protéine d'holoenzyme en utilisant un agent d'affinité comprenant plus de 10 nucléotides consécutifs d'un oligonucléotide complémentaire d'un acide ribonucléique selon l'une quelconque des revendications 1 à 4, et la récupération ensuite de la protéine à partir de l'agent d'affinité.

29. Procédé selon la revendication 28, dans lequel l'agent d'affinité est attaché à un support solide ou il est modifié chimiquement pour une immobilisation ultérieure sur un support solide.

30. Procédé d'identification d'un composant ARN d'une télomérase d'une espèce mammifère non humaine comprenant la mise en contact d'un acide nucléique à partir du tissu de ladite espèce avec une amorce oligonucléotidique telle que définie dans la revendication 17 ou la revendication 18.

31. Acide nucléique isolé, recombinant ou synthétique comprenant une région régulatrice de la transcription ayant au moins une des propriétés suivantes :
(a) elle comprend la séquence suivante, ou son complément : 
5'-GATCAGTTAGAAAGTTACTAGTCCACATATAAAGTGCCAAGTCTTGT
ACTCAAGATTATAAGCAATAGGAATTTAAAAAAAGAAATTATGAAAACTG
ACAAGATTTAGTGCCTACTTAGATATGAAGGGGAAAGAAGGGTTTGAGAT
AATGTGGGATGCTAAGAGAATGGTGGTAGTGTTGACATATAACTCAAAGC
ATTTAGCATCTACTCTATGTAAGGTACTGTGCTAAGTGCAATAGTGCTAA
AAACAGGAGTCAGATTCTGTCCGTAAAAAACTTTACAACCTGGCAGATGC
TATGAAAGAAAAAGGGGATGGAGAGAGAGAAGGAGGGAGAGAGATGGAG
AGGGAGATATTTTACTTTTCTTTCAGATCGAGGACCGACAGCGACAACTC
CACGGAGTTTATCTAACTGAATACGAGTAAAACTTTTAAGATCATCCTGT
CATTTATATGTAAAACTGCACTATACTGGCCATTATAAAAATTCGCGGCC
GGGTGCGGTGGCTCATACCTGTAATCCCAGCACTTTGGGAGGCCGAAGCG
GGTGGATCACTTGAGCCCTGGCGTTCGAGACCAGCCTGGGCAACATGGTG
AAACCCCCGTCTCTACTAAAAACACAAAAACTAGCTGGGCGTGGTGGCAG
GCGCCTGTAATCCCAGCTACTCAGGAGGCTGAGACACGAGAATCGCTTGA
ACCCGGGAGCAGAGGTTGCAGTGAGCCGAGATCACGCCACTAGACTCCAT
CCAGCCTGGGCGAAAGAGCAAGACTCCGTCTCAAAAAAAAAAATCGTTAC
AATTTATGGTGGATTACTCCCCTCTTTTACCTCATCAAGACACAGCACT
ACTTTAAAGCAAAGTCAATGATTGAAACGCCTTTCTTTCCTAATAAAAGG
GAGATTCAGTCCTTAAGATTAATAATGTAGTAGTTACACTTGATTAAAGC
CATCCTCTGCTCAAGGAGAGGCTGGAGAAGGCATTCTAAGGAGAAGGGGG
CAGGGTAGGAACTCGGACGCATCCCACTGAGCCGAGACAAGATTCTGCTG
TAGTCAGTGCTGCCTGGAATCTATTTCACAAAGTTCTCCAAAAAATGT
GATGATCAAAACTAGGAATTAGTGTTCTGTGTCTTAGGCCCTAAAATCTT
CCTGTGAATTCCATTTTTAAGGTAGTCGAGGTGAACCGCGTCTGGTCTGC
AGAGGATAGAAAAAAGGCCCTCTGATACCTCAAGTTAGTTTCACCTTTAA
AGAAGGTCGGAAGTAAAGACGCAAAGCCTTTCCCGGACGTGCGGAAGGGC
AACGTCCTTCCTCATGGCCGGAAATGGAACTTTAATTTCCCGTTCCCCCC
AACCAGCCCGCCCGAGAGAGTGACTCTCACGAGAGCCGCGAGAGTCAGCT
TGGCCAATCCGTGCGGTCGGCGGCCGCTCCCTTTATAAGCCGACTCGCCC
GGCAGCGCACCGGGTTGCGGAGGGAGGGTGGGCCTGGAGGGGTGGTGGC
CATTTTTTGTCTAACCCTAACTGAGAAGGGCGTAGGCGCCGTGCTTTTGC
TCCCCGCGCGCTGTTTTTCTCGCTGACTTTCAGCGGGCGGAAAAGCCTCG
GCCTGCCGCCTTCCACCGTTCATTCTAGAGCAAACAAAAAATGTCAGCTG
CTGGCCCGTTCGCCCCTCCCGGACCTGCGGCGGGTCGCTGCCCAGCCCC
CGAACCCCGCCTGGAGGCCGCGGTCGGCCCGGGGCTTCTCCGGAGGCACCC
ACTGCCACCGCGAAGAGTTGGGCTCTGTCAGCCGCGGGTCTCTCGGGGGC
GAGGGCGAGGTTCACCGTTTCAGGCCGCAGGAAGAGGAACGGAGCGAGTC
CCGCGCGCGGCGCGATTCCCTGAGCTATGGACGTGCACCCAGGACTCGG
CTCACACATGCAGTTCGCTTTCCTGTTGGTGGGGGAACGCCGATCGTGC
GCATCCGTCACCCCTCGCCGGCAGTGGGGGCTTGTGAACCCCCAAACCTG
ACTGACTGGGCCAGTGTGCTGCAAATTGGCAGGAGACGTGAAGGCACCTC
CAAAGTCGGCCAAAATGAATGGGCAGTGAGCCGGGGTTGCCTGGAGCCGT
TCCTGCGTGGGTTCTCCCGTCTTCCGCTTTTTGTTGCCTTTTATGGTTGT
ATTACAACTTAGTTCCTGCTCTGCAGATTTTGTTGAGGTTTTTGCTTCTC
CCAAGGTAGATCTCGACCAGTCCCTCAACGGGGTGTGGGGAGAACAGTCA
TTTTTTTTGAGAGATCATTTAACATTTAATGAATATTTAATTAGAAGAT
CTAAATGAACATTGGAAATTGTGTTCCTTTAATGGTCATCGGTTTATGCC
AGAGGTTAGAAGTTTCTTTTTTGAAAAATTAGACCTTGGCGATGACCTTG
AGCAGTAGGATATAACCCCCACAAGCTT-3',
(b) elle comprend une séquence qui est identique à au moins 25 nucléotides consécutifs de la séquence de (a) ;
(c) elle comprend une séquence qui est identique à au moins 80 % ou 95 % à au moins 100 nucléotides consécutifs de la séquence en (a) ou son complément ;
(d) elle s'hybride spécifiquement à l'insert HindIII-Sacl d'environ 2,5 kb du plasmide pGRN33 (ATCC 75928) ; ou
(e) elle s'hybride spécifiquement à un insert SauIIIA1 - HindIII du clone lambda 28-1 (ATCC 75925).

32. Acide nucléique selon la revendication 31, dans lequel la région régulatrice de la transcription contrôle la transcription d'une séquence génique à laquelle elle est liée de manière fonctionnelle.

33. Plasmide recombinant comprenant une séquence nucléotidique selon la revendication 31.

34. Cellule hôte transformée avec un plasmide selon la revendication 33.

35. Mammifère non humain transgénique ayant au moins un allèle d'un composant ARN d'une télomérase mammifère inactivé d'un point de vue fonctionnel, lequel a été interrompu par ciblage homologue.
